(19) Europäisches Patentamt
European Patent Office
Office européen des brevets

(11) EP 1 255 751 B1

(12) FASCICULE DE BREVET EUROPEEN

(45) Date de publication et mention de la délivrance du brevet:
**16.06.2004 Bulletin 2004/25**

(21) Numéro de dépôt: **01907685.0**

(22) Date de dépôt: **24.01.2001**

(51) Int Cl.[7]: **C07D 403/04**, A61K 31/404, A61P 43/00, C07D 405/14

(86) Numéro de dépôt international:
**PCT/FR2001/000226**

(87) Numéro de publication internationale:
**WO 2001/055130 (02.08.2001 Gazette 2001/31)**

# (54) DERIVES DE 1,3-DIHYDRO-2H-INDOL-2-ONE ET LEUR UTILISATION EN TANT QUE LIGANDS DES RECEPTEURS V1B OU V1B ET V1A DE L'ARGININE-VASOPRESSINE

1,3-DIHYDRO-2H-INDOL-2-ON DERIVATE UND DEREN VERWENDUNG ALS LIGANDEN DER ARGININ-VASOPRESSIN REZEPTOREN V1B ODER V1B UND V1A

1,3-DIHYDRO-2H-INDOL-2-ONE DERIVATIVES AND THEIR USE AS LIGANDS FOR V1B OR V1B AND V1A ARGININE-VASOPRESSIN RECEPTORS

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **25.01.2000 FR 0000957**

(43) Date de publication de la demande:
**13.11.2002 Bulletin 2002/46**

(73) Titulaire: **SANOFI-SYNTHELABO**
**75013 Paris (FR)**

(72) Inventeurs:
- **ROUX, Richard**
  **F-34570 Vailhauques (FR)**
- **SERRADEIL-LE GAL, Claudine**
  **F-31750 Escalquens (FR)**
- **TONNERRE, Bernard**
  **F-34570 Vailhauques (FR)**
- **WAGNON, Jean**
  **F-34070 Montpellier (FR)**

(74) Mandataire: **Thouret-Lemaitre, Elisabeth et al**
**Sanofi-Synthélabo,**
**Département Brevets,**
**174, avenue de France**
**75013 Paris (FR)**

(56) Documents cités:
**WO-A-95/18105** **US-A- 5 338 755**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 255 751 B1

## Description

La présente invention a pour objet de nouveaux dérivés de 1,3-dihydro-2H-indol-2-one, un procédé pour leur préparation et les compositions pharmaceutiques en contenant.

Les composés selon la présente invention présentent une affinité et une sélectivité pour les récepteurs $V_{1b}$ ou à la fois pour les récepteurs $V_{1b}$ et $V_{1a}$ de l'arginine-vasopressine (AVP).

L'AVP est une hormone connue pour son effet antidiurétique et son effet dans la régulation de la pression artérielle. Elle stimule plusieurs types de récepteurs : $V_1$ ($V_{1a}$,$V_{1b}$), $V_2$. Ces récepteurs sont localisés en particulier dans le foie, les vaisseaux (coronaires, rénaux, cérébraux), les plaquettes, le rein, l'utérus, les glandes surrénales, le pancréas, le système nerveux central, l'hypophyse. L'AVP exerce ainsi des effets cardiovasculaires, hépatiques, pancréatiques, antidiurétiques, agrégants des plaquettes et des effets sur le système nerveux central et périphérique, et sur la sphère utérine.

La localisation des différents récepteurs est décrite dans : S. JARD et al., Vasopressin and oxytocin receptors : an overview, *in* Progress in Endocrinology. H. IMURA and K. SHIZURNE ed., Experta Medica, Amsterdam, 1988, 1183-1188, ainsi que dans les articles suivants : J. Lab. Clin. Med., 1989, 114 (6), 617-632 et Pharmacol. Rev., 1991, 43 (1), 73-108.

Plus particulièrement, les récepteurs $V_{1a}$ de l'AVP sont localisés dans de nombreux organes périphériques et dans le cerveau. Ds ont été clonés chez le rat et l'homme et ils régulent la majorité des effets connus de l'AVP : l'agrégation plaquettaire ; les contractions utérines; la contraction des vaisseaux; la sécrétion d'aldostérone, de cortisol, du CRF (de l'anglais corticotropin-releasing factor) et de l'hormone adrénocorticotrophique (ACTH, de l'anglais adrenocorticotrophic hormone) ; la glycogénolyse hépatique, la prolifération cellulaire et les principaux effets centraux de l'AVP (hypothermie, mémoire, ...).

Les récepteurs $V_{1b}$ ont été initialement identifiés dans l'adénohypophyse de différentes espèces animales (rat, porc, boeuf, mouton...) y compris chez l'homme (S. JARD et al., Mol. Pharmacol., 1986, 30, 171-177 ; Y. ARSENIJEVIC et al., J. Endocrinol., 1994, 141, 383-391 ; J. SCHWARTZ et al., Endocrinology, 1991, 129 (2), 1107-1109 ; Y. DE KEYSER et al., FEBS Letters, 1994, 356, 215-220) où ils stimulent la libération d'hormone adrénocorticotrophique par l'AVP et potentialisent les effets du CRF sur la libération d'ACTH (G.E. GILLIES et al., Nature, 1982, 299, 355). Dans l'hypothalamus, les récepteurs $V_{1b}$ induisent aussi une libération directe de CRF (Neuroendocrinology, 1994, 60, 503-508) et sont, à ces divers titres, impliqués dans les situations de stress.

Ces récepteurs $V_{1b}$ ont été clonés chez le rat, l'homme et la souris (Y. DE KEYSER, FEBS Letters, 1994, 356, 215-220 ; T. SUGIMOTO et al., J. Biol. Chem., 1994, 269 (43), 27088-27092 ; M. SAITO et al., Biochem. Biophys. Res. Commun., 1995, 212 (3), 751-757 ; S.J. LOLAIT et al., Neurobiology, 1996, 92, 6783-6787 ; M.A. VENTURA et al., Journal of Molecular Endocrinology, 1999, 22, 251-260) et différentes études (hybridation *in situ,* PCR, de l'anglais Polymerase Chain Reaction, ...) révèlent une localisation ubiquitaire de ces récepteurs dans divers tissus centraux (cerveau, hypothalamus et adénohypophyse, en particulier) et périphériques (rein, pancréas, surrénales, coeur, poumons, intestin, estomac, foie, mésentère, vessie, thymus, rate, utérus, rétine, thyroïde...) et dans certaines tumeurs (hypophysaires, pulmonaires...) suggérant un rôle biologique et/ou pathologique étendu de ces récepteurs et une implication potentielle dans diverses maladies.

A titre d'exemples, chez le rat, des travaux ont montré que l'AVP via les récepteurs $V_{1b}$ régule le pancréas endocrine en stimulant la sécrétion d'insuline et de glucagon (B. LEE et al., Am. J. Physiol. 269 (Endocrinol. Metab. 32) : E1095-E1100, 1995) ou la production de catécholamines dans la medullo-surrénale qui est le siège d'une synthèse locale d'AVP (E. GRAZZINI et al., Endocrinology, 1996, 137 (a), 3906-3914). Ainsi, dans ce dernier tissu, l'AVP via ces récepteurs aurait un rôle crucial dans certains types de phéochromocytomes surrénaliens sécrétants de l'AVP et induisant de ce fait une production soutenue de catécholamines à l'origine d'hypertension résistantes aux antagonistes des récepteurs de l'angiotensine II et aux inhibiteurs de l'enzyme de conversion. Le cortex surrénalien est aussi riche en récepteurs $V_{1a}$ impliqués dans la production de gluco- et minéralo-corticoides (aldostérone et cortisol). Via ces récepteurs, l'AVP (circulante ou synthétisée localement) peut induire une production d'aldostérone avec une efficacité comparable à celle de l'angiotensine II (G. GUILLON et al., Endocrinology, 1995, 136 (3), 1285-1295). Le cortisol est un puissant régulateur de la production d'ACTH, l'hormone du stress.

De récents travaux ont aussi montré que les surrénales étaient capables de libérer directement du CRF et/ou de l'ACTH via l'activation des récepteurs $V_{1b}$ et/ou $V_{1a}$ portés par les cellules de la médulla (G. MAZZOCCHI et al., Peptides, 1997, 18 (2), 191-195 ; E. GRAZZINI et al., J. Clin. Endocrinol. Metab., 1999, 84 (6), 2195-2203).

Les récepteurs $V_{1b}$ sont aussi considérés comme un marqueur des tumeurs sécrétantes d'ACTH que sont certaines tumeurs pituitaires, certains carcinomes bronchiques (cancers pulmonaires à petites cellules ou SCLC, de l'anglais Small Cell Lung Cancers), pancréatiques, surrénaliens et thyroidiens, induisant un syndrome de Cushing dans certains cas (J. BERTHERAT et al., Eur. J. Endocrinol., 1996, 135,173 ; G.A. WITTERT et al., Lancet, 1990, 335, 991-994 ; G. DICKSTEIN et al., J. Clin. Endocrinol. Metab., 1996, 81 (8), 2934-2941). Les récepteurs $V_{1a}$ sont, quant à eux, un marqueur plus spécifique des cancers pulmonaires à petites cellules (SCLC) (P.J. WOLL et al., Biochem.

Biophys. Res. Commun., 1989, 164 (1), 66-73). Ainsi, les composés selon la présente invention sont des outils de diagnostic évidents et offrent une approche thérapeutique nouvelle dans la prolifération et la détection de ces tumeurs, à un stade même précoce (radiomarquage ; SPECT, de l'anglais Single Photon Emission Computed Tomography ; PET Scan, de l'anglais Positron Emission Tomography Scanner).

La présence abondante du messager des récepteurs $V_{1b}$ au niveau stomacal et intestinal, suggère une implication de l'AVP via ce récepteur sur la libération d'hormones gastrointestinales comme la cholécystokinine, la gastrine ou la sécrétine (T. SUGIMOTO et al., Molecular cloning and functional expression of $V_{1b}$ receptor gene, dans Neurohypophysis : Récent Progress of Vasopressin and Oxytocin Research ; T. SAITO, K. KUROKAWA and S. YOSHIDA ed., Elvesier Science, 1995, 409-413).

Des dérivés de 1,3-dihydro-2*H*-indol-2-one ont été décrits dans certaines demandes de brevet comme des ligands des récepteurs de l'arginine-vasopressine et/ou de l'ocytocine : on peut citer les demandes de brevets WO 93/15051, EP 636608, EP 636609, WO 95/18105, WO 97/15556 et WO 98/25901.

A ce jour, aucun composé non peptidique ayant une affinité et une sélectivité pour les récepteurs $V_{1b}$ ou à la fois pour les récepteurs $V_{1b}$ et $V_{1a}$ de l'arginine-vasopressine n'est connu.

On a maintenant trouvé de nouveaux dérivés de 1,3-dihydro-2*H*-indol-2-one qui présentent une affinité et une sélectivité pour les récepteurs $V_{1b}$ ou à la fois pour les récepteurs $V_{1b}$ et $V_{1a}$ de l'arginine-vasopressine.

Ces composés peuvent être utilisés pour la préparation de médicaments utiles dans le traitement ou la prévention de toute pathologie où l'arginine-vasopressine et/ou les récepteurs $V_{1b}$ ou à la fois les récepteurs $V_{1b}$ et les récepteurs $V_{1a}$ sont impliqués, notamment dans le traitement ou la prévention des affections du système cardiovasculaire, par exemple l'hypertension ; du système nerveux central, par exemple le stress, l'anxiété, la dépression, le trouble obsessionnel-compulsif, les attaques de panique ; du système rénal ; du système gastrique ainsi que dans le traitement des cancers pulmonaires à petites cellules ; de l'obésité ; du diabète de type II ; de la résistance à l'insuline ; de l'hypertriglycéridémie ; de l'athérosclérose ; du syndrome de Cushing ; de toutes pathologies consécutives au stress et des états de stress chroniques.

Ainsi, selon un de ses aspects, la présente invention a pour objet des composés de formule (Ia) sous forme d'isomère lévogyre :

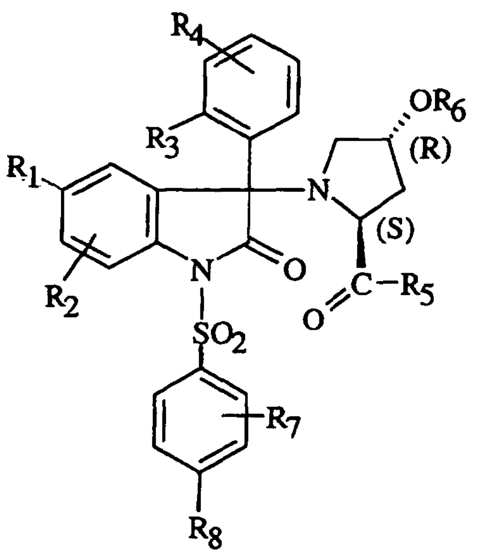

(Ia)

dans laquelle :

- $R_1$ représente un atome de chlore, un radical méthyle ou un radical trifluorométhoxy;
- $R_2$ représente un atome d'hydrogène ou est en position -6- de l'indól-2-one et représente un atome de chlore, un radical méthyle, un radical méthoxy ou un radical trifluorométhyle ;
- $R_3$ représente un atome de chlore, un atome de fluor, un radical méthoxy ou un radical éthoxy ;
- $R_4$ représente un atome d'hydrogène ou est en position -3- ou -4- du phényle et représente un atome de fluor ou un radical méthoxy ;
- ou bien $R_4$ est en position -3- du phényle et ensemble avec $R_3$ représentent un radical méthylènedioxy ;
- $R_5$ représente un radical diméthylamino ou un radical méthoxy ;
- $R_6$ représente un atome d'hydrogène ; un radical méthyle ; un radical éthyle ; un radical *tert*-butyloxycarbonylméthyle ; un radical carboxyméthyle ; un radical [[2-hydroxy-1-(hydroxyméthyl)-1-méthyléthyl]amino]carbonylméthyle ; un radical (1-pipérazinyl)carbonylméthyle ; un radical (4-morpholinyl)carbonylméthyle ; un radical 3-(4-morpholinyl)propanoyle ;
- $R_7$ est en position -2- du phényle et représente un radical méthoxy ;
- $R_8$ représente un radical méthoxy ;

ainsi que leurs sels avec des acides minéraux ou organiques, leurs solvats et/ou hydrates.

Les composés de formule (Ia) isomère lévogyre comprennent au moins 3 atomes de carbone asymétriques, l'atome de carbone portant le substituant $COR_5$ a la configuration (S), et l'atome de carbone portant le substituant $OR_6$ a la configuration (R).

Les sels sont en général préparés avec des acides pharmaceutiquement acceptables mais les sels d'autres acides utiles pour la purification ou l'isolement des composés de formule (Ia) font également partie de l'invention. Les sels des composés de formule (Ia) pharmaceutiquement acceptables sont par exemple le chlorhydrate, le bromhydrate, le sulfate, l'hydrogénosulfate, le dihydrogénophosphate, le méthanesulfonate, le benzènesulfonate, le naphtalènesulfonate, le para-toluènesulfonate, le maléate, le fumarate, le succinate, le citrate, l'acétate, le gluconate, l'oxalate.

Les composés suivants :

- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-méthoxy-N,N-diméthyl-2-pyrrolidinecarboxamide, isomère lévogyre ;
- (2S, 4R)-1-[5-Chloro-3-(2-chlorophényl)-1-[(2,4-diméthoxyphényl)sulfonyl]-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-3-(2-chlorophényl)-1-[(2,4-diméthoxyphényl)sulfonyl]-6-méthoxy-2-oxo-2,3-dihydro-1H-indol-3-yl)-4-méthoxy-N,N-diméthyl-2-pyrrolidinecarboxamide, isomère lévogyre ;
- (2S, 4R)-1-[5-Méthyl-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Trifluorométhoxy-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2-méthoxyphényl)-6-méthyl-2-oxo-2,3-dihydro-1*H*-indol-3-yl]-4-hydroxy-N,N-diméthyl-2-pyrrolidine carboxamide, isomère lévogyre ;
- (2S, 4R)-1-[3-(2-chlorophényl)-1-[(2,4-diméthoxyphényl)sulfonyl]-5,6-diméthyl-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2,3-diméthoxyphényl)-2-oxo-2,3-dihydro-1*H*-indol-3-yl]-4-méthoxy-N,N-diméthyl-2-pyrrolidinecarboxamide, isomère lévogyre;
- (2S, 4R)-1-[5-Chloro-1-[(2,4-diméthoxyphényl)sulfonyl]-3-(2-méthoxyphényl)-6-trifluorométhyl-2-oxo-2,3-dihydro-1*H*-indol-3-yl]-4-méthoxy-N,N-diméthyl-2-pyrrolidinecarboxamide, isomère lévogyre ;
- (2S, 4R)-1-[6-Chloro-1-[(2,4-diméthoxyphényl)sulfonyl]-3-(2-méthoxyphényl)-5-méthyl-2-oxo-2,3-dihydro-1*H*-indol-3-yl]-4-méthoxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-éthoxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2,3-diméthoxy phényl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5,6-Dichloro-3-(2-chlorophényl)-1-[(2,4-diméthoxyphényl)sulfonyl]-2-oxo-2,3-dihydro-1*H*-indol-3-yl]-4-hydroxy-N,N-diméthyl-2-pyrrolidinecarboxamide, isomère lévogyre ;
- Ester méthylique de l'acide (2S,4R)-1-[5-chloro-1-[(2,4-diméthoxyphényl) sulfonyl)-3-(2-méthoxyphényl)-2-oxo-2,3-dihydro-1*H*-indol-3-yl]-4-méthoxy-2-pyrrolidinecarboxylique, isomère lévogyre ;
- Ester méthylique de l'acide (2S, 4R)-1-[5-chloro-1-[(2,4-diméthoxyphényl) sulfonyl]-3-(2-méthoxyphényl)-6-méthyl-2-oxo-2,3-dihydro-1*H*-indol-3-yl]-4-méthoxy-2-pyrrolidinecarboxylique, isomère lévogyre ;
- (2S,4R)-1-[5-Chloro-1-[(2,4-diméthoxyphényl)sulfonyl]-3-(2-éthoxyphényl)-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2,3-difluorophényl)-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2,4-diméthoxy phényl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5-Chloro-1-[(2,4-diméthoxyphényl)sulfonyl]-3-(1,3-benzodioxol-4-yl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5,6-Dichloro-1-[(2,4-diméthoxyphényl)sulfonyl]-3-(2-méthoxy phényl)-2-oxo-2,3-dihydro-1*H*-indol-3-yl]-4-hydroxy-N,N-diméthyl-2-pyrrolidine carboxamide, isomère lévogyre;

- Ester *tert*-butylique de l'acide 2-[[(3R, 5S)-1-[5-chloro-1-[(2,4-diméthoxy phényl)sulfonyl]-3-(2-méthoxyphényl)-2-oxo-2,3-dihydro-1*H*-indol-3-yl ]-5-[(diméthyl amino)carbonyl]-3-pyrrolidinyl]oxy]acétique, isomère lévogyre ;
- Acide 2-[[(3R, 5S)-1-[5-chloro-1-[(2,4-diméthoxy phényl)sulfonyl]-3-(2-méthoxyphényl)-2-oxo-2,3-dihydro-1*H*-indol-3-yl]-5-[(diméthyl amino)carbonyl]-3-pyrrolidinyl]oxy]acétique, isomère lévogyre ;
- (2S, 4R)-1-[5-chloro-1-[(2,4-diméthoxyphényl)sulfonyl]-3-(2-méthoxyphényl)-2-oxo-2,3-dihydro-1*H*-indol-3-yl]-4-[2-[[2-hydroxy-1-(hydroxyméthyl)-1-méthyléthyl] amino]-2-oxoéthoxy]-N,N-diméthyl-2-pyrrolidinecarboxamide, isomère lévogyre ;
- (2S, 4R)-1-[5-chloro-1-[(2,4-diméthoxyphényl)sulfonyl]-3-(2-méthoxyphényl)-2-oxo-2,3-dihydro-1*H*-indol-3-yl]-N, N-diméthyl-4-[2-oxo-2-(1-pipérazinyl)éthoxy]-2-pyrrolidinecarboxamide, isomère lévogyre ;
- (2S, 4R)-1-[(2,4-diméthoxyphényl)sulfonyl]-3-(2-méthoxyphényl)-2-oxo-2,3-dihydro-1*H*-indol-3-yl]-N,N-diméthyl-4-[2-oxo-2-(4-morpholinyl)éthoxy]-2-pyrrolidinecarboxamide, isomère lévogyre ;
- 3-(4-Morpholinyl)propanoate de (3R, 5S)-1-[5-chloro-1-[(2,4-diméthoxyphényl) sulfonyl]-3-(2-méthoxyphényl)-2-oxo-2,3-dihydro-1*H*-indol-3-yl]-5-[(diméthylamino) carbonyl]-3-pyrrolidinyle, isomère lévogyre ;

ainsi que leurs sels éventuels avec des acides minéraux ou organiques, leurs solvats et/ou hydrates sont plus particulièrement préférés.

Le composé suivant :

- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidine carboxamide, isomère lévogyre ; est tout particulièrement préféré.

Selon un autre de ses aspects, la présente invention a pour objet un procédé de préparation des composés de formule (Ia) isomère lévogyre, de leurs sels éventuels avec des acides minéraux ou organiques, de leurs solvats et/ou leurs hydrates caractérisé en ce que :

on fait réagir, en présence d'une base, un composé de formule :

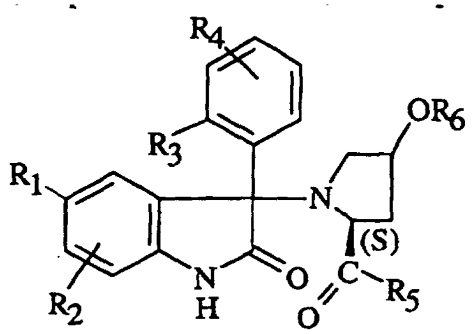

(II)

dans laquelle l'atome de carbone portant le substituant $OR_6$ a la configuration (R), $R_1$, $R_2$, $R_3$, $R_4$, $R_5$ et $R_6$ sont tels que définis pour un composé de formule (Ia) isomère lévogyre, avec un halogénure de formule :.

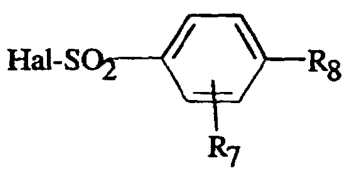

(III)

dans laquelle $R_7$ et $R_8$ sont tels que définis pour un composé de formule (Ia) isomère lévogyre et Hal représente un atome d'halogène.

Eventuellement, on transforme le composé de formule (Ia) isomère lévogyre en l'un de ses sels avec des acides minéraux ou organiques.

La réaction s'effectue en présence d'une base forte comme un hydrure métallique tel que l'hydrure de sodium ou un alcoolate alcalin tel que le *tert*-butylate de potassium, dans un solvant anhydre tel que le N,N-diméthylformamide ou le tétrahydrofurane et à une température comprise entre -70°C et +60°C. La réaction s'effectue de préférence en utilisant un composé de formule (III) dans laquelle Hal = Cl.

Un composé de formule (Ia) dans laquelle $R_6$ représente un méthyle ou un éthyle peut, également, se préparer par réaction d'un composé de formule (Ia) dans laquelle $R_6$ représente l'hydrogène avec un halogénure de méthyle

ou d'éthyle, en présence d'une base telle qu'un hydrure métallique, dans un solvant inerte tel que le N,N-diméthylformamide ou le tétrahydrofurane selon les méthodes classiques.

Un composé de formule (Ia) dans laquelle $R_6$ représente un groupe $-CH_2COOH$ se prépare préférentiellement par hydrolyse d'un composé de formule (Ia) dans laquelle $R_6$ représente un groupe $-CH_2COOC(CH_3)_3$, en milieu acide, en utilisant un acide fort tel que l'acide trifluoroacétique ou l'acide chlorhydrique dans un solvant tel que le dichlorométhane ou le dioxane et à une température comprise entre 0°C et la température ambiante.

Un composé de formule (Ia) dans laquelle $R_6$ représente un groupe [[2-hydroxy-1-(hydroxyméthyl)-1-méthyléthyl]amino]carbonylméthyle, un groupe (1-pipérazinyl) carbonylméthyle ou un groupe (4-morpholinyl)carbonylméthyle se prépare préférentiellement par réaction d'un composé de formule (Ia) dans laquelle $R_6$ représente un groupe $-CH_2COOH$ avec le 2-amino-2-méthyl-1,3-propanediol, la pipérazine ou la morpholine selon les méthodes classiques du couplage peptidique.

Les composés de formule (Ia) isomère lévogyre ainsi obtenus peuvent être ultérieurement séparés du milieu réactionnel et purifiés selon les méthodes classiques, par exemple par cristallisation ou chromatographie.

Les composés de formule (Ia) isomère lévogyre ainsi obtenus sont isolés sous forme de base libre ou de sel, selon les techniques classiques.

Lorsque les composés de formule (Ia) isomère lévogyre sont obtenus sous forme de base libre, la salification est effectuée par traitement avec l'acide choisi dans un solvant organique. Par traitement de la base libre, dissoute par exemple dans un éther tel que l'éther diéthylique ou dans un alcool tel que le propan-2-ol ou dans l'acétone ou dans le dichlorométhane, ou dans l'acétate d'éthyle ou dans l'acétonitrile avec une solution de l'acide choisi dans un des solvants précités, on obtient le sel correspondant qui est isolé selon les techniques classiques.

Ainsi, on prépare par exemple le chlorhydrate, le bromhydrate, le sulfate, le trifluoroacétate, l'hydrogénosulfate, le dihydrogénophosphate, le méthanesulfonate, l'oxalate, le maléate, le succinate, le fumarate, le naphtalène-2-sulfonate, le benzènesulfonate, le para-toluènesulfonate, le gluconate, le citrate, l'acétate.

A la fin de la réaction, les composés de formule (Ia) isomère lévogyre peuvent être isolés sous forme d'un de leurs sels, par exemple le chlorhydrate, ou l'oxalate ; dans ce cas, s'il est nécessaire, la base libre peut être préparée par neutralisation dudit sel avec une base minérale ou organique, telle que l'hydroxyde de sodium ou la triéthylamine ou avec un carbonate ou bicarbonate alcalin, tel que le carbonate ou bicarbonate de sodium ou de potassium.

Les composés de formule (II) se préparent par réaction d'un composé 3-halogéno-1,3-dihydro-2H-indol-2-one de formule :

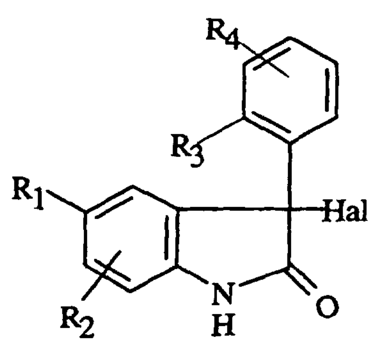

(IV)

dans laquelle $R_1$, $R_2$, $R_3$ et $R_4$ sont tels que définis pour un composé de formule (Ia) isomère lévogyre et Hal représente un atome d'halogène, de préférence le chlore ou le brome, avec un composé de formule :

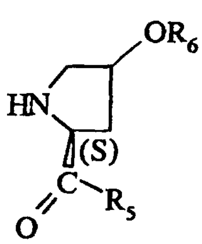

(V)

dans laquelle l'atome de carbone portant le substituant $OR_6$ a la configuration (R), $R_5$ et $R_6$ sont tels que définis pour un composé de formule (Ia) isomère lévogyre. La réaction s'effectue en présence d'une base telle que la diisopropyléthylamine ou la triéthylamine, dans un solvant inerte tel que le dichlorométhane ou le tétrahydrofurane ou un mélange

de ces solvants et à une température comprise entre la température ambiante et la température de reflux du solvant.

Les composés de formule (III) sont connus ou préparés par des méthodes connues telles que celles décrites dans EP-0 469 984 B et WO 95/18105. Par exemple, les composés de formule (III) peuvent être préparés par halogénation des acides benzènesulfoniques correspondants ou de leurs sels, par exemple de leurs sels de sodium ou de potassium. La réaction s'effectue en présence d'un agent halogénant tel que l'oxychlorure de phosphore, le chlorure de thionyle, le trichlorure de phosphore, le tribromure de phosphore ou le pentachlorure de phosphore, sans solvant ou dans un solvant inerte tel qu'un hydrocarbure halogéné ou le N,N-diméthylformamide et à une température comprise entre -10°C et 200°C.

Le chlorure de 2,4-diméthoxybenzènesulfonyle est préparé selon J. Am. Chem. Soc.,1952, 74, 2008.

Les composés de formule (IV) sont connus et se préparent selon des méthodes connues telles que celles décrites dans WO 95/18105.

Par exemple, on transforme un composé de formule :

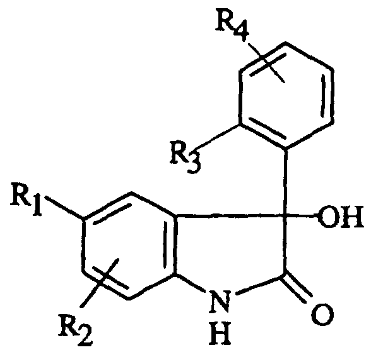

(VI)

dans laquelle $R_1$, $R_2$, $R_3$ et $R_4$ sont tels que définis pour un composé de formule (Ia) isomère lévogyre, en un composé de formule (IV) dans laquelle Hal = Cl par action du chlorure de thionyle en présence d'une base telle que la pyridine, dans un solvant inerte tel que le dichlorométhane et à une température comprise entre 0°C et la température ambiante.

Selon un autre exemple de préparation des composés de formule (IV), on transforme un composé de formule :

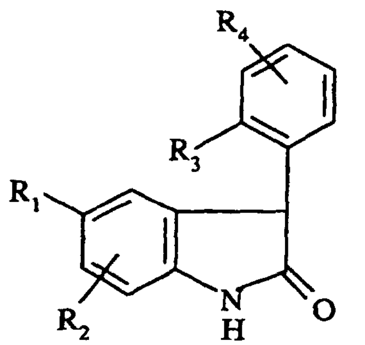

(VII)

dans laquelle $R_1$, $R_2$, $R_3$ et $R_4$ sont tels que définis pour un composé de formule (Ia) isomère lévogyre, en un composé de formule (IV) au moyen d'un agent d'halogénation comme le brome selon le procédé décrit dans Farm. Zh.(Kiev), 1976,5,30-33.

Les composés de formule (VI) sont connus et se préparent selon des méthodes connues telles que celles décrites dans WO 95/18105.

Par exemple, on prépare un composé de formule (VI) par réaction d'un dérivé de 1*H*-indole-2,3-dione de formule:

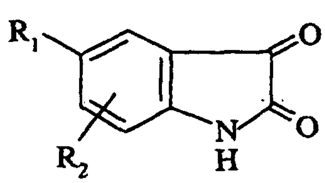

(VIII)

dans laquelle $R_1$ et $R_2$ sont tels que définis pour un composé de formule (Ia) isomère lévogyre, avec un dérivé organomagnésien de formule :

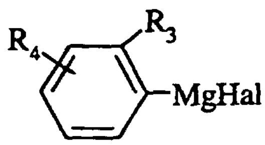 (IX)

dans laquelle $R_3$ et $R_4$ sont tels que définis pour un composé de formule (Ia) isomère lévogyre et Hal représente un atome d'halogène, de préférence le brome ou l'iode, dans un solvant inerte tel que le tétrahydrofurane ou l'éther diéthylique.

On peut également préparer un composé de formule (VI) dans laquelle $R_3$ est tel que défini pour un composé de formule (Ia) isomère lévogyre et $R_4$, différent de l'hydrogène, est en position -3- du phényle, par réaction d'un composé de formule :

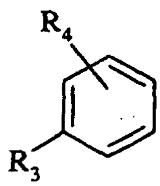 (XVII)

dans laquelle $R_3$ est tel que défini pour un composé de formule (Ia) isomère lévogyre et $R_4$ est en position -2- du phényle, avec un dérivé du lithium tel que le n-butyllithium, puis l'intermédiaire lithié ainsi obtenu est mis en réaction avec un composé de formule (VIII]. La réaction s'effectue dans un solvant tel que l'éther diéthylique, le tétrahydrofurane, l'hexane ou un mélange de ces solvants, à une température comprise entre -70°C et la température ambiante.

Les dérivés de 1*H*-indole-2,3-dione (VIII) sont commerciaux ou se préparent selon les méthodes décrites dans Tetrahedron Letters, 1998, 39, 7679-7682 ; Tetrahedron Letters, 1994, 35, 7303-7306 ; J. Org. Chem., 1977, 42 (8), 1344-1348 ; J. Org. Chem., 1952, 17, 149-156 ; J. Am. Chem. Soc., 1946, 68, 2697-2703 ; Organic Syntheses, 1925, V, 71-74 et Advances in Heterocyclic Chemistry, A.R. Katritzky and A.J. Boulton, Academic Press, New-York, 1975, 18, 2-58.

Les dérivés organomagnésien (IX) sont préparés selon les méthodes classiques bien connues de l'homme de l'art.

Les composés de formule (XVII) sont connus ou préparés selon des méthodes connues.

On peut également préparer un composé de formule (VI), par oxydation par l'air d'un composé de formule (VII) en présence d'une base telle que l'hydrure de sodium et en présence de diméthyldisulfure.

De façon particulière, on peut préparer les composés de formule (VI) dans laquelle $R_3$ = ($C_1$-$C_2$)alcoxy et $R_4$ = H, ou bien $R_3$ = $R_4$ = méthoxy avec $R_4$ en position -3 du phényle, $R_2$ est différent d'un atome de chlore et $R_1$ est tel que défini pour un composé de formule (Ia) isomère lévogyre, en suivant le procédé décrit dans le SCHEMA 1.

SCHEMA 1

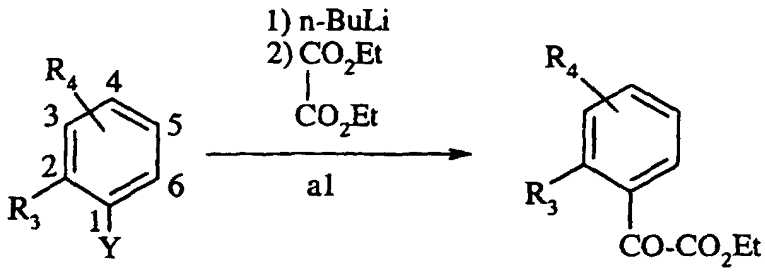


(XI)

(X) : $R_3$ = ($C_1$-$C_2$)alcoxy, $R_4$ = H ;
$R_3$ = $R_4$ = méthoxy avec
$R_4$ en position -3 ;
Y = H ou Br.

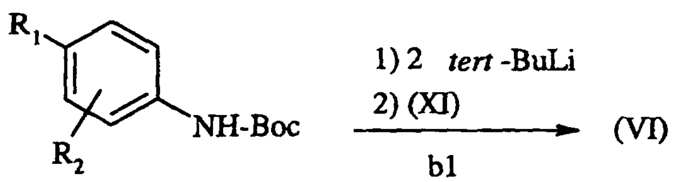


(XII)

A l'étape a1 du SCHEMA 1, on fait d'abord réagir un composé de formule (X) avec un dérivé du lithium tel que le n-butyllithium, en l'absence ou en présence d'une base telle que le N,N,N',N'-tétraméthylènediamine, puis l'intermédiaire lithié ainsi obtenu est mis en réaction avec l'oxalate de diéthyle pour donner le composé de formule (XI). La réaction s'effectue dans un solvant inerte tel que l'éther diéthylique, le tétrahydrofurane, l'hexane ou un mélange de ces solvants et à une température comprise entre -70°C et la température ambiante.

A l'étape b1, on fait d'abord réagir un composé de formule (XII) avec deux équivalents d'un dérivé du lithium tel que le *tert*-butyllithium, puis l'intermédiaire lithié ainsi obtenu est mis en réaction avec le composé de formule (XI) pour donner le composé de formule (VI) attendu. La réaction s'effectue dans un solvant inerte tel que l'éther diéthylique, le tétrahydrofurane, le pentane ou un mélange de ces solvants et à une température comprise entre -70°C et la température ambiante.

Les composés de formule (X) sont commerciaux ou synthétisés de manière classique.

Les composés de formule (XII) sont préparés par réaction des dérivés de l'aniline correspondants avec du di-*tert*-butyldicarbonate selon les méthodes classiques.

Les composés de formule (VII) sont connus et se préparent selon des méthodes connues telles que celles décrites dans WO 95/18105 ou dans J. Org. Chem., 1968, 33, 1640-1643.

Les composés de formule (V) dans laquelle $R_5$ représente un méthoxy et $R_6$ = H sont commerciaux.

Les composés de formule (V) dans laquelle $R_5$ représente un méthoxy et $R_6$ = méthyle ou éthyle sont connus ou se préparent selon des méthodes connues telles que celles décrites dans J. Med. Chem., 1988, 31, 875-885 à partir de l'acide (2S, 4R)-4-hydroxypyrrolidine-2-carboxylique protégé sur l'atome d'azote de la pyrrolidine.

Les composés de formule (V) dans laquelle $R_5$ est un groupe diméthylamino et $R_6$ = H ou ($C_1$-$C_2$)alkyle se préparent selon le SCHEMA 2 ci-après dans lequel Pr représente un groupe N-protecteur, en particulier, le benzyloxycarbonyle ou le *tert*-butoxycarbonyle.

SCHEMA 2

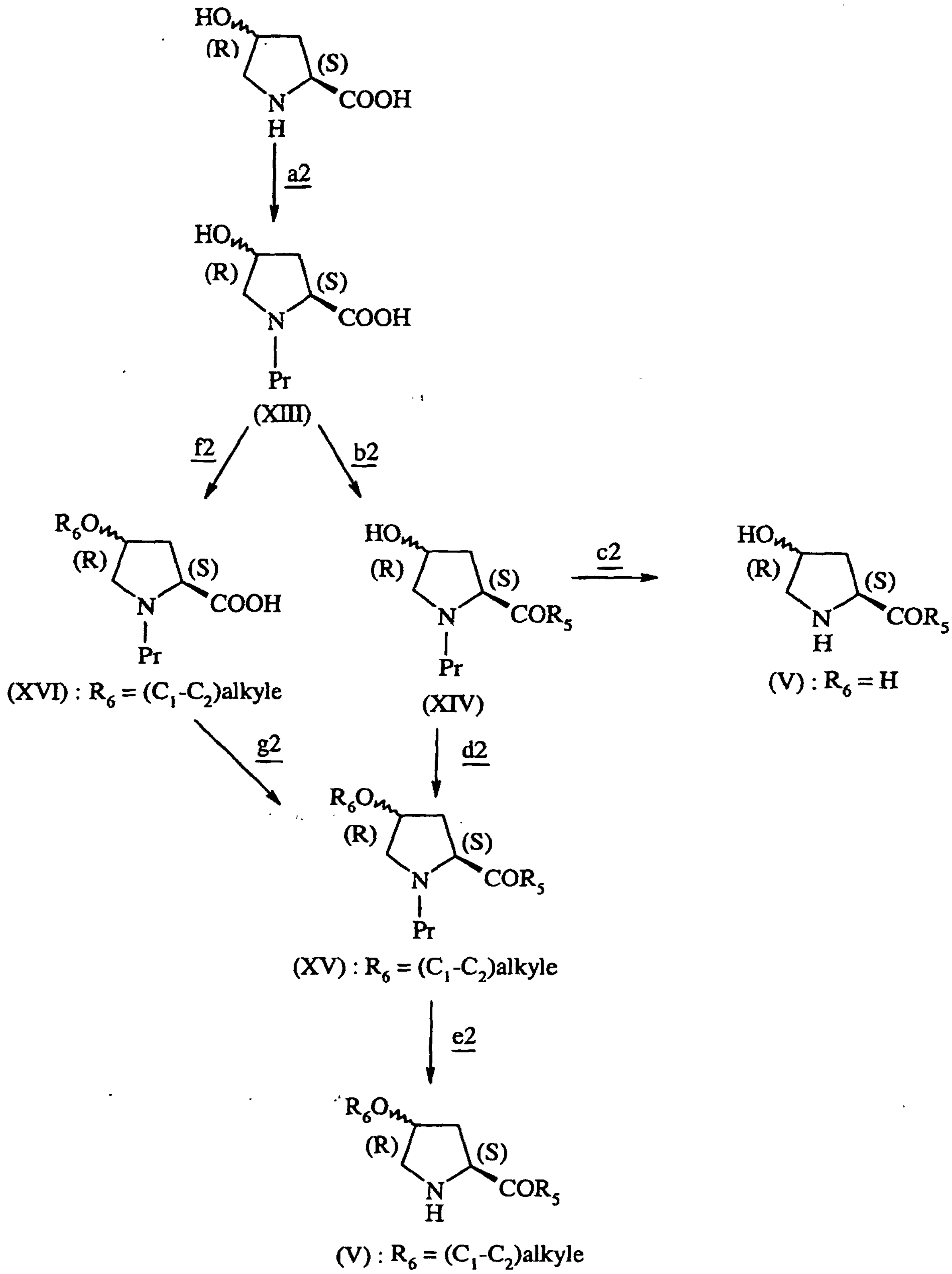


(XIII)

(XVI) : $R_6$ = $(C_1-C_2)$alkyle

(XIV)

(V) : $R_6$ = H

(XV) : $R_6$ = $(C_1-C_2)$alkyle

(V) : $R_6$ = $(C_1-C_2)$alkyle

A l'étape a2 du SCHEMA 2, on protège l'atome d'azote de la 4(R)-hydroxy-(S)-proline selon les méthodes classiques pour obtenir un composé de formule (XIII).

L'acide (XIII) est mis en réaction à l'étape b2 avec la diméthylamine selon les méthodes classiques du couplage peptidique pour donner le composé (XIV), qui est déprotégé, selon les méthodes connues, pour donner un composé de formule (V) dans laquelle $R_6$ = H.

A l'étape d2, le composé (XIV) peut être mis en réaction avec un halogénure de ($C_1$-$C_2$)alkyle, en présence d'une base telle qu'un hydrure métallique ou un carbonate alcalin ou alcalino-terreux comme $K_2CO_3$ ou $Cs_2CO_3$, dans un solvant inerte tel que le tétrahydrofurane ou le N,N-diméthylformamide et à une température comprise entre 0°C et la température de reflux du solvant pour donner un composé (XV).

On peut également effectuer la réaction d'un composé (XIV) avec un halogénure de ($C_1$-$C_2$)alkyle dans des conditions de catalyse par transfert de phase, en présence d'une base telle qu'un hydroxyde de métal alcalin, l'hydroxyde de sodium par exemple, et d'un catalyseur de transfert de phase tel qu'un sel d'ammonium quaternaire substitué, le tétrabutylammonium hydrogène sulfate par exemple, dans un solvant inerte tel que le dichlorométhane ou le benzène en mélange avec de l'eau.

Par déprotection du groupe N-protecteur du composé (XV) on obtient à l'étape e2 les composés de formule (V) dans laquelle $R_6$ = ($C_1$-$C_2$)alkyle.

Alternativement, à l'étape f2, on alkyle l'hydroxy du composé (XIII) par réaction avec un halogénure de ($C_1$-$C_2$) alkyle dans les conditions de l'étape d2, et l'acide (XVI) ainsi obtenu est mis en réaction à l'étape g2 avec la diméthylamine selon les méthodes classiques du couplage peptidique pour donner le composé (XV).

Les acides (2S,4R)-4-hydroxypyrrolidine-2-carboxylique sont commerciaux.

Les composés de formule (V) dans laquelle $R_5$ représente un groupe diméthylamino ou un méthoxy et $R_6$ = $-CH_2COOC(CH_3)_3$ se préparent selon le SCHEMA 3 ci-après dans lequel Pr représente un groupe N-protecteur, en particulier le benzyloxycarbonyle ou le *tert*-butoxycarbonyle.

SCHEMA 3

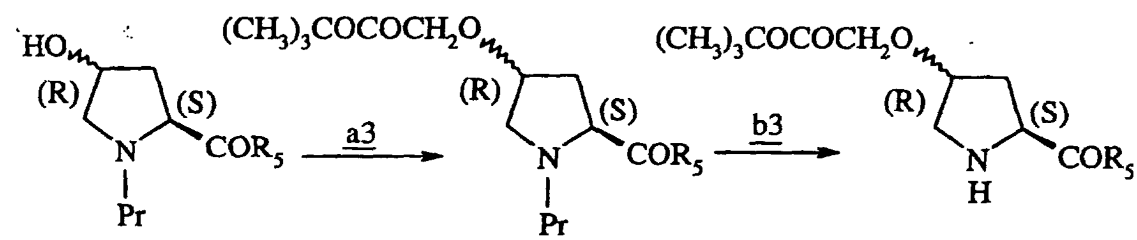


(XVIII) (XIX) (V)

A l'étape a3 du SCHEMA 3 on fait réagir un composé de formule (XVIII), préparé comme précédemment décrit, avec un composé de formule Hal-$CH_2$-$COOC(CH_3)_3$ dans laquelle Hal réprésente un atome d'halogène, de préférence le chlore ou le brome. La réaction s'effectue dans les conditions précédemment décrites à l'étape d2 du SCHEMA 2 et on obtient un composé (XIX).

Par déprotection du groupe N-protecteur du composé (XIX) on obtient à l'étape b3 les composés (V) attendus.

Les composés de formule (V) dans laquelle $R_5$ est tel que défini pour un composé de formule (Ia) et $R_6$ = $-CH_2$-CO-OH se préparent par hydrolyse acide d'un composé de formule (XIX) dans laquelle Pr représente un benzyloxycarbonyle. La réaction s'effectue en utilisant un acide fort tel que l'acide trifluoroacétique ou l'acide chlorhydrique dans un solvant tel que le dichlorométhane ou le dioxane et à une température comprise entre 0°C et la température ambiante. Par déprotection du groupe N-protecteur selon les méthodes classiques on obtient les composés (V) attendus.

Les composés de formule (V) dans laquelle $R_5$ est tel que défini pour un composé de formule (Ia) et $R_6$ représente un groupe [[2-hydroxy-1-(hydroxyméthyl)-1-méthyléthyl]amino]carbonylméthyle, un groupe (1-pipérazinyl) carbonylméthyle ou un groupe (4-morpholinyl)carbonylméthyle se préparent par réaction d'un composé correspondant dans lequel $R_6$ représente un groupe $-CH_2COOH$ et protégé sur l'atome d'azote de la pyrrolidine, avec le 2-amino-2-méthyl-1,3-propanediol, la pipérazine ou la morpholine selon les méthodes classiques du couplage peptidique.

Par déprotection du groupe N-protecteur selon les méthodes classiques on obtient les composés (V) attendus.

Les composés de formule (V) dans laquelle $R_5$ représente un groupe diméthylamino ou un méthoxy et $R_6$ représente un groupe 3-(4-morpholinyl)propanoyle se préparent selon le SCHEMA 4 ci-après dans lequel Pr représente un groupe N-protecteur, en particulier le benzyloxycarbonyle ou le *tert*-butoxycarbonyle.

SCHEMA 4

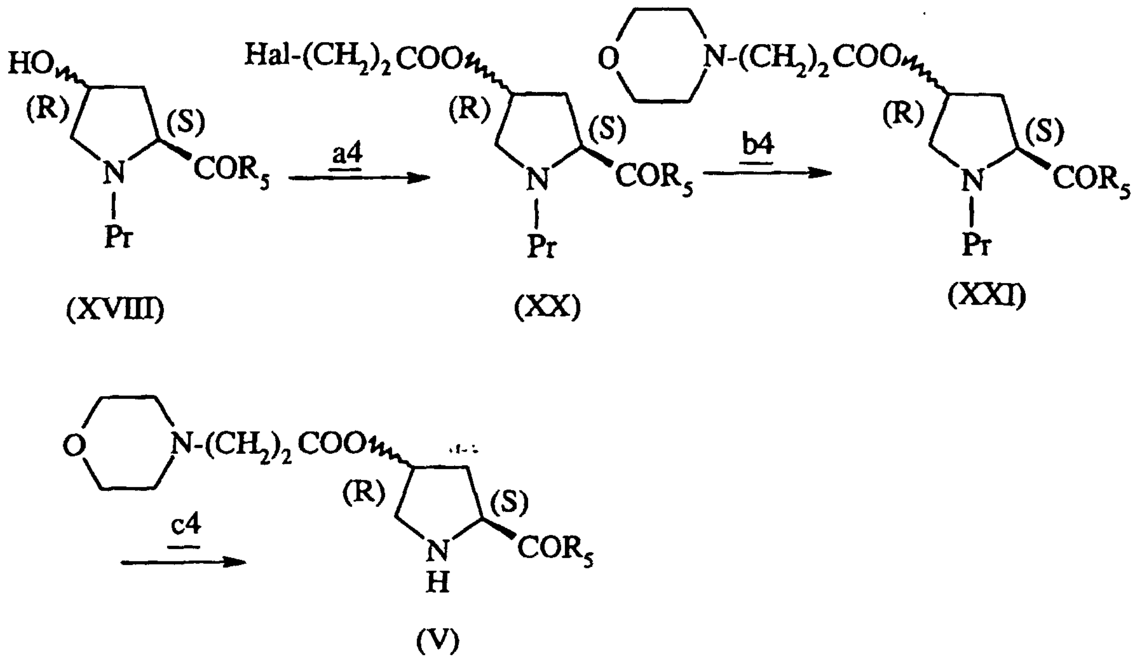


(XVIII) (XX) (XXI) (V)

A l'étape a4 du SCHEMA 4 on fait réagir un composé de formule (XVIII) avec un composé de formule Hal-CO-$(CH_2)_2$-Hal' dans laquelle Hal et Hal' représentent chacun indépendamment un atome d'halogène, de préférence le chlore ou le brome. La réaction s'effectue en présence d'une base telle que la triéthylamine ou la diisopropyléthylamine, dans un solvant tel que le dichlorométhane ou le tétrahydrofurane et à une température comprise entre 0°C et la température de reflux du solvant.

A l'étape b4, la réaction du composé de formule (XX) ainsi obtenu avec la morpholine permet d'obtenir un composé de formule (XXI). La réaction s'effectue en présence d'une base telle que la triéthylamine ou la N,N-diisopropyléthylamine, ou en utilisant un excès de morpholine, dans un solvant tel que le dichlorométhane ou le tétrahydrofurane et à une température comprise entre 0°C et la température de reflux du solvant.

Par déprotection du groupe N-protecteur du composé (XXI) on obtient à l'étape c4, le composé de formule (V) attendu.

De façon particulière, on peut également préparer un composé de formule (V) dans laquelle $R_6$ représente un groupe 3-(4-morpholinyl)propanoyle selon le SCHEMA 5 ci-après dans lequel Pr représente un groupe N-protecteur, en particulier le benzyloxycarbonyle ou le *tert*-butoxycarbonyle.

SCHEMA 5

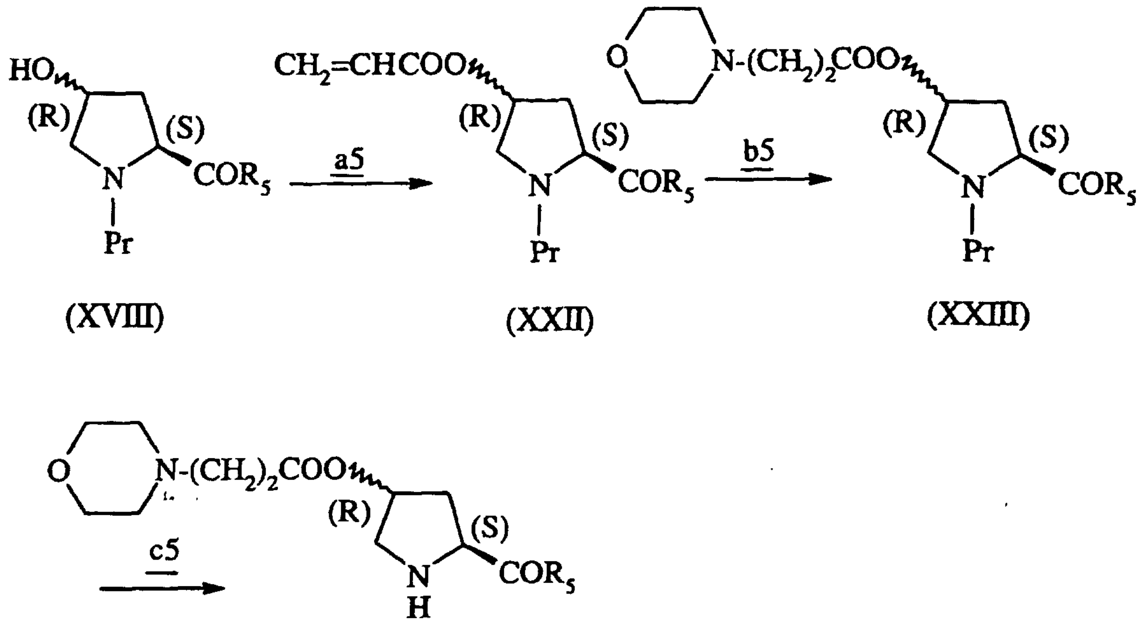


(XVIII) (XXII) (XXIII)

(V)

A l'étape a5 du SCHEMA 5 on fait réagir un composé de formule (XVIII) avec le chlorure d'acryloyle, dans les conditions précédemment décrites à l'étape a4 du SCHEMA 4, et obtient le composé de formule (XXII).

A l'étape b5, la réaction du composé (XXII) avec la morpholine permet d'obtenir un composé de formule (XXIII). La réaction s'effectue en présente de chlorure ferrique, dans un solvant tel que le dichlorométhane et à une température comprise entre la température ambiante et la température de reflux du solvant.

Par déprotection du groupe N-protecteur du composé (XXIII) on obtient à l'étape c5, le composé de formule (V) attendu.

Lorsque l'on souhaite préparer un composé de formule (Ia) optiquement pur, on fait réagir, de préférence, un composé de formule (II) optiquement pur avec un composé de formule (III) selon le procédé de l'invention.

Les composés de formule (II) optiquement purs se préparent par réaction du composé de formule (IV) racémique avec un composé de formule (V) optiquement pur, puis séparation du mélange des diastéréoisomères selon les méthodes classiques, par exemple par cristallisation ou chromatographie.

Alternativement, on peut faire réagir le mélange des diastéréoisomères du composé de formule (II) avec le composé de formule (III) et séparer le mélange des diastéréoisomères du composé de formule (Ia) ainsi obtenu.

Au cours de l'une quelconque des étapes de préparation des composés de formule (Ia) ou des composés intermédiaires de formule (II), (IV), (V) ou (VI), il peut être nécessaire et/ou souhaitable de protéger les groupes fonctionnels réactifs ou sensibles, tels que les groupes amine, hydroxyle ou carboxy, présents sur l'une quelconque des molécules concernées. Cette protection peut s'effectuer en utilisant les groupes protecteurs conventionnels, tels que ceux décrits dans Protective Groups in Organic Chemistry, J.F.W. McOmie, Ed. Plenum Press, 1973, dans Protective Groups in Organic Synthesis, T.W. Greene et P.G.M. Wutts, Ed. John Wiley et sons, 1991 ou dans Protecting Groups, Kocienski P.J., 1994, Georg Thieme Verlag. L'élimination des groupes protecteurs peut s'effectuer à une étape ultérieure opportune en utilisant les méthodes connues de l'homme de l'art et qui n'affectent pas le reste de la molécule concernée.

Les groupes N-protecteurs éventuellement utilisés sont les groupes N-protecteurs classiques bien connus de l'homme de l'art tels que par exemple le groupe *tert*-butoxycarbonyle, fluorénylméthoxycarbonyle, benzyle, benzhydrylidène ou benzyloxycarbonyle.

Les composés de formule (II) sont nouveaux et font partie de l'invention.

Ainsi, selon un autre de ses aspects, l'invention a pour objet des composés de formule:

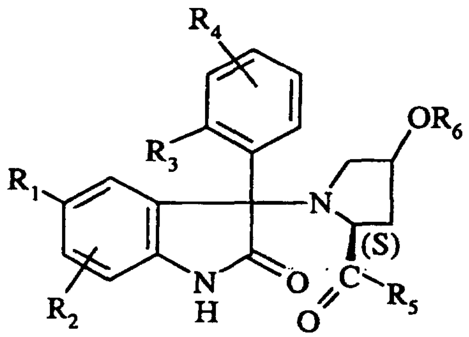

(II)

dans laquelle:

- l'atome de carbone portant le substituant $-OR_6$ a la configuration (R) ;
- $R_1$, $R_2$, $R_3$, $R_4$, $R_5$ et $R_6$ sont tels que définis pour un composé de formule (Ia) isomère lévogyre;

ainsi que leurs sels avec des acides minéraux ou organiques, sous forme d'isomères optiquement purs ou sous forme de mélange de diastéréoisomères.

Les sels des composés de formule (II) comprennent ceux avec des acides minéraux ou organiques qui permettent une séparation ou une cristallisation convenable des composés de formule (II) tels que le chlorhydrate, le bromhydrate, l'oxalate, le maléate, le succinate, le fumarate, le citrate, l'acétate.

Les composés de formule (Ia) isomère lévogyre ci-dessus comprennent également ceux dans lesquels un ou plusieurs atomes d'hydrogène, ou de carbone ont été remplacés par leur isotope radioactif par exemple le tritium, ou le carbone-14. De tels composés marqués sont utiles dans des travaux de recherche, de métabolisme ou de pharmacocinétique, dans des essais biochimiques en tant que ligand de récepteurs.

Les composés selon l'invention ont fait l'objet d'études biochimiques.

L'affinité des composés de formule (Ia) isomère lévogyre selon l'invention pour les récepteurs $V_{1b}$ de l'arginine-vasopressine a été déterminée *in vitro* en utilisant la méthode décrite par Y. DE KEYSER et al., Febs Letters, 1994, 356, 215-220. Cette méthode consiste à étudier in *vitro* le déplacement de l'arginine-vasopressine tritiée (($^3$H] -AVP) aux récepteurs $V_{1b}$ présents sur des préparations membranaires adénohypophysaires ou cellulaires portant les récepteurs $V_{1b}$ de rat ou humains. Les concentrations inhibitrices de 50 % ($CI_{50}$) de la fixation de l'arginine-vasopressine tritiée des composés selon l'invention sont faibles et varient de $10^{-6}$ à $10^{-9}$ M, plus particulièrement de $10^{-7}$ à $10^{-9}$M.

L'affinité des composés de formule (Ia) isomère lévogyre selon l'invention pour les récepteurs $V_{1a}$ de l'arginine-vasopressine a été déterminée *in vitro* en utilisant la méthode décrite par M. THIBONNIER et al., J. Biol. Chem., 1994, 269, 3304-3310. Cette méthode consiste à étudier in *vitro* le déplacement de l'arginine-vasopressine tritiée ([$^3$H] -AVP) aux récepteurs $V_{1a}$ présents sur des préparations membranaires ou cellulaires portant les récepteurs $V_{1a}$ de rat ou humains. Parmi les composé de formule (Ia) isomère lévogyre, certains présentent aussi une affinité pour les récepteurs $V_{1a}$ de l'arginine-vasopressine avec des $CI_{50}$ qui varient de $10^{-6}$ à $10^{-9}$M, plus particulièrement de $10^{-7}$ à $10^{-8}$ M.

L'affinité des composés de formule (Ia) isomère lévogyre selon l'invention pour les récepteurs $V_2$ de la vasopressine a également été étudiée (méthode décrite par M. Birnbaumer et al., Nature (Lond.), 1992, 357, 333-335). Les composés étudiés sont peu ou pas affins pour les récepteurs $V_2$.

Les composés de la présente invention sont notamment des principes actifs de compositions pharmaceutiques, dont la toxicité est compatible avec leur utilisation en tant que médicaments.

Selon un autre de ses aspects, la présente invention concerne l'utilisation des composés de formule (Ia) isomère lévogyre, ou l'un de leurs sels, solvats et/ou hydrates pharmaceutiquement acceptables, pour la préparation de médicaments destinés à traiter toute pathologie où l'arginine-vasopressine et/ou ses récepteurs $V_{1b}$ ou à la fois ses récepteurs $V_{1b}$ et ses récepteurs $V_{1a}$ sont impliqués.

Selon un autre de ses aspects, la présente invention concerne l'utilisation des composés de formule (Ia), isomère lévogyre ou l'un de leurs sels, solvats et/ou hydrates pharmaceutiquement acceptables pour la préparation de médicaments destinés à traiter les pathologies du système cardiovasculaire, du système nerveux central, du système rénal, du système gastrique ainsi que les cancers du poumon à petites cellules, l'obésité, le diabète de type II, la résistance à l'insuline, l'hypertriglycéridémie, l'athérosclérose, le syndrome de Cushing, toutes pathologies consécutives au stress et les états de stress chroniques.

Ainsi les composés selon l'invention peuvent être utilisés, chez l'homme ou chez l'animal, dans le traitement ou la prévention de différentes affections vasopressine-dépendantes tels que les affections cardiovasculaires, comme l'hypertension, l'hypertension pulmonaire, l'insuffisance cardiaque, l'infarctus du myocarde, ou le vasospasme coronaire, en particulier chez le fumeur, la maladie de Raynaud, les angines instables et PTCA (de l'anglais percutaneous transluminal coronary angioplasty), l'ischémie cardiaque, les dérèglements de l'hémostase ; les affections du système nerveux central comme la migraine, le vasospasme cérébral, l'hémorragie cérébrale, les oedèmes cérébraux, la dépression, l'anxiété, le stress, le trouble obsessionnel-compulsif, les attaques de panique, les états psychotiques, les troubles de la mémoire par exemple ; les affections du système rénal comme le vasospasme rénal, la nécrose du cortex rénal, le diabète insipide néphrogénique ; les affections du système gastrique comme le vasospasme gastrique, l'hépatocirrhose, les ulcères, la pathologie des vomissements, par exemple la nausée y compris la nausée due à une chimiothérapie, le mal des transports ; la néphropathie diabétique. Les composés selon l'invention peuvent également être utilisés dans le traitement des troubles du comportement sexuel ; chez la femme, les composés selon l'invention, peuvent être utilisés pour traiter la dysménorrhée ou le travail prématuré. On peut également utiliser les composés selon l'invention dans le traitement des cancers pulmonaires à petites cellules ; des encéphalopathies hyponatrémiques ; du syndrome pulmonaire, de la maladie de Menière ; du glaucome, de la cataracte ; de l'obésité ; du diabète de type II ; de l'athérosclérose ; du syndrome de Cushing ; de la résistance à l'insuline ; de l'hypertriglycéridémie ; dans les traitements post-opératoires, notamment après une chirurgie abdominale.

Les composés selon l'invention peuvent aussi être utilisés dans le traitement ou la prévention de toutes les pathologies consécutives au stress comme la fatigue et ses syndrômes, les désordres ACTH dépendants, les troubles cardiaques, la douleur, les modifications de la vidange gastrique, de l'excrétion fécale (colite, syndrôme du colon irritable, maladie de Crohn), de la sécrétion acide, l'hyperglycémie, l'immunosuppression, les processus inflammatoires (arthrite rhumatoïde et ostéoarthrite), les infections multiples, les cancers, l'asthme, le psoriasis, les allergies et les désordres neuropsychiatriques variés tel que l'anorexie nerveuse, la boulimie, les troubles de l'humeur, la dépression, l'anxiété, les troubles du sommeil, les états de panique, les phobies, l'obsession, les troubles de la perception de la douleur (fibromyalgie), les maladies neurodégénératrices (maladie d'Alzheimer, maladie de Parkinson, maladie d'Huntington), la dépendance à une substance, le stress hémorragique, les spasmes musculaires, l'hypoglycémie. Les composés selon l'invention peuvent également être utilisés dans le traitement ou la prévention des états de stress chronique comme l'immunodépression, les troubles de la fertilité, les dysfonctionnements de l'axe hypothalamo-hypophysorurrénalien.

Les composés selon l'invention peuvent également être utilisés comme psychostimulants, provoquant l'augmentation de l'éveil, la réactivité émotionnelle face à l'environnement et facilitant l'adaptation.

Les composés de formule (Ia) isomère lévogyre ci-dessus, ou l'un de leurs sels, solvats et/ou hydrates pharmaceutiquement acceptables peuvent être utilisés à des doses journalières de 0,01 à 100 mg par kilo de poids corporel du mammifère à traiter, de préférence à des doses journalières de 0,1 à 50 mg/kg. Chez l'être humain, la dose peut varier de préférence de 0,1 à 4000 mg par jour, plus particulièrement de 0,5 à 1000 mg selon l'âge du sujet à traiter ou le type de traitement : prophylactique ou curatif.

Pour leur utilisation comme médicaments, les composés de formule (Ia) isomère lévogyre sont généralement administrés en unités de dosage. Lesdites unités de dosage sont de préférence formulées dans des compositions pharmaceutiques dans lesquelles le principe actif est mélangé avec un ou plusieurs excipients pharmaceutiques.

Ainsi, selon un autre de ses aspects, la présente invention concerne des compositions pharmaceutiques renfermant, en tant que principe actif, un composé de formule (Ia) isomère lévogyre ou l'un de ses sels, solvats et/ou hydrates pharmaceutiquement acceptables.

Dans les compositions pharmaceutiques de la présente invention pour l'administration par voie orale, sublinguale, inhalée, sous-cutanée, intramusculaire, intraveineuse, transdermique, locale ou rectale, les principes actifs peuvent être administrés sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intraoculaire et les formes d'administration rectale.

Lorsque l'on prépare une composition solide sous forme de comprimés ou de gélules, on ajoute au principe actif, micronisé ou non, un mélange d'excipients pharmaceutiques qui peut être composé de diluants comme par exemple le lactose, la cellulose microcristalline, l'amidon, le phosphate dicalcique, de liants comme par exemple la polyvinylpyrrolidone, l'hydroxypropylméthylcellulose, des délitant comme la polyvinylpyrrolidone réticulée, la carboxyméthylcellulose réticulée, des agents d'écoulement comme la silice, le talc, des lubrifiants comme le stéarate de magnésium, l'acide stéarique, le tribéhénate de glycérol, le stéarylfumarate de sodium.

Des agents mouillants ou tensioactifs tels que le laurylsulfate de sodium, le polysorbate 80, le poloxamer 188 peuvent être ajoutés à la formulation.

Les comprimés peuvent être réalisés par différentes techniques, compression directe, granulation sèche, granulation humide, fusion à chaud (hot-melt).

Les comprimés peuvent être nus ou dragéifiés (par du saccharose par exemple) ou enrobés avec divers

polymères ou autres matières appropriés.

Les comprimés peuvent avoir une libération flash, retardée ou prolongée en réalisant des matrices polymériques ou en utilisant des polymères spécifiques au niveau du pelliculage.

Les gélules peuvent être molles ou dures, pelliculées ou non de manière à avoir une activité flash, prolongée ou retardée (par exemple par une forme entérique).

Elles peuvent contenir non seulement une formulation solide formulée comme précédemment pour les comprimés mais aussi des liquides ou des semi-solides.

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le polysorbate 80 ou le poloxamer 188. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres, des sprays.

Pour l'administration transdermique, on peut utiliser des patches sous forme multilaminée ou à réservoir dans lequel le principe actif peuvent être en solution alcoolique, des sprays.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane, des substituts des fréons ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, $\alpha$, $\beta$,-$\gamma$-cyclodextrine, 2-hydroxypropyl-$\beta$-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Dans chaque unité de dosage le principe actif de formule (Ia) isomère lévogyre est présent dans les quantités adaptées aux doses journalières envisagées. En général chaque unité de dosage est convenablement ajustée selon le dosage et le type d'administration prévu, par exemple comprimés, gélules et similaires, sachets, ampoules, sirops et similaires, gouttes de façon à ce qu'une telle unité de dosage contienne de 0,1 à 1000 mg de principe actif, de préférence de 0,5 à 250 mg devant être administrés une à quatre fois par jour.

Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Les compositions de la présente invention peuvent contenir, à côté des composés de formule (Ia) isomère lévogyre, ou l'un de leurs sels, solvats et/ou hydrates pharmaceutiquement acceptables, d'autres principes actifs qui peuvent être utiles dans le traitement des troubles ou maladies indiquées ci-dessus.

Ainsi, la présente invention a également pour objet des compositions pharmaceutiques contenant plusieurs principes actifs en association dont l'un est un composé selon l'invention.

Ainsi, selon la présente invention, on peut préparer des compositions pharmaceutiques contenant un composé selon l'invention associé à un composé agissant sur les récepteurs du CRF.

Les composés selon l'invention pourront également être utilisés pour la préparation de compositions à usage vétérinaire.

Les PREPARATIONS et EXEMPLES suivants illustrent l'invention sans toute fois la limiter.

Dans les Préparations et dans les Exemples on utilise les abréviations suivantes :

éther : éther diéthylique

éther iso : éther diisopropylique
DMF: N,N-diméthylformamide
THF: tétrahydrofurane
DCM: dichlorométhane
AcOEt: acétate d'éthyle
DIPEA: diisopropyléthylamine
TFA: acide trifluoroacétique
Boc: *tert*-butoxycarbonyle
Cbz : benzyloxycarbonyle
BOP: benzotriazol-1-yloxytris(diméthylamino)phosphonium hexafluorophosphate
DCC : 1,3-dicyclohexylcarbodiimide
HOBT: 1-hydroxybenzotriazole hydrate
PS-Trisamine : Tris-(2-aminoéthyl)amine polystyrène réticulé à 1 % avec du divinylbenzène à 3,62 millimoles de fonction amine par gramme de résine, commercialisé par Argonaut Technologie.
F: point de fusion
TA : température ambiante
Eb : température d'ébullition
CLHP : chromatographie liquide haute performance.

Les spectres de résonance magnétique du proton (RMN $^1H$) sont enregistrés à 200 MHz dans du DMSO-$d_6$, en utilisant le pic du DMSO-$d_6$ comme référence. Les déplacements chimiques δ sont exprimés en parties par million (ppm). Les signaux observés sont exprimés ainsi : s: singulet ; se : singulet élargi ; d : doublet ; d.d :

doublet dédoublé ; t : triplet ; q : quadruplet ; m : massif ; mt : multiplet.

Les spectres de masse indiquent la valeur $MH^+$.

PREPARATIONS

Préparations des composés de formule (TV).

Préparation 1.1

3,5-Dichloro-3-(2-méthoxyphényl)-1,3-dihydro-2*H*-indol-2-one.

(IV) : $R_1$ = Cl ; $R_2$ = H ; $R_3$ = OCH3 ; $R_4$ = H ; Hal = Cl.

A) 5-Chloro-3-hydroxy-3-(2-méthoxyphényl)-1,3-dihydro-2*H*-indol-2-one.

On prépare ce composé selon le mode opératoire décrit dans WO 95/18105. On prépare une solution de bromure de 2-méthoxyphénylmagnésium à partir de 16 g de magnésium dans 35 ml d'éther et d'une solution de 124 g de 1-bromo-2-méthoxybenzène dans 175 ml d'éther. On ajoute, en goutte à goutte, sous atmosphère d'argon, cette solution à un mélange de 30 g de 5-chloro-1*H*-indole-2,3-dione dans 250 ml de THF, préalablement refroidi au bain de glace, puis laisse sous agitation en laissant remonter la température à TA. Après 1 heure d'agitation à TA, on verse lentement le mélange réactionnel sur une solution saturée de $NH_4Cl$ et évapore le THF sous vide. On essore le précipité formé et le lave à l'éther iso. On obtient 42 g du produit attendu que l'on utilise tel quel à l'étape suivante.

B) 3,5-Dichloro-3-(2-méthoxyphényl)-1,3-dihydro-2*H*-indol-2-one.

On prépare ce composé selon le mode opératoire décrit dans WO 95/18105. On refroidit à 0°C un mélange de 12,71 g du composé obtenu à l'étape précédente dans 105 ml de DCM, ajoute 5,3 ml de pyridine puis, 4,9 ml de chlorure de thionyle. Après 30 minutes sous agitation, on ajoute de l'eau au mélange réactionnel et évapore le DCM sous vide. On essore le précipité formé, le lave trois fois à l'eau puis trois fois à l'éther iso et le sèche. On obtient 13,66 g du produit attendu que l'on utilise tel quel.

Préparation 1.2

3-Bromo-5-chloro-3-(2-chlorophényl)-1,3-dihydro-2*H*-indol-2-one.

(IV) : $R_1$=Cl ; $R_2$ = H ; $R_3$ =Cl ; $R_4$ = H ; Hal = Br.

On prépare ce composé selon les modes opératoires décrits dans WO 95/18105 aux étapes A), B) et C) de la Préparation 2.

Préparation 1.3

3-Chloro-5-méthyl-3-(2-méthoxyphényl)-1,3-dihydro-2*H*-indol-2-one.

(IV) : $R_1$ = $CH_3$ ; $R_2$ = H ; $R_3$ = $OCH_3$ ; $R_4$ = H ; Hal = Cl.

A) 5-Méthyl-3-hydroxy-3-(2-méthoxyphényl)-1,3-dihydro-2*H*-indol-2-one.

On prépare une solution de bromure de 2-méthoxyphénylmagnésium à partir de 6,8 g de magnésium dans 15 ml de THF et d'une solution de 52,5 g de 1-bromo-2-méthoxybenzène dans 75 ml de THF. On ajoute à TA, goutte à goutte, sous atmosphère d'argon, cette solution à un mélange de 8,9 g de 5-méthyl-1*H*-indole-2,3-dione dans 80 ml de THF puis chauffe à reflux pendant 3 heures. Après refroidissement à TA, on ajoute une solution saturée de $NH_4Cl$ au mélange réactionnel, extrait trois fois à l'AcOEt, lave deux fois les phases organiques jointes à l'eau, par une solution saturée de NaCl, sèche sur $Na_2SO_4$ et concentre en partie le solvant. On essore le précipité formé et obtient 9 g du produit attendu.

B) 3-Chloro-5-méthyl-3-(2-méthoxyphényl)-1,3-dihydro-2*H*-indol-2-one.

On refroidit à 0°C un mélange de 2 g du composé obtenu à l'étape précédente dans 15 ml de DCM, ajoute 0,82 ml de pyridine puis 0,76 ml de chlorure de thionyle. Après 20 minutes sous agitation, on ajoute de l'eau au mélange réactionnel et évapore le DCM sous vide. On extrait la phase aqueuse à l'AcOEt, lave la phase organique à l'eau, par une solution saturée de NaCl, sèche sur $Na_2SO_4$ et évapore sous vide le solvant. On obtient 1,5 g du produit attendu après cristallisation dans le mélange DCM/éther iso.

Préparation 1.4

3-Chloro-3-(2-méthoxyphényl)-5-trifluorométhoxy-1,3-dihydro-2*H*-indol-2-one.

(IV) : $R_1$ = $OCF_3$ ; $R_2$ = H ; $R_3$ = $OCH_3$ ; $R_4$ = H ; Hal = Cl.

A) 3-Hydroxy-3-(2-méthoxyphényl)-5-trifluorométhoxy-1,3-dihydro-2H-indol-2-one.

On prépare une solution de bromure de 2-méthoxyphénylmagnésium à partir de 1,9 g de magnésium dans 4 ml d'éther et d'une solution de 14,54 g de 1-bromo-2-méthoxybenzène dans 21 ml d'éther. On ajoute, goutte à goutte, sous atmosphère d'argon, cette solution à une mélange de 5 g de 5-trifluorométhoxy-1*H*-indole-2,3-dione dans 26 ml de THF, préalablement refroidi au bain de glace, puis chauffe à reflux de l'éther pendant 1 heure 30 minutes et laisse revenir à TA. On verse lentement le mélange réactionnel sur une solution saturée de $NH_4Cl$, extrait à l'AcOEt, lave la phase organique par une solution à 5 % de $K_2CO_3$, à l'eau, par une solution saturée de NaCl, sèche sur $Na_2SO_4$ et évapore sous vide le solvant. On obtient 2,8 g du produit attendu.

B) 3-Chloro-3-(2-méthoxyphényl)-5-trifluorométhoxy-1,3-dihydro-2*H*-indol-2-one.

On refroidit à 0°C un mélange de 2 g du composé obtenu à l'étape précédente dans 20 ml de DCM, ajoute 0,7 g de pyridine, puis 1,05 g de chlorure de thionyle et laisse 15 minutes sous agitation. On concentre le mélange réactionnel jusqu'à un volume de 10 ml et utilise cette solution telle quelle aux Préparations 3.7 et 3.8.

Préparation 1.5

3,5-Dichloro-3-(2-méthoxyphényl)-6-méthyl-1,3-dihydro-2*H*-indol-2-one.

(IV): $R_1$ = Cl ; $R_2$ = 6-$CH_3$ ; $R_3$ = $OCH_3$ ; $R_4$ = H ; Hal = Cl.

A) 2-(2-méthoxyphényl)-2-oxoacétate d'éthyle.

On refroidit à -70°C, sous atmosphère d'argon, une solution de 27 g de 1-bromo-2-méthoxybenzène dans 270 ml d'éther, ajoute, goutte à goutte, 90 ml d'une solution 1,6 M de n-butyllithium dans le pentane, puis laisse 45 minutes sous agitation. On ajoute rapidement 78 ml d'oxalate de diéthyle et laisse sous agitation en laissant remonter la température à TA. Après 1 heure d'agitation à TA, on ajoute au mélange réactionnel une solution saturée de $NH_4Cl$, décante, extrait la phase aqueuse à l'éther, lave les phases organiques jointes à l'eau, par une solution saturée de NaCl, sèche sur $Na_2SO_4$ et évapore les solvants sous vide. On élimine l'oxalate de diéthyle en excès par distillation sous vide (Eb = 87°C sous 2000 Pa). On chromatographie le produit résultant sur gel de silice en éluant par le mélange DCM/hexane (50/50; v/v) puis au DCM. Le produit obtenu est purifié par distillation sous vide. On obtient 13 g du produit attendu, Eb = 110°C sous 3 Pa.

B) 5-Chloro-3-hydroxy-3-(2-méthoxyphényl)-6-méthyl-1,3-dihydro-2*H*-indol-2-one.

a) 4-Chloro-3-méthylphénylcarbamate de *tert*-butyle.
On laisse 24 heures sous agitation à TA un mélange de 10 g de 4-chloro-3-méthylaniline et 15,26 g de di-*tert*-butyldicarbonate dans 50 ml de dioxane. On concentre sous vide le mélange réactionnel et chromatographie le résidu sur gel de silice en éluant par le gradient du mélange DCM/hexane de (50/50 ; v/v) à (70/30 ; v/v). On obtient 5,6 g du produit attendu que l'on utilise tel quel.

b) On refroidit à -70°C, sous atmosphère d'argon, une solution de 5 g de 4-chloro-3-méthylphénylcarbamate de *tert*-butyle dans 45 ml d'éther, ajoute, goutte à goutte, 30 ml d'une solution 1,5M de *tert*-butyllithium dans le pentane, laisse 1 heure sous agitation en faisant remonter la température à -10°C et laisse 1 heure 45 minutes sous agitation à -10°C. On refroidit le mélange réactionnel à -70°C, ajoute, goutte à goutte, une solution de 5 g du composé obtenu à l'étape A dans 25 ml de THF, laisse 1 heure sous agitation en laissant remonter la température à -30°C puis une nuit en laissant remonter la température à TA. On ajoute une solution saturée de $NH_4Cl$ au mélange réactionnel, évapore le THF, extrait trois fois la phase aqueuse résultante à l'AcOEt, lave la phase organique à l'eau, par une solution saturée de NaCl, sèche sur $Na_2SO_4$, évapore partiellement le solvant et essore le produit cristallisé. On obtient 2,6 g du produit attendu, F = 254-256°C.

C) 3,5-Dichloro-3-(2-méthoxyphényl)-6-méthyl-1,3-dihydro-2*H*-indol-2-one.

On refroidit à 0°C un mélange de 1,25 g du composé obtenu à l'étape B dans 20 ml de DCM, ajoute 0,51 ml de pyridine puis 0,47 ml de chlorure de thionyle et laisse 1 heure sous agitation après avoir laissé la température remonter à TA. On ajoute de l'eau et du DCM au mélange réactionnel, après décantation lave quatre fois la phase organique à l'eau, sèche sur $Na_2SO_4$, concentre sous vide jusqu'à un volume de 20 ml et utilise cette solution telle quelle aux Préparations 3.9 et 3.10 ou 3.29.

Préparation 1.6

3-Chloro-3-(2-chlorophényl)-5,6-diméthyl-1,3-dihydro-2*H*-indol-2-one.

(IV) : $R_1$ = $CH_3$ ; $R_2$ = 6-$CH_3$ ; $R_3$ = Cl ; $R_4$ = H ; Hal = Cl.

. A) N-(3,4-diméthylphényl)-DL-2-chloromandélamide.

On chauffe à 227°C pendant 7 heures un mélange de 50 g de 3,4-diméthylaniline et 76,5 g d'acide DL-2-chloromandélique dans 250 ml de 1,2-dichlorobenzène en éliminant l'eau formée à l'aide d'un appareil de Dean-Stark. On concentre sous vide de moitié le volume réactionnel et laisse en cristallisation à TA. On essore le produit cristallisé formé et le lave à l'éther iso. On obtient 89,42 g du produit attendu dont on recristallise un échantillon dans le mélange DCM/éther iso, F = 172-173°C.

B) 3-(2-Chlorophényl)-5,6-diméthyl-1,3-dihydroindol-2-one.

On refroidit à -10°C 100 ml d'acide sulfurique à 95 %, ajoute, goutte à goutte en 30 minutes, 12 ml d'acide sulfurique fumant (oléum à 65%) et laisse sous agitation en laissant remonter la température à +10°C. On refroidit de nouveau à 0°C, ajoute, par portions et en 10 minutes, 23,8 g du composé obtenu à l'étape précédente et laisse sous agitation en laissant remonter la température qui se stabilise à 29°C. Après 2 heures sous agitation à TA, on verse le mélange réactionnel sur de la glace et essore le précipité formé. On dissout le précipité dans 1000 ml de DCM et 200 ml de THF, amène le pH à 2 par ajout de $K_2CO_3$ solide, filtre et concentre sous vide le filtrat. On chromatographie le résidu sur gel de silice en éluant par le gradient du mélange DCM/AcOEt/THF de (90/10/5 ; v/v/v) à (80/20/5 ; v/v/v). On obtient 7,72 g du produit attendu, F = 231°C.

C) 3-(2-Chlorophényl)-3-hydroxy-5,6-diméthyl-1,3-dihydroindol-2-one.

A une solution de 4 g du composé obtenu à l'étape précédente dans 70 ml de THF, on ajoute à TA, sous atmosphère d'argon, 0,65 g d'hydrure de sodium à 60 % dans l'huile. Puis après cessation du dégagement gazeux, on ajoute 1,7 ml de diméthyldisulfure et fait barboter dans le mélange réactionnel, pendant 4 heures, un courant d'air à TA. On verse le mélange réactionnel dans l'eau, concentre sous vide le THF, extrait la phase aqueuse à l'AcOEt, lave la phase organique à l'eau, par une solution saturée de NaCl, sèche sur $Na_2SO_4$, concentre partiellement sous vide le solvant et essore le produit cristallisé formé. On obtient 3,3 g du produit attendu, F = 251-253°C.

D) 3-Chloro-3-(2-chlorophényl)-5,6-diméthyl-1,3-dihydro-2*H*-indol-2-one.

On refroidit à 0°C une suspension de 1 g du composé obtenu à l'étape précédente dans 7 ml de DCM, ajoute 0,4 ml de pyridine puis 0,37 ml de chlorure de thionyle et laisse 30 minutes sous agitation. On dilue le mélange réactionnel par ajout de 30 ml de DCM, lave la phase organique par 20 ml d'eau, sèche sur $Na_2SO_4$ et concentre partiellement sous vide le solvant à une température inférieure à 40°C. On utilise cette solution telle quelle aux Préparations 3.11 et 3.12.

Préparation 1.7

3,5-Dichloro-3-(2,3-diméthoxyphényl)-1,3-dihydro-2*H*-indol-2-one.

(IV) : $R_1$ = Cl ; $R_2$ = H ; $R_3$ = $OCH_3$ ; $R_4$ = 3-$OCH_3$ ; Hal = Cl.

A) 2-(2,3-Diméthoxyphényl)-2-oxoacétate d'éthyle.

On refroidit à -40°C un mélange de 27,6 g de 1,2-diméthoxybenzène dans 160 ml d'éther, ajoute, goutte à goutte, 250 ml d'une solution 1,6 M de n-butyllithium dans l'hexane, puis laisse 24 heures sous agitation en laissant remonter la température à TA. On refroidit le mélange réactionnel à -20°C, ajoute rapidement 136 ml d'oxalate de diéthyle et laisse sous agitation en laissant remonter la température à TA. Après 30 minutes d'agitation à TA, on verse le mélange réactionnel sur une solution saturée de $NH_4Cl$, décante, extrait la phase aqueuse à l'éther, lave les phases organiques jointes deux fois à l'eau, sèche sur $Na_2SO_4$ et évapore sous vide les solvants. On élimine l'oxalate de diéthyle en excès par distillation sous vide (Eb=90°C sous 2400Pa). On chromatographie le produit brut résultant sur gel de silice en éluant par le mélange heptane/éther iso (90/10 ; v/v). On obtient 25 g du produit attendu que l'on utilise tel quel à l'étape suivante.

B) 5-chloro-3-hydroxy-3-(2,3-diméthoxyphényl)-1,3-dihydro-2*H*-indol-2-one.

a) 4-Chlorophénylcarbamate de *tert*-butyle.
On laisse 24 heures sous agitation à TA un mélange de 12,7 g de 4-chloroaniline et 22 g de di-*tert*-butyldicarbonate dans 60 ml de dioxane. On concentre sous vide le mélange réactionnel, reprend le résidu au pentane, essore le précipité formé et le sèche. On obtient 22,5 g du produit attendu.
b) On refroidit à -40°C, sous atmosphère d'azote sec, un mélange de 11,4 g de 4-chlorophénylcarbamate de *tert*-butyle dans 100ml d'éther, ajoute, goutte à goutte, 80 ml d'une solution 1,5 M de *tert*-butyllithium dans le pentane et laisse 3 heures sous agitation à -20°C. On refroidit le mélange réactionnel à 40°C, ajoute, en une heure, une solution de 14 g du composé obtenu à l'étape A dans 50 ml de THF et laisse 4 jours sous agitation à TA. On verse

le mélange réactionnel sur une solution saturée de $NH_4Cl$, essore le précipité formé et le sèche. On obtient 10,2 g du produit attendu que l'on utilise tel quel à l'étape suivante.

C) 3,5-Dichloro-3-(2,3-diméthoxyphényl)-1,3-dihydro-2*H*-indol-2-one.

A un mélange de 2 g du composé obtenu à l'étape B dans 50 ml de DCM on ajoute, à TA, 0,8 ml de pyridine puis 1,2 ml de chlorure de thionyle et laisse sous agitation jusqu'à dissolution. On lave le mélange réactionnel par une solution d'HCl 1N, puis deux fois à l'eau, sèche sur $Na_2SO_4$ et évapore sous vide le solvant. On chromatographie le résidu sur gel de silice en éluant par le mélange DCM/AcOEt (95/5 ; v/v). On obtient 1,2 g du produit attendu que l'on utilise tel quel.

Préparation 1.8

3,5-Dichloro-3-(2-méthoxyphényl)-6-trifluorométhyl-1,3-dihydro-2*H*-indol-2-one.

(IV) : $R_1$ = Cl ; $R_2$ = 6-$CF_3$ ; $R_3$ = $OCH_3$ ; $R_4$ = H ; Hal = Cl.

A) 5-Chloro-3-hydroxy-3-(2-méthoxyphényl)-6-trifluorométhyl-1,3-dihydro-2*H*-indol-2-one.

a) 4-Chloro-3-trifluorométhylphénylcarbamate de *tert*-butyle.
On prépare ce composé selon le mode opératoire décrit à l'étape B a) de la Préparation 1.5 à partir de 4-chloro-3-trifluorométhylaniline et de di-*tert*-butyldicarbonate dans le dioxane. On obtient le produit attendu sous forme d'huile qui se solidifie, F = 90°C.
b) On refroidit à -70°C, sous atmosphère d'argon, une solution de 4 g de 4-chloro-3-trifluorométhylphénylcarbamate de *tert*-butyle dans 30 ml d'éther, ajoute, goutte à goutte, 22 ml d'une solution 1,5M de *tert*-butyllithium dans le pentane, laisse 1 heure sous agitation en faisant remonter la température à -10°C et laisse 2 heures 30 minutes sous agitation à -10°C. On refroidit le mélange réactionnel à -70°C, ajoute, goutte à goutte, une solution de 3,05 g du composé obtenu à l'étape A de la Préparation 1.5 dans 15 ml de THF, laisse 1 heure sous agitation en laissant remonter la température à - 30°C puis 16 heures en laissant remonter la température à TA. On ajoute une solution saturée de $NH_4Cl$ au mélange réactionnel, évapore l'éther et le THF, extrait la phase aqueuse résultante à l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le mélange DCM/AcOEt (90/10 ; v/v). On obtient 1,48 g du produit attendu après cristallisation dans le mélange éther iso/hexane, F = 230-231°C.

B) 3,5-Dichloro-3-(2-méthoxyphényl)-6-trifluorométhyl-1,3-dihydro-2*H*-indol-2-one.

On refroidit à 0°C une suspension de 1,3 g du composé obtenu à l'étape A dans 8 ml de DCM, ajoute 0,43 ml de pyridine puis 0,4 ml de chlorure de thionyle et laisse 15 minutes sous agitation. On lave trois fois le mélange réactionnel à l'eau, sèche la phase organique sur $Na_2SO_4$ et évapore partiellement sous vide le solvant jusqu'à un volume de 10 ml. On utilise cette solution telle quelle aux Préparations 3.15 et 3.16.

Préparation 1.9

3,5-Dichloro-3-(2-chlorophényl)-6-méthoxy-1,3-dihydro-2*H*-indol-2-one.

(IV) : $R_1$ = Cl ; $R_2$ = 6-$OCH_3$ ; $R_3$ = Cl ; $R_4$ = H ; Hal = Cl.

A) 4-Chloro-3-méthoxyaniline.

On hydrogène dans un appareil de Parr pendant 4 heures, à 35°C et sous une pression de 1,3 bars, un mélange de 36 g de 2-chloro-5-nitroanisole et du nickel de Raney® dans 150 ml de MeOH et 200 ml de THF. On filtre le catalyseur sur Célite® et concentre sous vide le filtrat. On obtient 28 g du produit attendu que l'on utilise tel quel.

B) N-(4-chloro-3-méthoxyphényl)-DL-2-chloromandélamide.

On chauffe à 230°C pendant 4 heures un mélange de 28 g du composé obtenu à l'étape précédente et 33,13

g d'acide DL-2-chloromandélique dans 128 ml de 1,2-dichlorobenzène en éliminant l'eau formée à l'aide d'un appareil de Dean-Stark. On concentre sous vide en partie le mélange réactionnel et laisse en cristallisation. On essore le produit cristallisé formé et le lave à l'éther iso. On obtient 40 g du produit attendu.

C) 5-Chloro-3-(2-chlorophényl)-6-méthoxy-1,3-dihydro-2*H*-indol-2-one.

On ajoute rapidement 40 g du composé obtenu à l'étape précédente à 550 g d'acide polyphosphorique puis on chauffe à 60°C pendant 8 heures et laisse une nuit sous agitation en laissant revenir la température à TA. On ajoute de l'eau glacée au mélange réactionnel, essore le précipité formé et le lave à l'eau. On reprend le précipité dans l'AcOEt, essore le produit blanc obtenu après trituration et le lave à l'éther iso. On obtient 17,2 g du produit attendu, F = 243-247°C.

D) 5-Chloro-3-(2-chlorophényl)-3-hydroxy-6-méthoxy-1,3-dihydro-2*H*-indol-2-one.

A une solution de 17,2 g du composé obtenu à l'étape précédente dans 220 ml de THF, on ajoute à TA, sous atmosphère d'argon, 2,56 g d'hydrure de sodium à 60 % dans l'huile. Après cessation du dégagement gazeux, on ajoute 6,85 g de diméthyldisulfure, fait barboter de l'air dans le mélange réactionnel et laisse 72 heures sous agitation à TA. On ajoute de l'eau au mélange réactionnel, évapore sous vide le THF, extrait la phase aqueuse restante à l'AcOEt, lave la phase organique à l'eau, par une solution saturée de NaCl, sèche sur $Na_2SO_4$ et évapore sous vide le solvant. On dissout le produit obtenu dans du DCM, concentre en partie le solvant, laisse en cristallisation et essore le produit cristallisé formé. On obtient 6 g du produit attendu, F = 237-240°C.

E) 3,5-Dichloro-3-(2-chlorophényl)-6-méthoxy-1,3-dihydro-2*H*-indol-2-one.

On refroidit au bain de glace une suspension de 1,5 g du composé obtenu à l'étape précédente dans 20 ml de DCM, ajoute 0,375 ml de pyridine puis 0,33 ml de chlorure de thionyle et laisse 30 minutes sous agitation. A la fin de la réaction, on obtient une suspension du produit attendu qui a précipité dans le DCM et utilise directement cette suspension aux Préparations 3.17 et 3.18.

Préparation 1.10

3,6-Dichloro-3-(2-méthoxyphényl)-5-méthyl-1,3-dihydro-2*H*-indol-2-one.

(IV) : $R_1 = CH_3$ ; $R_2$ = 6-Cl ; $R_3 = OCH_3$ ; $R_4$ = H ; Hal = Cl.

A) 6-Chloro-5-méthyl-3-méthylthio-1,3-dihydro-2*H*-indol-2-one et 4-chloro-5-méthyl-3-méthylthio-1,3-dihydro-2H-indol-2-one.

Dans 320 ml de DCM refroidi à -70°C, on introduit 8,5 ml de chlore, puis ajoute, en 20 minutes et à -70°C, une solution de 24 ml de méthylthioacétate d'éthyle dans 60 ml de DCM et laisse 15 minutes sous agitation à -70°C. On ajoute ensuite, à -70°C et en 30 minutes, une solution de 52,64 g de 3-chloro-4-méthylaniline dans 100 ml de DCM et laisse 1 heure 45 minutes sous agitation à -70°C. On ajoute enfin, à -70°C, 41,3 ml de triéthylamine et laisse 1 heure sous agitation en laissant remonter la température à TA. On lave le mélange réactionnel deux fois par 250 ml d'eau, sèche la phase organique sur $MgSO_4$ et évapore sous vide le solvant. On reprend le résidu dans un mélange de 600 ml d'éther et 130 ml d'HCl 2N et laisse 72 heures sous agitation à TA. On filtre une insoluble, décante le filtrat, lave deux fois la phase organique à l'eau, sèche sur $MgSO_4$ et évapore sous vide le solvant. On chromatographie le résidu sur gel de silice en éluant au DCM puis par le mélange DCM/AcOEt (85/15 ; v/v). On rechromatographie le mélange obtenu sur gel de silice en éluant au DCM puis par le mélange DCM/AcOEt (95/5 ; v/v). On sépare les deux isomères :

- l'isomère le moins polaire qui est le G-chloro-5-méthyl-3-méthylthio-1,3-dihydro-2*H*-indol-2-one et obtient 1,16 g.
- l'isomère le plus polaire qui est le 4-chloro-5-méthyl-3-méthylthio-1,3-dihydro-2*H*-indol-2-one et obtient 0,72 g.

B) b-Chloro-5-méthyl-1*H*-indole-2,3=dione.

On chauffe à reflux pendant 1 heure un mélange de 1,16 g de 6-chloro-5-méthyl-3-méthylthio-1,3-dihydro-2*H*-indol-2-one obtenu à l'étape précédente et 0,681 g de N-chlorosuccinimide dans 100 ml de tétrachlorure de carbone. On concentre sous vide le mélange réactionnel, reprend le résidu dans un mélange de 80 ml de THF et 20 ml d'eau puis chauffe à reflux pendant 16 heures. On évapore sous vide le THF, extrait la phase aqueuse restante à

l'AcOEt, lave la phase organique à l'eau, par une solution saturée de NaCl, sèche sur $Na_2SO_4$ et évapore sous vide le solvant. On chromatographie le résidu sur gel de silice en éluant au DCM puis par le gradient du mélange DCM/AcOEt jusqu'à (85/15 ; v/v). On obtient 0,793 g du produit attendu, F = 264°C.

C) 6-Chloro-3-hydroxy-3-(2-méthoxyphényl)-5-méthyl-1,3-dihydro-2*H*-indol-2-one.

On prépare une solution de bromure de 2-méthoxyphénylmagnésium à partir de 0,687 g de magnésium dans 1,5 ml d'éther et d'une solution de 5,35 g de 1-bromo-2-méthoxybenzène dans 7,55 ml d'éther. On ajoute, en goutte à goutte, sous atmosphère d'argon, cette solution à un mélange de 1,4 g du composé obtenu à l'étape précédente dans 14 ml de THF préalablement refroidi au bain de glace, puis laisse sous agitation en laissant remonter la température à TA. Après 1 heure d'agitation à TA, on verse lentement le mélange réactionnel sur une solution saturée de $NH_4Cl$, évapore le THF sous vide, extrait à l'AcOEt, lave la phase organique à l'eau, par une solution saturée de NaCl, sèche sur $Na_2SO_4$ et évapore sous vide l'AcOEt. On chromatographie le résidu sur gel de silice en éluant au DCM puis par le mélange DCM/MeOH (98/2 ; v/v). On obtient 1,6 g du produit attendu après cristallisation dans le mélange THF/MeOH, F = 266°C.

D) 3,6-Dichloro-3-(2-méthoxyphényl)-5-méthyl-1,3-dihydro-2*H*-indol-2-one.

On refroidit au bain de glace une suspension de 2,5 g du composé obtenu à l'étape précédente dans 15 ml de DCM, ajoute 1 ml de pyridine puis 1,09 ml de chlorure de thionyle et laisse 2 heures sous agitation. On concentre partiellement sous vide le mélange réactionnel jusqu'à un volume de 10 ml et utilise cette solution telle quelle aux Préparations 3.19 et 3.20.

Préparation 1.11 1

3-Bromo-5,6-dichloro-3-(2-chlorophényl)-1,3-dihydro-2*H*-indol-2-one.

(IV):$R_1$=Cl;$R_2$=6-Cl;$R_3$ =Cl ; $R_4$=H ; Hal = Br.

On prépare ce composé selon les modes opératoires décrits dans WO 95/18105 aux étapes A), B) et C) de la Préparation 72.

Préparation 1.12

3,5-Dichloro-3-(2-éthoxyphényl)-1,3-dihydro-2*H*-indol-2-one.

(IV) : $R_1$ = Cl ; $R_2$ = H ; $R_3$ = $OCH_2CH_3$ ; $R_4$ = H ; Hal = Cl.

A) 1-Bromo-2-éthoxybenzène.

On chauffe 2 heures à reflux un mélange de 17,5 g de 2-bromophénol, 66 ml de sulfate de diéthyle et 170 ml d'une solution de NaOH à 10%. Après refroidissement du mélange réactionnel à TA, on extrait à l'AcOEt, lave la phase organique par une solution de NaOH 2N, sèche sur $Na_2SO_4$ et évapore sous vide le solvant. On obtient 19,6 g du produit attendu.

B) 5-Chloro-3-(2-éthoxyphényl)-3-hydroxy-1,3-dihydro-2*H*-indol-2-one.

On prépare une solution de bromure de 2-éthoxyphénylmagnésium à partir de 2,2 g de magnésium dans 10 ml d'éther et d'une solution de 16,5 g du composé obtenu à l'étape précédente dans 40 ml d'éther. On ajoute, en goutte à goutte et sous atmosphère d'azote, cette solution à un mélange de 5 g de 5-chloro-1*H*-indole-2,3-dione dans 20 ml de THF, en maintenant la température du milieu réactionnel inférieure à 35°C. Après 2 heures d'agitation à TA, on verse le mélange réactionnel sur 200 ml d'HCl 2N, extrait à l'AcOEt, sèche la phase organique sur $Na_2SO_4$ et évapore sous vide les solvants. On reprend le résidu dans l'éther iso à chaud et laisse en cristallisation. On essore le produit cristallisé formé, le lave à l'éther iso et le sèche. On obtient 5,7 g du produit attendu, F = 251 °C.

C) 3,5-Dichloro-3-(2-éthoxyphényl)1,3-dihydro-2*H*-indol-2-one.

A un mélange de 3 g du composé obtenu à l'étape précédente et 2 ml de pyridine dans 50 ml de DCM, on

ajoute, à TA, 1 ml de chlorure de thionyle et laisse 1 heure sous agitation à TA. On chromatographie le mélange réactionnel sur gel de silice en éluant au DCM. On obtient 2,4 g du produit attendu après cristallisation dans l'éther iso, F = 198°C.

Préparation 1.13

3,5-Dichloro-3-(2,3-difluorophényl)-1,3-dihydro-2*H*-indol-2-one.

(IV) : $R_1$ = Cl; $R_2$ = H; $R_3$ = F; $R_4$ = 3-F; Hal = Cl.

A) 5-Chloro-3-(2,3-difluorophényl)-3-hydroxy-1,3-dihydro-2*H*-indol-2-one.

On refroidit à -10°C une solution de 5,6 g de 1,2-difluorobenzène dans 50 ml d'éther, ajoute, goutte à goutte, 31 ml d'une solution 1,6M de n-butyllithium dans l'hexane et laisse 2 heures sous agitation à -10°C. On refroidit à -50°C le mélange réactionnel, ajoute une solution de 4 g de S-chloro-1*H*-indole-2,3-dione dans 40 ml de THF et laisse 12 heures sous agitation en laissant remonter la température à TA. On verse le mélange réactionnel sur un mélange HCl concentré/glace/eau, extrait à l'AcOEt, lave la phase organique par une solution de NaOH 1N, à l'eau, sèche sur $Na_2SO_4$ et évapore le solvant sous vide. On obtient 2,8 g du produit attendu après cristallisation dans l'éther iso, F = 248°C.

B) 3,5-Dichloro-3-(2,3-difluorophényl)-1,3-dihydro-2*H*-indol-2-one.

A un mélange de 2,8 g du composé obtenu à l'étape précédente et 1 ml de pyridine dans 30 ml de DCM, on ajoute 0,9 ml de chlorure de thionyle et laisse 1 heure sous agitation à TA. On lave deux fois le mélange réactionnel à l'eau, sèche sur $Na_2SO_4$ et évapore le solvant sous vide. On chromatographie le résidu sur gel de silice en éluant au DCM. On obtient 0,9 g du produit attendu.

Préparation 1.14

3,5-Dichloro-3-(2,4-diméthoxyphényl)-1,3-dihydro-2*H*-indol-2-one.

(IV) : $R_1$ = Cl ; $R_2$ = H ; $R_3$ = $OCH_3$ ; $R_4$ = 4-$OCH_3$ ; Hal = Cl.

A) 5-Chloro-3-hydroxy-3-(2,4-diméthoxyphényl)-1,3-dihydro-2*H*-indol-2-one

On prépare une solution de bromure de 2,4-diméthoxyphénylmagnésium à partir de 2,2 g de magnésium dans 10 ml de THF et d'une solution de 18 g de 1-bromo-2,4-diméthoxybenzène dans 40 ml de THF. On ajoute, en goutte à goutte, cette solution à un mélange de 5 g de 5-chloro-1*H*-indole-2,3-dione dans 50 ml de THF à une température de 30°C, puis chauffe 2 heures à reflux. On refroidit le mélange réactionnel à TA, le verse sur une solution saturée de $NH_4Cl$, extrait à l'AcOEt, lave la phase organique à l'eau, sèche sur $Na_2SO_4$ et évapore sous vide le solvant. On obtient 7,2 g du produit attendu après cristallisation dans l'éther iso à chaud.

B) 3,5-Dichloro-3-(2,4-diméthoxyphényl)-1,3-dihydro-2*H*-indol-2-one.

On refroidit à une température inférieure à 10°C un mélange de 2,5 g du composé obtenu à l'étape précédente et 0,6 ml de pyridine dans 20 ml de DCM, ajoute, en goutte à goutte, 0,6 ml de chlorure de thionyle et laisse 15 minutes sous agitation. On lave deux fois le mélange réactionnel à l'eau, sèche sur $Na_2SO_4$ et évapore le solvant sous vide. On obtient le produit attendu que l'on utilise tel quel aux Préparations 3.33 et 3.34.

Préparation 1.15

3,5-Dichloro-3-(1,3-benzodioxol-4-yl)-1,3-dihydro-2*H*-indol-2-one.

(IV) : $R_1$ = Cl ; $R_2$ = H ; $R_3$ + $R_4$= 2,3-O-$CH_2$-O-; Hal = Cl.

A) 4-Bromo-1,3-benzodioxole

On prépare ce composé selon le procédé décrit dans Tetrahedron Lett., 1995, 36, 6413-6414.

B) 5-Chloro-3-(1,3-benzodioxol-4-yl)-3-hydroxy-1,3-dihydro-2*H*-indol-2-one.

On prépare une solution de bromure de 1,3-benzodioxol-4-ylmagnésium à partir de 0,85 g de magnésium dans 10 ml de THF et d'une solution de 6,7 g du composé obtenu à l'étape précédente dans 40 ml de THF. On ajoute, en goutte à goutte et à une température inférieure à 40°C, cette solution à un mélange de 3 g de 5-chloro-1*H*-indole-2,3-dione dans 50 ml de THF puis laisse une heure sous agitation. On verse le mélange réactionnel sur une solution saturée de $NH_4Cl$, extrait à l'AcOEt, lave la phase organique à l'eau, sèche sur $Na_2SO_4$ et évapore sous vide le solvant, on obtient 1,12 g du produit attendu après cristallisation dans le DCM, F = 271°C.

C) 3,5-Dichloro-3-(1,3-benzodioxol-4-yl)-1,3-dihydro-2*H*-indol-2-one.

A un mélange de 1,1 g du composé obtenu à l'étape précédente et 0,4 ml de pyridine dans 20 ml de DCM, on ajoute, à une température inférieure à 25°C, 0,3 ml de chlorure de thionyle et laisse 30 minutes sous agitation. On lave deux fois le mélange réactionnel à l'eau, sèche la phase organique sur $Na_2SO_4$ et évapore sous vide le solvant. On obtient 0,62 g du produit attendu après cristallisation dans le DCM, F = 241°C.

Préparation 1.16

3,5,6-Trichloro-3-(2-méthoxyphényl)-1,3-dihydro-2*H*-indol-2-one.

(IV) : $R_1$ = Cl ; $R_2$ = 6-Cl ; $R_3$ = $OCH_3$ ; $R_4$ = H ; Hal = Cl.

A) 5,6-Dichloro-1*H*-indole-2,3-dione.

On prépare ce composé selon le mode opératoire décrit dans J. Am. Chem. Soc., 1946, 68, 2697-2703 ou selon le mode opératoire décrit dans J. Org. Chem., 1952, 17, 149-156.

B) 5,6-Dichloro-3-hydroxy-3-(2-méthoxyphényl)-1,3-dihydro-2*H*-indol-2-one.

A une suspension de 0,72 g de magnésium dans 15 ml d'éther contenant quelques cristaux d'iode, on ajoute, en goutte à goutte, 5,57 g de 1-bromo-2-méthoxybenzène, en maintenant le reflux lorsque celui-ci a démarré. A la fin de l'addition on chauffe 2 heures à reflux. On ajoute ensuite une suspension de 2,7 g de 5,6-dichloro-1*H*-indole-2,3-dione dans 30 ml de THF et chauffe à reflux pendant 30 minutes. Après refroidissement à TA, on verse le mélange réactionnel sur un mélange eau/glace/HCl concentré, extrait à l'AcOEt, sèche la phase organique sur $Na_2SO_4$ et évapore sous vide le solvant. On triture le résidu dans l'éther iso à chaud, essore le précipité formé et le lave à l'éther. On obtient 3 g du produit attendu.

C) 3,5,6-Trichloro-3-(2-méthoxyphényl)-1,3-dihydro-2*H*-indol-2-one.

On refroidit au bain de glace une suspension de 1,5 g du composé obtenu à l'étape précédente dans 30 ml de DCM, ajoute 0,56 ml de pyridine puis 0,5 ml de chlorure de thionyle. Après 1 heure sous agitation à TA, on dilue le mélange réactionnel par ajout de DCM, lave la phase organique à l'eau jusqu'à pH neutre, sèche sur $Na_2SO_4$ et évapore sous vide le solvant. On obtient 1,5 g du produit attendu sous forme de mousses que l'on utilise tel quel.

Préparations des composés de formule (V).

Préparation 2.1 a)

Chlorhydrate de (2S,4R)-4-hydroxy-N,N-diméthyl-2-pyrrolidinecarboxamide.

(V), HCl : $R_5$ = $N(CH_3)_2$ ; $R_6$ = H.

A) (2S, 4R)-1-(*tert*-Butoxycarbonyl)-4-hydroxy-N,N-diméthyl-2-pyrrolidine carboxamide.

On refroidit à 0°C un mélange de 11,2 g d'acide (2S, 4R)-1-(*tert*-butoxycarbonyl)-4-hydroxy-2-pyrrolidine carboxylique dans 50 ml de DCM, ajoute 8,45 ml de DIPEA puis 21,2 g de BOP et laisse 10 minutes sous agitation. Puis on ajoute de la diméthylamine gaz par barbotage et laisse 3 heures sous agitation à TA. On concentre partiellement sous vide le mélange réactionnel jusqu'à un volume de 20 ml et filtre un insoluble. On chromatographie le filtrat sur

gel de silice en éluant par le mélange DCM/MeOH (94/6 ; v/v) et rechromatographie le produit obtenu sur alumine en éluant par le mélange DCM/MeOH (96/4 ; v/v). On obtient 11,1 g du produit attendu.

B) Chlorhydrate de (2S,4R)-4-hydroxy-N,N-diméthyl-2-pyrrolidinecarboxamide.

On laisse 2 heures sous agitation à TA un mélange de 6,9 g du composé obtenu à l'étape précédente dans 69 ml d'une solution 4N d'HCl dans l'éther. On concentre sous vide le mélange réactionnel, reprend le résidu dans l'éther, évapore sous vide le solvant et répète plusieurs fois cette opération. On obtient 4 g du produit attendu.

Préparation 2.1 b)

Trifluoroacétate de (2S, 4R)-4-hydroxy-N,N-diméthyl-2-pyrrolidinecarboxamide.

(V), $CF_3COOH$ : $R_5 = N(CH_3)_2$ ; $R_6 = H$.

On refroidit à 0°C une solution de 2,1 g du composé obtenu à l'étape A de la Préparation 2.1 a) dans 5 ml de DCM, ajoute 10 ml d'acide trifluoroacétique et laisse 2 heures sous agitation à TA. On concentre sous vide le mélange réactionnel, reprend le résidu au DCM, évapore sous vide le solvant et répète plusieurs fois cette opération. On obtient le produit attendu qui est directement utilisé aux Préparations 3.1 et 3.2.

Préparation 2.2

Chlorhydrate de (2S, 4R)-4-méthoxy-N,N-diméthyl-2-pyrrolidinecarboxamide.

(V), HCl : $R_5 = N(CH_3)_2$ ; $R_6 = CH_3$.

A) (2S, 4R)-1-(*tert*-Butoxycarbonyl)-4-méthoxy-N,N-diméthyl-2-pyrrolidine carboxamide.

On refroidit à 0°C une solution de 6,5 g du composé obtenu à l'étape A de la Préparation 2.1 a) dans 70 ml de THF, ajoute par petites fractions 1,2 g d'hydrure de sodium à 60 % dans l'huile et laisse 30 minutes sous agitation à 0°C. On ajoute ensuite goutte à goutte, une solution de 2,35 ml d'iodure de méthyle dans 10 ml de THF et laisse 2 heures sous agitation en laissant remonter la température à TA. On ajoute 5 gouttes d'eau, neutralise le mélange réactionnel par ajout d'HCl concentré et concentre sous vide. On azéotrope l'eau résiduelle par ajout de benzène et concentration sous vide. On chromatographie le résidu sur gel de silice en éluant par le mélange DCM/MeOH (96/4 ; v/v). On obtient 6,1 g du produit attendu.

B) Chlorhydrate de (2S, 4R)-4-méthoxy-N,N-diméthyl-2-pyrrolidinecarboxamide.

On laisse 2 heures sous agitation un mélange de 6,1 g du composé obtenu à l'étape précédente et 65 ml d'une solution 4N d'HCl dans l'éther. On concentre sous vide le mélange réactionnel, reprend le résidu au DCM, évapore sous vide le solvant et répète plusieurs fois l'opération. On obtient 4,45 g du produit attendu.

Préparation 2.3

Trifluoroacétate de (2S, 4R)-4-éthoxy-N,N-diméthyl-2-pyrrolidinecarboxamide.

(V), $CF_3COOH$ : $R_5 = N(CH_3)_2$ ; $R_6 = -CH_2CH_3$.

A) Acide (2S, 4R)-1-(*tert*-butoxycarbonyl)-4-éthoxy-2-pyrrolidinecarboxylique

A une solution de 5 g d'acide (2S, 4R)-1-(*tert*-butoxycarbonyl)-4-hydroxy-2-pyrrolidinecarboxylique dans 100 ml de THF, on ajoute, sous atmosphère d'azote, 1,72 g d'hydrure de sodium à 60 % dans l'huile et laisse 45 minutes sous agitation à TA. On ajoute ensuite 3,27 g d'iodure d'éthyle, chauffe à reflux pendant 3 heures et laisse 18 heures sous agitation en laissant revenir la température à TA. On concentre sous vide le mélange réactionnel, reprend le résidu par une solution à 5 % de $KHSO_4$, extrait à l'AcOEt, sèche la phase organique sur $Na_2SO_4$ et évapore sous vide le solvant. On obtient 4,5 g du produit attendu sous forme d'huile.

B) (2S, 4R)-1-(*tert*-Butoxycarbonyl)-4-éthoxy-N,N-diméthyl-2-pyrrolidine carboxamide.

A une solution de 4,5 g du composé obtenu à l'étape précédente dans 100 ml de DCM, on ajoute 3,5 g de triéthylamine puis 7,6 g de BOP et laisse 15 minutes sous agitation à TA. Puis on ajoute de la diméthylamine gaz par barbotage et laisse 3 heures sous agitation à TA. On concentre sous vide le mélange réactionnel, extrait le résidu à l'AcOEt, lave la phase organique par une solution à 5 % de $Na_2CO_3$, par une solution à 5 % de $KHSO_4$, sèche sur $Na_2SO_4$ et évapore sous vide le solvant. On chromatographie le résidu sur gel de silice en éluant par le mélange DCM/MeOH (95/5 ; v/v). On obtient 2 g du produit attendu sous forme d'huile.

C) Trifluoroacétate de (2S, 4R)-4-éthoxy-N,N-diméthyl-2-pyrrolidine carboxamide.

On refroidit à 0°C une solution de 2 g du composé obtenu à l'étape précédente dans 10 ml de DCM, ajoute 10 ml d'acide trifluoroacétique et laisse 2 heures sous agitation à TA. On concentre sous vide, reprend le résidu au DCM, évapore sous vide le solvant et répète plusieurs fois cette opération. On obtient 2 g du produit attendu.

Préparation 2.4

Ester *tert*-butylique de l'acide 2-[[(3R,5S)-5-[(diméthylamino)carbonyl]-3-pyrrolidinyl]oxy]acétique.

(V) : $R_5 = N(CH_3)_2$ ; $R_6 = -CH_2COO-C(CH_3)_3$.

A) (2S,4R)-1-(Benzyloxycarbonyl)-4-hydroxy-N,N-diméthyl-2-pyrrolidine carboxamide.

On laisse 1 heure sous agitation à TA un mélange de 15 g d'acide (2S,4R)-1-(benzyloxycarbonyl)-4-hydroxy-2-pyrrolidinecarboxylique, 7,64 g d'HOBT et 11,65 g de DCC dans 250 ml de DCM. On refroidit le mélange réactionnel au bain de glace, ajoute de la diméthylamine gaz par barbotage pendant 10 minutes et laisse 3 heures sous agitation à TA. On filtre un insoluble et concentre sous vide le filtrat. On reprend le résidu par une solution saturée de $Na_2CO_3$, extrait au DCM, sèche la phase organique sur $Na_2SO_4$ et évapore le solvant sous vide. On obtient 13 g du produit attendu sous forme d'huile.

B) Ester *tert*-butylique de l'acide 2-[[(3R,5S)-1-[(benzyloxycarbonyl)-5-[(diméthylamino)carbonyl]-3-pyrrolidinyl]oxy] acétique.

On refroidit à 0°C un mélange de 5 g du composé obtenu à l'étape précédente et 3 g de tétrabutylammonium hydrogène sulfate dans 100 ml de benzène, ajoute 50 ml d'une solution aqueuse à 50 % de NaOH, puis, goutte à goutte, 5 g de bromoacétate de *tert*-butyle et laisse 30 minutes sous forte agitation. On dilue le mélange réactionnel par un mélange benzène/DCM, décante, sèche la phase organique sur $Na_2SO_4$ et évapore les solvants sous vide. On chromatographie le résidu sur gel de silice en éluant à l'AcOEt. On obtient 6,3 g du produit attendu sous forme d'huile.

C) Ester *tert*-butylique de l'acide 2-[[(3R,5S)-5-[(diméthylamino)carbonyl]-3-pyrrolidinyl)oxy)acétique.

On hydrogène pendant 3 heures, à TA et sous pression atmosphérique, un mélange de 6,3 g du composé obtenu à l'étape précédente et 0,7 g de palladium sur charbon à 10 % dans 200 ml d'AcOEt. On filtre le catalyseur sur Célite et concentre de moitié le filtrat sous vide. On obtient une solution du produit attendu que l'on utilise aux Préparations 3.38 et 3.39.

Préparation 2.5

3-(4-morpholinyl)propionate de (3R,5S)-5-[(diméthylamino)carbonyl]-3-pyrrolidine.

(V) : $R_5 = -N(CH_3)_2$ ;

$R_6 = -COCH_2CH_2-$ 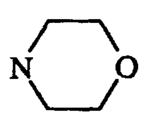

A) (2S,4R)-4-(acryloyloxy)-2-[(diméthylamino)carbonyl]-1-pyrrolidinecarboxylate de benzyle.

On refroidit à 0°C un mélange de 5 g du composé obtenu à l'étape A de la Préparation 2.4 et 2,31 g de triéthylamine dans 100 ml de DCM, ajoute, goutte à goutte, 1,6 ml de chlorure d'acryloyle et laisse 2 heures sous agitation à 0°C. On lave le mélange réactionnel à l'eau, sèche sur $Na_2SO_4$ et évapore sous vide le solvant. On obtient 5,5 g du produit attendu sous forme d'huile.

B) (2S,4R)-2-[(diméthylamino)carbonyl]-4-[[3-(4-morpholinyl)propanoyl]oxy]-1-pyrrolidinecarboxylate de benzyle.

A une solution de 5,5 g du composé obtenu à l'étape précédente dans 100 ml de DCM, on ajoute 0,265 g de chlorure ferrique puis 2,13 g de morpholine et laisse 18 heures sous agitation à TA. On lave le mélange réactionnel par une solution saturée de $Na_2SO_4$, décante, sèche sur $Na_2SO_4$ et évapore le solvant sous vide. On chromatographie le résidu sur gel de silice en éluant au DCM puis par le mélange DCM/MeOH (94/6 ; v/v). On obtient 4,5 g du produit attendu sous forme d'huile.

C) 3-(4-morpholinyl)propionate de (3R,5S)-5-f(diméthylamino)carbonyl]-3-pyrrolidine.

On hydrogène pendant 3 heures, à TA et à pression atmosphérique, un mélange de 4,2 g du composé obtenu à l'étape précédente et 0,45 g de palladium sur charbon à 10 % dans 200 ml d'AcOEt. On filtre le catalyseur sur Célite et concentre de moitié sous vide le filtrat. On obtient une solution du produit attendu que l'on utilise telle quelle à la Préparation 3.40.

Préparations des composés de formule (II).

Préparations 3.1 et 3.2

(2S, 4R)-1-[ 5-Chloro-3-(2-méthoxyphényl)-2-oxo-2,3-dihydro-1*H*-indol-3-yl]-4-hydroxy-N,N-diméthyl-2-pyrrolidinecarboxamide, isomère A et isomère B.

(II):$R_1$=Cl;$R_2$=H;$R_3$=$OCH_3$;$R_4$=H;$R_5$=$N(CH_3)_2$;$R_6$=H.

On dissout le composé obtenu à la Préparation 2.1 b) dans 5 ml de DCM, ajoute 1,62 g de triéthylamine puis une suspension de 2,2 g du composé obtenu à la Préparation 1.1 dans 2 ml de THF et laisse 6 heures sous agitation à TA. On ajoute ensuite 3 x 0,8 g de triéthylamine sur une période de 24 heures tout en laissant sous agitation. A la fin de la réaction on observe la formation d'un abondant précipité. On essore le précipité formé, le reprend dans un mélange constitué d'une solution à 5 % de $K_2CO_3$ et de 100 ml d'AcOEt contenant 10 ml de MeOH, lave la phase organique par une solution à 5 % de $K_2CO_3$, par une solution saturée de NaCl, sèche sur $Na_2SO_4$ et évapore partiellement sous vide les solvants. On essore le précipité formé et obtient 0,875 g de l'isomère A. On réunit toutes les eaux mères d'essorage, et les chromatographie sur alumine en éluant par le gradient du mélange DCM/MeOH de (96/4 ; v/v) à (95/5 ; v/v). On sépare les deux isomères :

- le moins polaire, isomère A : composé de la Préparation 3.1 et obtient 0,359 g supplémentaire, F = 265-268°C. $\alpha_D^{25}$ = + 180° (c = 0,16 ; chloroforme).
- le plus polaire, isomère B : composé de la Préparation 3.2, que l'on recristallise dans le mélange DCM/éther iso et obtient 0,72 g, contenant 0,15 mole d'éther iso.
  $\alpha_D^{25}$ = - 193,7° (c = 0, 16 ; chloroforme).

Préparations 3.3 et 3.4

(2S, 4R)-1-[5-Chloro-3-(2-chlorophényl)-2-oxo-2,3-dihydro-1*H*-indol-3-yl]-4-hydroxy-N,N-diméthyl-2-pyrrolidinecarboxamide, isomère A et isomère B.

(II) : $R_1$ = Cl ; $R_2$ = H; $R_3$ = Cl ; $R_4$ = H ; $R_5$ = $N(CH_3)_2$ ; $R_6$ = H.

A un mélange de 3 g du composé obtenu à la Préparation 1.2 dans 50 ml de DCM, on ajoute à TA 0,8 g du composé obtenu à la Préparation 2.1 a) puis 3,5 ml de DIPEA et laisse 12 heures sous agitation à TA. On concentre sous vide le mélange réactionnel, extrait le résidu à l'AcOEt, lave la phase organique par une solution à 5 % de $K_2CO_3$, trois fois à l'eau, par une solution saturée de NaCl, sèche sur $Na_2SO_4$ et évapore sous vide le solvant. On chromatographie le résidu sur gel de silice en éluant par le mélange DCM/MeOH (95/5; v/v). On sépare les deux isomères :

- le moins polaire, isomère A : composé de la Préparation 3.3 que l'on rechromatographie sur alumine en éluant par le mélange DCM/MeOH (95/5; v/v) et obtient 0,182 g.
  $\alpha_D^{25}$ = +235,3° (c = 0,15 ; chloroforme).
- le plus polaire, isomère B : composé de la Préparation 3.4, que l'on rechromatographie sur alumine en éluant par le mélange DCM/MeOH (95/5; v/v). On obtient 0,68 g après cristallisation dans le mélange DCM/éther iso, F = 266-267°C.
  $\alpha_D^{25}$ = - 225,6° (c = 0.117 ; chloroforme).

Préparations 3.5 et 3.6

(2S, 4R)-1-[5-Méthyl-3-(2-méthoxyphényl)-2-oxo-2,3-dihydro-1*H*-indol-3-yl]-4-hydroxy-N,N-diméthyl-2-pyrrolidinecarboxamide, isomère A et isomère B.

(II): $R_1 = CH_3$; $R_2$ = H; $R_3 = OCH_3$; $R_4$ = H; $R_5 = N(CH_3)_2$; $R_6$ = H.

A un mélange de 1,5 g du composé obtenu à la Préparation 1.3 dans 15 ml de DCM et 3 ml de THF, on ajoute à TA 3,5 ml de DIPEA puis 1 g du composé obtenu à la Préparation 2.1 a) et laisse 5 heures sous agitation à TA. On concentre sous vide le mélange réactionnel, extrait le résidu à l'AcOEt, lave la phase organique par une solution à 5 % de $K_2CO_3$, trois fois à l'eau, par une solution saturée de NaCl, sèche sur $Na_2SO_4$ et évapore sous vide le solvant. On chromatographie le résidu sur gel de silice en éluant par le mélange DCM/MeOH (96/4; v/v). On sépare les deux isomères :

- le moins polaire, isomère A : composé de la Préparation 3.5 que l'on cristallise dans le mélange DCM/éther iso. On obtient 0,183 g, F = 257-258°C.
  $\alpha_D^{25}$ = + 151,6° (c = 0,122; chloroforme).
- le plus polaire, isomère B : composé de la Préparation 3.6, que l'on rechromatographie sur alumine en éluant par le mélange DCM/MeOH (97/3 ; v/v). On obtient 0,498 g que l'on utilise tel quel.

Préparations 3.7 et 3.8

(2S, 4R)-1-[3-(2-Méthoxyphényl)-5-trifluorométhoxy-2-oxo-2,3-dihydro-1*H*-indol-3-yl]-4-hydroxy-N,N-diméthyl-2-pyrrolidinecarboxamide, isomère A et isomère B.

(II):$R_1=OCF_3$ ; $R_2$=H ; $R_3=OCH_3$ ; $R_4$=H ; $R_5=N(CH_3)_2$ ; $R_6$=H.

A la solution du composé obtenu à la Préparation 1.4 dans le DCM, on ajoute 4 ml de DIPEA puis 1,26 g du composé obtenu à la Préparation 2.1 a) et laisse 4 heures sous agitation à TA. On concentre sous vide le mélange réactionnel, extrait le résidu à l'AcOEt, lave la phase organique par une solution à 5 % de $K_2CO_3$, deux fois à l'eau, par une solution saturée de NaCl, sèche sur $Na_2SO_4$ et évapore sous vide le solvant. On chromatographie le résidu sur alumine en éluant au DCM puis par le gradient du mélange DCM/MeOH jusqu'à (95,5/4,5 ; v/v). On sépare les deux isomères :

- le moins polaire, isomère A : composé de la Préparation 3.7 que l'on cristallise dans l'éther iso et obtient 0,09 g, F = 231-233°C.
  $\alpha_D^{25}$ = + 152° (c = 0,123 ; chloroforme).
- le plus polaire, isomère B : composé de la Préparation 3.8, et obtient 0,323 g, F = 219-220°C.
  $\alpha_D^{25}$ = - 220° (c = 0,11 ; chloroforme).

Préparations 3.9 et 3.10

(2S, 4R)-1-[5-Chloro-3-(2-méthoxyphényl)-6-méthyl-2-oxo-2,3-dihydro-1*H*-indol-3-yl]-4-hydroxy-N,N-diméthyl-2-pyrrolidinecarboxamide, isomère A et isomère B.

(II): $R_1$= Cl ; $R_2$ = 6-$CH_3$ ; $R_3 = OCH_3$ ; $R_4$ = H ; $R_5 = N(CH_3)_2$ ; $R_6$ = H.

On refroidit à 0°C la solution du composé obtenu à la Préparation 1.5 dans le DCM, ajoute 2,25 ml de DIPEA

puis 0,83 g du composé obtenu à la Préparation 2.1 a) et laisse 12 heures sous agitation en laissant remonter la température à TA. On concentre sous vide le mélange réactionnel, extrait le résidu à l'AcOEt, lave la phase organique par une solution à 5 % de $K_2CO_3$, à l'eau, par une solution saturée de NaCl, sèche sur $Na_2SO_4$ et évapore sous vide le solvant. On chromatographie le résidu sur gel de silice en éluant par le mélange DCM/MeOH (95/5 ; v/v). On sépare les deux isomères :

- le moins polaire, isomère A : composé de la Préparation 3.9 que l'on cristallise dans l'éther iso et obtient 0,139 g, F = 260-261 °C.
  $\alpha_D^{25}$ = + 162,5° (c = 0,144, chloroforme).
- le plus polaire, isomère B : composé de la Préparation 3.10, et obtient 0,606 g que l'on utilise tel quel.

Préparations 3.11 et 3.12

(2S, 4R)-1-[3-(2-Chlorophényl)-5,6-diméthyl-2-oxo-2,3-dihydro-1*H*-indol-3-yl]-4-hydroxy-N,N-diméthyl-2-pyrrolidinecarboxamide, isomère A et isomère B.

(11) : $R_1 = CH_3$ ; $R_2 = 6\text{-}CH_3$ ; $R_3 = Cl$ ; $R_4 = H$ ; $R_5 = N(CH_3)_2$ ; $R_6 = H$.

On refroidit à 0°C la solution du composé obtenu à la Préparation 1.6 dans le DCM, ajoute 0,6 ml de DIPEA puis 0,7 g du composé obtenu à la Préparation 2.1 a) et laisse une nuit sous agitation en laissant remonter la température à TA. On concentre sous vide, reprend par une solution à 5 % de $K_2CO_3$, extrait à l'AcOEt, sèche la phase organique sur $Na_2SO_4$ et évapore sous vide le solvant. On chromatographie le résidu sur gel de silice en éluant par le mélange DCM/MeOH (95/5 ; v/v). On sépare les deux isomères :

- le moins polaire, isomère A : composé de la Préparation 3.11.
- le plus polaire, isomère B : composé de la Préparation 3.12, et obtient 0,363 g, sous forme d'huile utilisée telle quelle.

Préparations 3.13 et 3.14

(2S, 4R)-1-[5-Chloro-3-(2,3-diméthoxyphényl)-2-oxo-2,3-dihydro-1*H*-indol-3-yl]-4-méthoxy-N,N-diméthyl-2-pyrrolidinecarboxamide, isomère A et isomère B.

(II) : $R_1 = Cl$ ; $R_2 = H$ ; $R_3 = OCH_3$ ; $R_4 = 3\text{-}OCH_3$ ; $R_5 = N(CH_3)_2$ ; $R_6 = CH_3$.

A une solution de 1,1 g du composé obtenu à la Préparation 1.7 dans 20 ml de DCM, on ajoute à TA 1,71 ml de DIPEA puis 0,75 g du composé obtenu à la Préparation 2.2 et laisse 3 heures sous agitation à TA. On concentre sous vide le mélange réactionnel, extrait le résidu à l'AcOEt, lave la phase organique par une solution à 5 % de $K_2CO_3$, deux fois à l'eau, par une solution saturée de NaCl, sèche sur $Na_2SO_4$ et évapore sous vide le solvant. On chromatographie le résidu sur alumine en éluant par le gradient du mélange DCM/MeOH de (98,5/1,5; v/v) à (98/2 ; v/v). On sépare les deux isomères :

- le moins polaire, isomère A : composé de la Préparation 3.13 et obtient 0,32 g.
- le plus polaire, isomère B : composé de la Préparation 3.14 que l'on recristallise dans l'éther iso et obtient 0,49 g, F = 235-237°C.
  $\alpha_D^{25}$ = - 160,7° (c = 0,102 ; chloroforme).

Préparations 3.15 et 3.16

(2S, 4R)-1-[5-Chloro-3-(2-méthoxyphényl)-6-trifluorométhyl-2-oxo-2,3-dihydro-1*H*-indol-3-yl]-4-méthoxy-N,N-diméthyl-2-pyrrolidinecarboxamide, isomère A et isomère B.

(II): $R_1 = Cl$ ; $R_2 = 6\text{-}CF_3$ ; $R_3 = OCH_3$ ; $R_4 = H$ ; $R_5 = N(CH_3)_2$ ; $R_6 = CH_3$.

A la solution du composé obtenu à la Préparation 1.8 dans 10 ml de DCM, on ajoute 2,5 ml de DIPEA et 0,870 g du composé obtenu à la Préparation 2.2 et laisse 10 heures sous agitation à TA. On concentre sous vide le mélange réactionnel, extrait le résidu à l'AcOEt, lave la phase organique par une solution à 5 % de $K_2CO_3$, deux fois à l'eau, par une solution saturée de NaCl, sèche sur $Na_2SO_4$ et évapore sous vide le solvant. On chromatographie le

résidu sur alumine en éluant par le gradient du mélange DCM/MeOH (98,5/1,5 ; v/v). On sépare les deux isomères :

- le moins polaire, isomère A : composé de la Préparation 3.15 que l'on cristallise dans le DCM et obtient 0,23 g, F = 291-293°C.
  $\alpha_D^{25}$ = + 131,6° (c = 0,12 ; chloroforme).
- le plus polaire, isomère B : composé de la Préparation 3.16 que l'on précipite dans l'hexane et obtient 0,44 g,F = 138-140°C.
  $\alpha_D^{25}$ = -157,1°(c=0,098 ; chloroforme).

Préparations 3.17 et 3.18

(2S, 4R)-1-[5-Chloro-3-(2-chlorophényl)-6-méthoxy-2-oxo-2,3-dihydro-1*H*-indol-3-yl]-4-méthoxy-N,N-diméthyl-2-pyrrolidinecarboxamide, isomère A et isomére B.

(II) : $R_1$ = Cl ; $R_2$ = 6-$OCH_3$ ; $R_3$ = Cl ; $R_4$ = H ; $R_5$ = $N(CH_3)_2$ ; $R_6$ = $CH_3$.

A la suspension du composé obtenu à la Préparation 1.9 dans le DCM, on ajoute sous atmosphère d'argon, 1,5 g du composé obtenu à la Préparation 2.2 puis, goutte à goutte, une solution de 1,8 g de DIPEA dans 2 ml de DCM, et laisse 2 heures sous agitation à TA. On concentre sous vide le mélange réactionnel, reprend le résidu par une solution à 5 % de $K_2CO_3$, extrait à l'AcOEt, lave la phase organique à l'eau, par une solution saturée de NaCl, sèche sur $Na_2SO_4$, concentre partiellement l'AcOEt, laisse en cristallisation et essore le précipité formé. On sépare un isomère :

- l'isomère A : composé de la Préparation 3.17 et obtient 0,581 g, F = 249-250°C.
  $\alpha_D^{25}$ = + 202,5° (c = 0,12 ; chloroforme).

Les jus d'essorage sont chromatographiés sur alumine en éluant par le mélange DCM/MeOH (98/2 ; v/v). On sépare l'autre isomère :

- le plus polaire, isomère B : composé de la Préparation 3.18 et obtient 0,519 g après cristallisation dans le mélange DCM/AcOEt, F = 243-244°C.
  $\alpha_D^{25}$ = -221,8° (c = 0,13 ; chloroforme).

Préparations 3.19 et 3.20

(2S, 4R)-1-[6-Chloro-3-(2-méthoxyphényl)-5-méthyl-2-oxo-2,3-dihydro-1*H*-indol-3-yl]-4-méthoxy-N,N-diméthyl-2-pyrrolidinecarboxamide, isomère A et isomére B.

(II) : $R_1$ = $CH_3$ ; $R_2$ = 6-Cl ; $R_3$ = $OCH_3$ ; $R_4$ = H ; $R_5$ = $N(CH_3)_2$ ; $R_6$ = $CH_3$.

A la solution du composé obtenu à la Préparation 1.10 dans le DCM, on ajoute 5,5 ml de DIPEA puis 1,85 g du composé obtenu à la Préparation 2.2 et laisse 12 heures sous agitation à TA. On concentre sous vide le mélange réactionnel, extrait le résidu à l'AcOEt, lave la phase organique par une solution à 5 % de $K_2CO_3$, deux fois à l'eau, par une solution saturée de NaCl, sèche sur $Na_2SO_4$ et évapore sous vide le solvant. On chromatographie le résidu sur alumine en éluant par le mélange DCM/MeOH de (99/1 ; v/v) à (98/2 ; v/v). On sépare les deux isomères :

- le moins polaire, isomère A : composé de la Préparation 3.19 et obtient 0,7 g après cristallisation dans l'éther iso, F = 264°C.
  $\alpha_D^{25}$ = + 183° (c = 0, 1 ; chloroforme).
- le plus polaire, isomère B : composé de la Préparation 3.20 et obtient 1,275 g après cristallisation dans l'éther iso, F = 245°C.
  $\alpha_D^{25}$ = -195,1° (c = 0,12 ; chloroforme).

Préparations 3.21 et 3.22

(2S, 4R)-1-[5-Chloro-3-(2-méthoxyphényl)-2-oxo-2,3-dihydro-1*H*-indol-3-yl]-4-éthoxy-N,N-diméthyl-2-pyrrolidinecarboxamide, isomère A et isomère B.

(II): $R_1$ = Cl; $R_2$ = H ; $R_3$ = $OCH_3$; $R_4$ = H; $R_5$ = $N(CH_3)_2$ ; $R_6$ = $-CH_2CH_3$.

On laisse 48 heures sous agitation à TA un mélange de 2,15 du composé obtenu à la Préparation 1.1, 2 g du composé obtenu à la Préparation 2.3 et 1,4 g de triéthylamine dans 50 ml de THF. On concentre sous vide, reprend le résidu à l'eau, extrait au DCM, sèche la phase organique sur $Na_2SO_4$ et évapore sous vide le solvant. On reprend le résidu par un mélange DCM/AcOEt (50/50 ; v/v), chauffe à reflux et laisse au repos. On essore le précipité formé et isole :

- l'isomère A : composé de la Préparation 3.21 et obtient 1,1 g, F = 236°C.
  $\alpha_D^{25}$ = + 109° (c = 0,22 ; chloroforme).

On chromatographie les jus d'essorage sur gel de silice en éluant par le mélangeAcOEt/MeOH (97/3 ; v/v) et sépare l'autre isomère :

- le plus polaire, isomère B : composé de la Préparation 3.22 et obtient 1 g.
  $\alpha_D^{25}$ = -164° (c = 0,25 ; chloroforme).

Préparations 3.23 et 3.24

(2S, 4R)-1-[5-Chloro-3-(2,3-diméthoxyphényl)-2-oxo-2,3-dihydro-1*H*-indol-3-yl]-4-hydroxy-N,N-diméthyl-2-pyrrolidinecarboxamide, isomère A et isomère B.

(II): $R_1$ = Cl ; $R_2$ = H ; $R_3$ = $OCH_3$ ; $R_4$ = 3-$OCH_3$ ; $R_5$ = $N(CH_3)_2$ ; $R_6$ = H.

A une solution de 1,6 g du composé obtenu à la Préparation 1.7 dans 10 ml de DCM, on ajoute à TA 2,5 ml de DIPEA puis 1 g du composé obtenu à la Préparation 2.1 a) et laisse 48 heures sous agitation à TA. On essore le précipité formé correspondant à l'isomère A ci-dessous. On concentre sous vide le filtrat, extrait le résidu à l'AcOEt, lave la phase organique par une solution à 5 % de $K_2CO_3$, à l'eau, par une solution saturée de NaCl, sèche sur $Na_2SO_4$ et évapore sous vide le solvant. On chromatographie le résidu sur gel de silice en éluant par le gradient du mélange DCM/MeOH de (99/1 ; v/v) à (93/7 ; v/v). On sépare les deux isomères :

- le moins polaire, isomère A : composé de la Préparation 3.23 que l'on recristallise avec le 1$^{er}$ jet ci-dessus dans le mélange DCM/éther iso, F = 261-263°C.
  $\alpha_D^{25}$ = +119,3° (c =0,135; chloroforme)
- le plus polaire, isomère B : composé de la Préparation 3.24 que l'on recristallise dans le mélange DCM/éther iso et obtient 0,94 g, F = 167-169°C.
  $\alpha_D^{25}$ = -168,6° (c=0,172; chloroforme)

Préparations 3.25 et 3.26

(2S, 4R)-1-[5,6-Dichloro-3-(2-chlorophényl)-2-oxo-2,3-dihydro-1*H*-indol-3-yl]-4-hydroxy-N,N-diméthyl-2-pyrrolidinecarboxamide, isomère A et isomère B.

(II): $R_1$ = Cl ; $R_2$ = 6-Cl ; $R_3$ = Cl; $R_4$ = H; $R_5$ = $N(CH_3)_2$; $R_6$ = H.

A un mélange de 0,8 g du composé obtenu à la Préparation 2.1 a) dans 15 ml de DCM, on ajoute à TA 1,6 g du composé obtenu à la Préparation 1.11 puis 2,13 ml de DIPEA et laisse 15 minutes sous agitation à TA. On concentre sous vide le mélange réactionnel, extrait le résidu à l'AcOEt, lave la phase organique par une solution à 5 % de $K_2CO_3$, à l'eau, par une solution saturée de NaCl, sèche sur $Na_2SO_4$ et évapore sous vide le solvant. On chromatographie le résidu sur gel de silice en éluant par le mélange DCM/MeOH (95/5; v/v). On sépare les deux isomères :

- le moins polaire, isomère A : composé de la Préparation 3.25 que l'on cristallise dans l'éther iso et obtient 0,08g, F > 260°C.

$\alpha_D^{25}$ = +219,4° (c = 0,103 ; chloroforme)

- le plus polaire, isomère B : composé de la Préparation 3.26 et obtient 0,661 g que l'on utilise tel quel.

Préparations 3.27 et 3.28

Ester méthylique de l'acide (2S, 4R)-1-[5-chloro-3-(2-méthoxyphényl)-2-oxo-2,3-dihydro-1*H*-indol-3-yl]-4-hydroxy-2-pyrrolidinecarboxylique, isomère A et isomère B.

(II] : $R_1$ =Cl; $R_2$ = H ; $R_3$ = $OCH_3$ ; $R_4$ = H ; $R_5$ = $OCH_3$ ; $R_6$ = H.

A un mélange de 1,4 g du composé obtenu à la Préparation 1.1 dans 20 ml de DCM, on ajoute à TA 4 ml de DIPEA puis 1,64 g de chlorhydrate de l'ester méthylique de l'acide (2S, 4R)-4-hydroxy-2-pyrrolidinecarboxylique et laisse 12 heures sous agitation à TA. On concentre sous vide, extrait le résidu à l'AcOEt, lave par une solution à 5 % de $K_2CO_3$, à l'eau, par une solution saturée de NaCl, sèche sur sulfate de sodium et évapore sous vide le solvant. On chromatographie le résidu sur gel de silice en éluant par le mélange DCM/MeOH (97/3 ; v/v). On sépare les deux isomères :

- l'isomère le moins polaire, isomère A : composé de la Préparation 3.27 et obtient 0,3 g, F = 234-235°C.
  $\alpha_D^{25}$ = + 143,3° (c = 0,136 ; chloroforme).
- l'isomère le plus polaire, isomère B : composé de la Préparation 3.28 que l'on recristallise dans le mélange DCM/éther iso/hexane et obtient 1,1 g.
  $\alpha_D^{25}$ = -199,1° (c =0,112; chloroforme)

Préparations 3.29

Ester méthylique de l'acide (2S, 4R)-1-[5-chloro-3-(2-méthoxyphényl)-6-méthyl-2-oxo-2,3-dihydro-1*H*-indol-3-yl]-4-hydroxy-2-pyrrolidinecarboxylique, mélange des deux diastéréoisomères.

(II): $R_1$ = Cl ; $R_2$ = 6-$CH_3$ ; $R_3$ = $OCH_3$ ; $R_4$ = H ; $R_5$ = $OCH_3$ ; $R_6$ = H.

On concentre sous vide la solution du composé obtenu à la Préparation 1.5 dans le DCM, reprend le résidu par un mélange de 20 ml de THF et 10 ml de DCM, ajoute à TA 0,715 g du chlorhydrate de l'ester méthylique de l'acide (2S, 4R)-4-hydroxy-2-pyrrolidinecarboxylique puis 0,8 g de triéthylamine et laisse 48 heures sous agitation à TA. On concentre sous vide, extrait le résidu au DCM, lave la phase organique à l'eau, sèche sur $Na_2SO_4$ et évapore sous vide le solvant. On chromatographie le résidu sur gel de silice en éluant par le mélange DCM/AcOEt (50/50; v/v). On obtient 1,8 g du mélange des deux diastéréoisomères.

Préparation 3.30

(2S, 4R)-1-[5-Chloro-3-(2-éthoxyphényl)-2-oxo-2,3-dihydro-1*H*-indol-3-yl]-4-hydroxy-N,N-diméthyl-2-pyrrolidinecarboxamide, isomère lévogyre.

(II) : $R_1$ = Cl ; $R_2$ = H ; $R_3$ = $OCH_2CH_3$ ; $R_4$ = H ; $R_5$ = $N(CH_3)_2$ ; $R_6$ = H.

A une solution de 2 g du composé obtenu à la Préparation 1.12 dans 20 ml de DCM, on ajoute 1,38 g du composé obtenu à la Préparation 2.1 a) puis 1,46 g de DIPEA et laisse 12 heures sous agitation à TA. On concentre sous vide, extrait le résidu à l'AcOEt, lave la phase organique par une solution à 5 % de $K_2CO_3$, sèche sur $Na_2SO_4$ et évapore le solvant sous vide. On chromatographie le résidu sur alumine en éluant par le mélange DCM/MeOH (95/5 ; v/v). On sépare les deux diastéréoisomères et recueille le composé le plus polaire que l'on rechromatographie sur gel de silice en éluant par le mélange DCM/AcOEt (60/40 ; v/v) puis par DCM/MeOH (94/6 ; v/v). On obtient 0,726 g du produit attendu.

Préparations 3.31 et 3.32

(2S, 4R)-1-[5-Chloro-3-(2,3-difluorophényl)-2-oxo-2,3-dihydro-1*H*-indol-3-yl]-4-hydroxy-N,N-diméthyl-2-pyrrolidinecarboxamide, isomère A et isomère B.

(II) : $R_1$ = Cl ; $R_2$ = H ; $R_3$ = F ; $R_4$ = 3-F ; $R_5$ = $N(CH_3)_2$ ; $R_6$ = H.

On laisse 2 heures sous agitation à TA un mélange de 0,4 g du composé obtenu à la Préparation 1.13, 0,3 g du composé obtenu à la Préparation 2.1 a) et 0,45 g de DIPEA dans 20 ml de DCM. On essore le précipité formé correspondant à l'isomère A, composé le moins polaire sur alumine, DCM/MeOH (98/2 ; v/v) (composé de la Préparation 3.31). On concentre sous vide les jus d'essorage, reprend le résidu par le mélange AcOEt/acétone, laisse 12 heures à froid et essore le précipité correspondant toujours à l'isomère A. On lave les jus d'essorage à l'eau, sèche la phase organique sur $Na_2SO_4$ et évapore le solvant sous vide. On chromatographie le résidu sur alumine en éluant par le mélange DCM/MeOH (98/2; v/v). On sépare l'autre isomère :

- le plus polaire, isomère B : composé de la Préparation 3.32 et obtient 0,1 g.
  $\alpha_D^{25}$ = - 231°(c=0,16 ;chloroforme).

Préparations 3.33 et 3.34

(2S, 4R)-1-[5-Chloro-3-(2,4-diméthoxyphényl)-2-oxo-2,3-dihydro-1*H*-indol-3-yl]-4-hydroxy-N,N-diméthyl-2-pyrrolidinecarboxamide, isomère A et isomère B.

(II): $R_1$ = Cl ; $R_2$ = H ; $R_3$ = $OCH_3$ ; $R_4$ = 4-$OCH_3$ ; $R_5$ = $N(CH_3)_2$ ; $R_6$ = H.

A une solution du composé obtenu à la Préparation 1.14 et 1 ml de triéthylamine dans 20 ml de DCM, on ajoute 1,5 g du composé obtenu à la Préparation 2.1 a) et laisse 1 heure sous agitation à TA. On lave deux fois le mélange réactionnel à l'eau, sèche la phase organique sur $Na_2SO_4$ et évapore le solvant sous vide. On chromatographie le résidu sur alumine en éluant au DCM puis par le mélange DCM/MeOH (98/2; v/v). On sépare les deux isomères:

- le moins polaire, isomère A : composé de la Préparation 3.33.
- le plus polaire, isomère B : composé de la Préparation 3.34 et obtient 0,26 g.
  $\alpha_D^{25}$ = - 157°(c =0,15; chloroforme).

Préparation 3.35

(2S, 4R)-1-[5-Chloro-3-(1,3-benzodioxol-4-yl)-2-oxo-2,3-dihydro-1*H*-indol-3-yl]-4-hydroxy-N,N-diméthyl-2-pyrrolidine-carboxamide, isomère lévogyre.

(II) : $R_1$ = Cl ; $R_2$ = H ; $R_3$ + $R_4$ = 2,3-O-$CH_2$-O- ; $R_5$ = $N(CH_3)_2$ ; $R_6$ = H.

On laisse 2 heures sous agitation à TA un mélange de 1,7 g du composé obtenu à la Préparation 1.15,0,9 g du composé obtenu à la Préparation 2.1 a) et 1 ml de DIPEA dans 20 ml de DCM. On lave le mélange réactionnel à l'eau, sèche la phase organique sur $Na_2SO_4$ et évapore le solvant sous vide. On chromatographie le résidu sur alumine en éluant par le mélange DCM/MeOH (97/3 ; v/v). On sépare les deux diastéréoisomères et recueille le composé le plus polaire. On obtient 0,42 g du produit attendu.
$\alpha_D^{25}$ = -108°(c=0,12;chloroforme)

Préparations 3.36 et 3.37

(2S, 4R)-1-[5,6-Dichloro-3-(2-méthoxyphényl)-2-oxo-2,3-dihydro-1*H*-indol-3-yl]-4-hydroxy-N,N-diméthyl-2-pyrrolidinecarboxamide, isomère A et isomère B.

(II): $R_1$ = Cl ; $R_2$ = 6-Cl ; $R_3$ = $OCH_3$ ; $R_4$ = H ; $R_5$ = $N(CH_3)_2$; $R_6$ = H.

On laisse 1 heure 30 minutes sous agitation à TA un mélange de 1,57 g du composé obtenu à la Préparation 1.16, 1,45 g du composé obtenu à la Préparation 2.1 a) et 0,8 ml de DIPEA dans 15 ml de DCM. On essore le précipité formé correspondant à l'isomère A, composé le moins polaire sur gel de silice, DCM/MeOH (94/6 ; v/v). On concentre

sous vide les jus d'essorage, extrait le résidu à l'AcOEt, lave la phase organique par une solution à 5 % de $K_2CO_3$, à l'eau, par une solution saturée de NaCl, sèche sur $Na_2SO_4$ et évapore le solvant sous vide. On chromatographie le résidu sur gel de silice en éluant par le mélange DCM/MeOH (94/6 ; v/v). On sépare les deux isomères :

- le moins polaire, isomère A : composé de la Préparation 3.36 que l'on cristallise dans le mélange éther iso/MeOH et obtient 0,295 g, F = 261-262°C.
  $\alpha_D^{25}$ = + 113,8° (c =0,12; chloroforme).
- le plus polaire, isomère B : composé de la Préparation 3.37 et obtient 0,74 g.

Préparations 3.38 et 3.39

Ester *tert*-butylique de l'acide 2-[[(3R, 5S)-1-[5-Chloro-3-(2-méthoxyphényl)-2-oxo-2,3-dihydro-1*H*-indol-3-yl]-5-[(diméthylamino)carbonyl)-3-pyrrolidinyl]oxy] acétique, isomère A et isomère B.

(II) : $R_1$ = Cl ; $R_2$ = H ; $R_3$ = $OCH_3$ ; $R_4$ = H ; $R_5$ = $N(CH_3)_2$; $R_6$ = $-CH_2COOC(CH_3)_3$.

A la solution du composé obtenu à la Préparation 2.4 on ajoute 200 ml de THF, 1,87 g de triéthylamine puis 4,5 g du composé obtenu à la Préparation 1.1 et chauffe à reflux pendant 48 heures. On concentre sous vide et chromatographie le résidu sur gel de silice en éluant par le mélange DCM/MeOH (96/4 ; v/v). On sépare les isomères :

- le moins polaire, isomère A : composé de la Préparation 3.38 et obtient 1 g.
- le plus polaire, isomère B : composé de la Préparation 3.39 et obtient 3 g sous forme d'huile.
  $\alpha_D^{25}$ = - 154° (c = 0,37 ; chloroforme).

Préparation 3.40

3-(4-morpholinyl)propanoate de (3R, 5S)-1-[5-Chloro-3-(2-méthoxyphényl)-2-oxo-2,3-dihydro-1*H*-indol-3-yl]-5-[(diméthylamino)carbonyl]-3-pyrrolidinyle, mélange des deux diastéréoisomères.

(II) : $R_1$ = Cl ; $R_2$ = H; $R_3$ = $OCH_3$ ; $R_4$ = H ; $R_5$ = $N(CH_3)_2$ ;

$R_6$ = $-CO-CH_2CH_2-$ 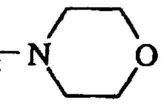

A la solution du composé obtenu à la Préparation 2.5 dans l'AcOEt, on ajoute une solution de 3 g du composé obtenu à la Préparation 1.1 dans 100 ml de THF et laisse 4 jours sous agitation à TA. On concentre sous vide, extrait le résidu à l'AcOEt, lave la phase organique à l'eau, sèche sur $Na_2SO_4$ et évapore le solvant sous vide. On chromatographie le résidu sur gel de silice en éluant par le mélange DCM/MeOH (92/8 ; v/v). On obtient 4,2 g du produit attendu sous forme de mousses.

EXEMPLE 1

(2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre, 0,25 éther iso.

(Ia) : $R_1$ = Cl ; $R_2$ = H ; $R_3$ = $OCH_3$ ; $R_4$ = H ; $R_5$ = $N(CH_3)_2$ ; $R_6$ = H; $R_7$ = 2-$OCH_3$ ; $R_8$ = $OCH_3$.

On refroidit à 0°C, sous atmosphère d'argon, un mélange de 0,67 g du composé obtenu à la Préparation 3.2 (isomère B) dans 10 ml de DMF, ajoute 0,069 g d'hydrure de sodium à 60 % dans l'huile et laisse sous agitation jusqu'à cessation du dégagement gazeux. On ajoute ensuite 0,404 g de chlorure de 2,4-diméthoxybenzènesulfonyle et laisse 3 heures sous agitation à TA. On verse le mélange réactionnel sur une solution à 5 % de $K_2CO_3$, extrait à l'AcOEt, lave la phase organique à l'eau, par une solution saturée de NaCl, sèche sur $Na_2SO_4$ et évapore sous vide le solvant. On chromatographie le résidu sur alumine en éluant par le mélange DCM/MeOH (99/1 ; v/v). On obtient 0,565 g du produit attendu après cristallisation dans le mélange DCM/éther iso.
$\alpha_D^{25}$ = -200° (c = 0,26; chloroforme).

RMN $^1H$: DMSO-$d_6$ + TFA, 360°K : $\delta$ (ppm) : 1,6 : mt : 2H ; 2,1 à 3,1 : m : 8H ; 3,35 : s : 3H ; 3,7 : s : 3H ; 3,9 :

s : 3H ; 4,4 : mt : 1H ; 4,6 : mt : 1H ; 6,6 à 8,1 : m : 10H.

EXEMPLE 2

(2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-méthoxy-N,N-diméthyl-2-pyrrolidinecarboxamide, isomère lévogyre.

(Ia) : $R_1$ = Cl ; $R_2$ = H ; $R_3$ = $OCH_3$ ; $R_4$ = H ; $R_5$ = $N(CH_3)_2$ ; $R_6$ = $CH_3$ ; $R_7$ = 2-$OCH_3$ ; $R_8$ = $OCH_3$.

A une solution de 0,559 g du composé obtenu à l'EXEMPLE 1 dans 6 ml de DMF, on ajoute, à TA et sous atmosphère d'argon, 0,04 g d'hydrure de sodium à 60 % dans l'huile et laisse sous agitation jusqu'à cessation du dégagement gazeux. On ajoute ensuite 0,11 ml d'iodure de méthyle et laisse 24 heures sous agitation à TA. On rajoute à nouveau 0,04 g d'hydrure de sodium à 60 % dans l'huile puis 0,33 ml d'iodure de méthyle et laisse 3 jours sous agitation à TA. On verse le mélange réactionnel dans l'eau, extrait à l'AcOEt, lave la phase organique à l'eau, par une solution saturée de NaCl, sèche sur $Na_2SO_4$ et évapore sous vide le solvant. On chromatographie le résidu sur gel de silice en éluant par le mélange DCM/MeOH (98/2 ; v/v). On obtient 0,082 g du produit attendu après cristallisation dans le mélange DCM/éther iso, F = 189-191°C.

EXEMPLE 3

(2S, 4R)-1-[5-Chloro-3-(2-chlorophényl)-1-[(2,4-diméthoxyphényl)sulfonyl]-2-oxo-2,3-dihydro-1*H*-indol-3-yl]-4-hydroxy-N,N-diméthyl-2-pyrrolidinecarboxamide, isomère lévogyre.

(Ia) : $R_1$ = Cl ; $R_2$ = H ; $R_3$ = Cl ; $R_4$ = H ; $R_5$ = $N(CH_3)_2$ ; $R_6$ = H; $R_7$ = 2-$OCH_3$ ; $R_8$ = $OCH_3$.

On refroidit à 0°C, sous atmosphère d'argon, un mélange de 0,567 g du composé obtenu à la Préparation 3.4 (isomère B) dans 5,5 ml de DMF, ajoute 0,062 g d'hydrure de sodium à 60 % dans l'huile et laisse 10 minutes sous agitation. On ajoute ensuite 0,338 g de chlorure de 2,4-diméthoxybenzènesulfonyle et laisse 3 heures sous agitation à TA. On ajoute de l'eau au mélange réactionnel, extrait trois fois à l'AcOEt, lave les phases organiques jointes par une solution saturée de NaCl, sèche sur $Na_2SO_4$ et évapore sous vide le solvant. On chromatographie le résidu sur gel de silice en éluant par le mélange DCM/MeOH (98/2 ; v/v). On obtient 0,647 g du produit attendu après cristallisation dans l'éther iso, F = 254-256°C.
$\alpha_D^{25}$ = - 250° (c = 0,142 ; chloroforme)

EXEMPLE 4

(2S, 4R)-1-(5-Chloro-3-(2-chlorophényl)-1-[(2,4-diméthoxyphényl)sulfonyl]-6-méthoxy-2-oxo-2,3-dihydro-1*H*-indol-3-yl]-4-méthoxy-N,N-diméthyl-2-pyrrolidinecarboxamide, isomère lévogyre.

(Ia) : $R_1$ = Cl ; $R_2$ = 6-$OCH_3$ ; $R_3$ = Cl ; $R_4$ = H ; $R_5$ = $N(CH_3)_2$ ; $R_6$ = $CH_3$ ; $R_7$ = 2-$OCH_3$ ; $R_8$ = $OCH_3$.

A une suspension de 0,719 g du composé obtenu à la Préparation 3.18 (isomère B) dans 7 ml de DMF, on ajoute à TA, sous atmosphère d'argon, 0,072 g d'hydrure de sodium à 60 % dans l'huile et laisse sous agitation jusqu'à cessation du dégagement gazeux. On ajoute ensuite 0,390 g de chlorure de 2,4-diméthoxybenzènesulfonyle et laisse 3 heures sous agitation à TA. On verse le mélange réactionnel sur une solution à 5 % de $K_2CO_3$, extrait à l'AcOEt puis au DCM, lave séparément les phases organiques à l'eau, les sèche sur $Na_2SO_4$, les réunit et concentre partiellement les solvants sous vide jusqu'à cristallisation. On essore le précipité formé et obtient 0,735 g du produit attendu, F = 283-288°C.
$\alpha_D^{25}$ = -266,3°(c=0,11;chloroforme).

En procédant selon les modes opératoires décrits dans les Exemples ci-dessus, à partir des composés de formule (II) décrits dans les Préparations 3 et le chlorure de 2,4-diméthoxybenzènesulfonyle, on prépare les composés selon l'invention rassemblés dans le TABLEAU I ci-après :

TABLEAU I

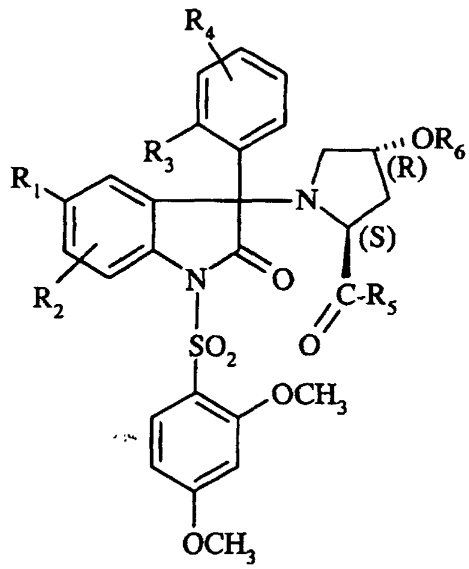

(Ia)

| Exemples | R1 | R2 | R3 | R4 | R5 | R6 | Solvat, hydrate ; F°C ; solvant de cristallisation ; $\alpha_D^{25}$ (chloroforme) |
|---|---|---|---|---|---|---|---|
| 5 (a) | $CH_3$ | H | $OCH_3$ | H | $-N(CH_3)_2$ | H | 0,65 $H_2O$ 162-164 éther iso -202,8° (c= ,139) |
| 6 (b) | $OCF_3$ | H | $OCH_3$ | H | $-N(CH_3)_2$ | H | - 147 DCM/hexane -223° (c=0,13) |
| 7 (c) | Cl | $6-CH_3$ | $OCH_3$ | H | $-N(CH_3)_2$ | H | - - éther iso -162,1° (c=0,103) |
| 8 (d) | $CH_3$ | $6-CH_3$ | Cl | H | $-N(CH_3)_2$ | H | 1 $H_2O$ 232 DCM/éther iso -239° (c=0,1) |
| 9 (e) | Cl | H | $OCH_3$ | $3-OCH_3$ | $-N(CH_3)_2$ | $-CH_3$ | - 233-234 DCM/éther iso -198° (c=0,11) |

| | | | | | | | |
|---|---|---|---|---|---|---|---|
| 10 (f) | Cl | 6-$CF_3$ | $OCH_3$ | H | -$N(CH_3)_2$ | -$CH_3$ | - 230-231 DCM/éther iso -170° (c=0,11) |
| 11 (g) | $CH_3$ | 6-Cl | $OCH_3$ | H | -$N(CH_3)_2$ | -$CH_3$ | - 238-240 DCM/éther iso -163,2° (c=0,12) |
| 12 (h) | Cl | H | $OCH_3$ | H | -$N(CH_3)_2$ | -$CH_2CH_3$ | - 169 éther iso/hexane -207° (c=0,2) |
| 13 (i) | Cl | H | $OCH_3$ | 3-$OCH_3$ | -$N(CH_3)_2$ | H | - 148-150 DMC/éther iso -207,3° (c=0,11) |
| 14 (j) | Cl | 6-Cl | Cl | H | -$N(CH_3)_2$ | H | - 181 éther iso -265,3° (c=0,17) |
| 15 (k) | Cl | H | $OCH_3$ | H | $OCH_3$ | H | - 185-186 DCM/éther iso -180,9° (c=0,15) |
| 16 (l) | Cl | 6-$CH_3$ | $OCH_3$ | H | $OCH_3$ | H | - 226 DCM/éther iso -131° (c=0,17) |
| 17 (m) | Cl | H | $OCH_2CH_3$ | H | -$N(CH_3)_2$ | H | - 135-149 éther/éther iso -188,3° (c = 0,11) |
| 18 (n) | Cl | H | F | 3-F | -$N(CH_3)_2$ | H | - - - -174° (c = 0,15) |
| 19 (o) | Cl | H | $OCH_3$ | 4-$OCH_3$ | -$N(CH_3)_2$ | H | - 183 éther iso -194° (c = 0,16) |

| | | | | | | | |
|---|---|---|---|---|---|---|---|
| 20 (p) | Cl | H | 2,3-O-$CH_2$-O- | | -$N(CH_3)_2$ | H | - 192 éther iso -200° (c = 0,16) |
| 21 (q) | Cl | 6-Cl | $OCH_3$ | H | -$N(CH_3)_2$ | H | - 160-161 éther iso/DCM -138,8° (c = 0,11) |

(a) On prépare ce composé selon le mode opératoire décrit à l'Exemple 3 à partir du composé obtenu à la Préparation 3.6 isomère B. On chromatographie sur gel de silice en éluant par le mélange DCM/MeOH (97/3 ; v/v).

(b) On prépare ce composé selon le mode opératoire décrit à l'Exemple 3 à partir du composé obtenu à la Préparation 3.8 isomère B. On chromatographie sur gel de silice en éluant par le mélange DCM/MeOH (96/4 ; v/v).

(c) On prépare ce composé selon le mode opératoire décrit à l'Exemple 3 à partir du composé obtenu à la Préparation 3.10 isomère B. On chromatographie sur gel de silice en éluant par le mélange DCM/MeOH (96/4 ; v/v).

(d) On prépare ce composé selon le mode opératoire décrit à l'Exemple 1 à partir du composé obtenu à la Préparation 3.12 isomère B. On chromatographie sur gel de silice en éluant par le mélange DCM/MeOH (98,5/1,5 ; v/v).

(e) On prépare ce composé selon le mode opératoire décrit à l'Exemple 1 à partir du composé obtenu à la Préparation 3.14 isomère B. On chromatographie sur gel de silice en éluant par le mélange DCM/MeOH (98,5/1,5 ; v/v).

(f) On prépare ce composé selon le mode opératoire décrit à l'Exemple 1 à partir du composé obtenu à la Préparation 3.16 isomère B. On chromatographie sur gel de silice en éluant par le mélange DCM/MeOH (98/2 ; v/v).

(g) On prépare ce composé selon le mode opératoire décrit à l'Exemple 1 à partir du composé obtenu à la Préparation 3.20 isomère B. On chromatographie sur gel de silice en éluant par le mélange DCM/MeOH (98,5/1,5 ; v/v).

(h) On prépare ce composé selon le mode opératoire décrit à l'Exemple 3 à partir du composé obtenu à la Préparation 3.22 isomère B. On chromatographie sur gel de silice en éluant par le mélange DCM/AcOEt (80/20 ; v/v).

(i) On prépare ce composé selon le mode opératoire décrit à l'Exemple 3 à partir du composé obtenu à la Préparation 3.24 isomère B. On chromatographie sur gel de silice en éluant par le mélange DCM/MeOH (91/9 ; v/v).

RMN[1]H : DMSO-$d_6$ : δ (ppm) : 1,4 à 3,3 : m : 10H ; 3,4 à 3,95 : 3s : 9H ; 4,2 à 5,0 : m : 3H ; 6,6 à 8,0 : m : 9H.

(j) On prépare ce composé selon le mode opératoire décrit à l'Exemple 3 à partir du composé obtenu à la Préparation 3.26 isomère B.

(k) On prépare ce composé selon le mode opératoire décrit à l'Exemple 3 à partir du composé obtenu à la Préparation 3.28 isomère B. On chromatographie sur gel de silice en éluant par le mélange DCM/MeOH (97/3 ; v/v).

(l) On prépare ce composé selon le mode opératoire décrit à l'Exemple 3 à partir du composé obtenu à la Préparation 3.29 (mélange de diastéréoisomères). On chromatographie sur gel de silice en éluant par le mélange DCM/AcOEt (50/50 ; v/v) et sépare l'isomère (-).

(m) On prépare ce composé selon le mode opératoire décrit à l'Exemple 3 à partir du composé obtenu à la Préparation 3.30. On chromatographie sur gel de silice en éluant par le mélange DCM/MeOH (95,5/4,5 ; v/v).

(n) On prépare ce composé selon le mode opératoire décrit à l'Exemple 3 à partir du composé obtenu à la Préparation 3.32 isomère B. On chromatographie sur alumine en éluant par le mélange DCM/AcOEt (97/3 ; v/v).

(o) On prépare ce composé selon le mode opératoire décrit à l'Exemple 3 à partir du composé obtenu à la Préparation 3.34 isomère B. On chromatographie sur gel de silice en éluant au DCM.

(p) On prépare ce composé selon le mode opératoire décrit à l'Exemple 3 à partir du composé obtenu à la Préparation 3.35. On chromatographie sur gel de silice en éluant au DCM puis par le mélange DCM/MeOH (99/1 ; v/v).

(q) On prépare ce composé selon le mode opératoire décrit à l'Exemple 3 à partir du composé obtenu à la Préparation 3.37. On chromatographie sur gel de silice en éluant par le mélange DCM/MeOH (96/4 ; v/v).

EXEMPLE 22

Ester *tert*-butylique de l'acide 2-[[(3R, 5S)-1-[5-chloro-1-((2,4-diméthoxy phényl)sulfonyl]-3-(2-méthoxyphényl)-2-oxo-2,3-dihydro-1*H*-indol-3-yl]-5-[(diméthyl amino)carbonyl]-3-pyrrolidinyl]oxy]acétique, isomère lévogyre.

(Ia) : $R_1$ = Cl ; $R_2$ = H ; $R_3$ = $OCH_3$ ; $R_4$ = H ; $R_5$ = $N(CH_3)_2$ ; $R_6$ = $-CH_2COOC(CH_3)_3$ ; $R_7$ = 2-$OCH_3$ ; $R_8$ = $OCH_3$.

On prépare ce composé selon le mode opératoire décrit à l'Exemple 3 à partir de 2,9 g du composé obtenu à la Préparation 3.39 (isomère B), 0,233 g d'hydrure de sodium à 60 % dans l'huile, 15 ml de DMF et 1,25 g de chlorure de 2,4-diméthoxybenzènesulfonyle. On chromatographie sur gel de silice en éluant par le mélange DCM/AcOEt (80/20 ; v/v). On obtient 3 g du produit attendu après cristallisation dans l'hexane.

$\alpha_D^{20}$ = -159° (c = 0,23 ; chloroforme).

EXEMPLE 23

0,55 Trifluoroacétate de l'acide 2-[((3R, SS)-1-[5-chloro-1-[(2,4-diméthoxy phényl)sulfonyl]-3-(2-méthoxyphényl)-2-oxo-2,3-dihydro-1*H*-indol-3-yl]-5-[(diméthyl amino)carbonyl]-3-pyrrolidinyl]oxy]acétique, isomère lévogyre.

(Ia) TFA : $R_1$ = Cl ; $R_2$ = H ; $R_3$ = $OCH_3$ ; $R_4$ = H ; $R_5$ = $N(CH_3)_2$ ; $R_6$ = $-CH_2COOH$ ; $R_7$ = 2-$OCH_3$ ; $R_8$ = $OCH_3$.

On laisse 3 heures sous agitation à TA un mélange de 3 g du composé obtenu à l'Exemple 22 et 15 ml de TFA dans 15 ml de DCM. On concentre sous vide le mélange réactionnel, reprend le résidu à l'éther iso et essore le précipité formé. On obtient 2,2 g du produit attendu.
$\alpha_D^{20}$ = - 179° (c = 0,31 ; chloroforme).

EXEMPLE 24

(2S, 4R)-1-[5-chloro-1-[(2,4-diméthoxyphényl)sulfonyl]-3-(2-méthoxyphényl)-2-oxo-2,3-dihydro-1*H*- indol-3-yl]-4-[2-[[2-hydroxy-1-(hydroxyméthyl)-1-méthyléthyl] amino]-2-oxoéthoxy]-N,N-diméthyl-2-pyrrolidinecarboxamide, isomère lévogyre.

(Ia) : $R_1$ = Cl ; $R_2$ = H ; $R_3$ = $OCH_3$ ; $R_4$ = H ; $R_5$ = $N(CH_3)_2$ ; $R_6$ = $-CH_2CONHC(CH_3)(CH_2OH)_2$ ; $R_7$ = 2-$OCH_3$ ; $R_8$ = $OCH_3$.

On laisse 3 heures sous agitation à TA un mélange de 0,5 g du composé obtenu à l'Exemple 23, 0,085 g de 2-amino-2-méthyl-1,3-propanediol, 0,290 g de BOP et 0,187 g de triéthylamine dans 20 ml de DCM. On dilue le mélange réactionnel par ajout de DCM, lave la phase organique à l'eau, par une solution saturée de $Na_2CO_3$, sèche sur $Na_2SO_4$ et évapore le solvant sous vide. On chromatographie le résidu sur gel de silice en éluant par le mélange DCM/MeOH (94/6; v/v). On obtient 0,31 g du produit attendu après cristallisation dans l'éther iso, F = 154°C.
$\alpha_D^{20}$ = - 142° (c = 0,19 ; chloroforme).

EXEMPLE 25

Ditrifluoroacétate de (2S, 4R)-1-[5-chloro-1-[(2,4-diméthoxyphényl)sulfonyl]-3-(2-méthoxyphényl)-2-oxo-2,3-dihydro-1*H*-indol-3-yl]-N,N-diméthyl-4-[2-oxo-2-(1-pipérazinyl)éthoxy]-2-pyrrolidinecarboxamide, isomère lévogyre.

(Ia) 2TFA : $R_1$ = Cl ; $R_2$ = H ; $R_3$ = $OCH_3$ ; $R_4$ = H ; $R_5$ = $-N(CH_3)_2$ ;

$R_6$ = 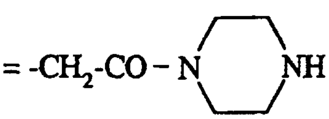 ;

$R_7$ = 2-$OCH_3$ ; $R_8$ = $OCH_3$.

A)

On laisse 2 heures sous agitation à TA un mélange de 0,7 g du composé obtenu à l'Exemple 23, 0,2 g de 1-(*tert*-butoxycarbonyl)pipérazine, 0,404 g de BOP et 0,263 g de triéthylamine dans 20 ml de DCM. On ajoute de l'eau au mélange réactionnel, extrait au DCM, lave la phase organique par une solution saturée de $Na_2CO_3$, sèche sur $Na_2SO_4$ et évapore le solvant sous vide. On chromatographie le résidu sur gel de silice en éluant par le mélange DCM/MeOH (97/3 ; v/v). On reprend le produit ainsi obtenu dans l'hexane, essore le précipité formé et obtient 0,7 g.

B)

On laisse 3 heures sous agitation un mélange de 0,7 g du composé obtenu à l'étape A et 10 ml de TFA dans 10 ml de DCM. On concentre sous vide, reprend le résidu dans l'éther et essore le précipité formé. On obtient 0,6 g du produit attendu, F = 166°C.
$\alpha_D^{25}$ = - 133° (c = 0,27 ; chloroforme).

EXEMPLE 26

(2S, 4R)-1-[(2,4-diméthoxyphényl)sulfonyl]-3-(2-méthoxyphényl)-2-oxo-2,3-dihydro-1*H*-indol-3-yl]-N,N-diméthyl-4-[2-oxo-2-(4-morpholinyl)éthoxy]-2-pyrrolidinecarboxamide, isomère lévogyre.

(Ia) : $R_1$ = Cl ; $R_2$ = H ; $R_3$ = $OCH_3$ ; $R_4$ = H ; $R_5$ =-$N(CH_3)_2$ ;

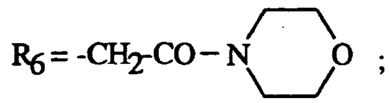

$R_7$ = 2-$OCH_3$ ; $R_8$ = $OCH_3$.

On laisse 2 heures sous agitation à TA, un mélange de 0,6 g du composé obtenu à l'Exemple 23, 0,085 g de morpholine, 0,347 g de BOP et 0,227 g de triéthylamine dans 20 ml de DCM. On extrait le mélange réactionnel au DCM, lave la phase organique à l'eau, sèche sur $Na_2SO_4$ et évapore le solvant sous vide. On chromatographie le résidu sur gel de silice en éluant par le mélange DCM/MeOH (95/5 ; v/v). On obtient 0,53 g du produit attendu après cristallisation dans l'éther iso, F = 210°C.
$\alpha_D^{20}$ = - 153° (c = 0,28 ; chloroforme).

EXEMPLE 27 et 28

3-(4-Morpholinyl)propanoate de (3R, 5S)-1-[5-chloro-1-[(2,4-diméthoxyphényl) sulfonyl]-3-(2-méthoxyphényl)-2-oxo-2,3-dihydro-1*H*-indol-3-yl]-5-[(diméthylamino) carbonyl]-3-pyrrolidinyle, isomère lévogyre et isomère dextrogyre.

(Ia) : $R_1$ Cl ; $R_2$ - H ; $R_3$ - $OCH_3$ ; $R_4$ = H ; $R_5$ = -$N(CH_3)_2$ ;

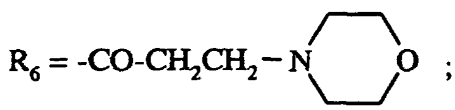

$R_7$ = 2-$OCH_3$ ; $R_8$ = $OCH_3$.

On prépare ces composés selon le mode opératoire décrit à l'Exemple 3 à partir de 3,1 g du composé obtenu à la Préparation 3.40, 20 ml de DMF, 0,238 g d'hydrure de sodium à 60 % dans l'huile et 1,27 g de chlorure de 2,4-diméthoxybenzènesulfonyle. On chromatographie sur gel de silice en éluant par le mélange DCM/MeOH (90/10 ; v/v). On sépare les deux diastéréoisomères :

- le moins polaire : composé de l'Exemple 27 dont on obtient 2,8 g après concrétisation dans l'hexane.
  $\alpha_D^{20}$ = - 154° (c = 0,3 ; chloroforme).
- le plus polaire : composé de l'Exemple 28 dont on obtient 1,3 g après concrétisation dans l'hyexane.
  $\alpha_D^{20}$ = + 127° (c = 0,29 ; chloroforme).

**Revendications**

**1.** Composé de formule (Ia) sous forme d'isomère lévogyre :

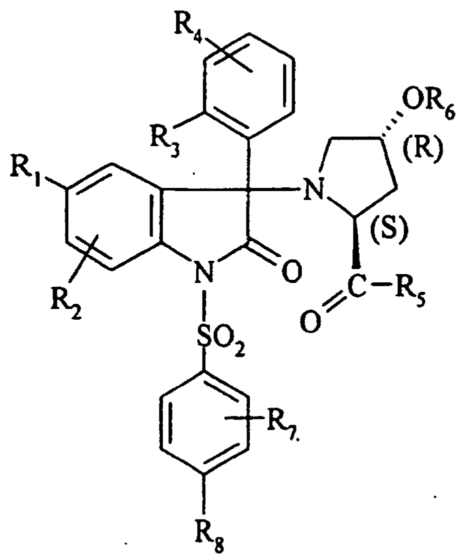

(Ia)

dans laquelle :

- $R_1$ représente un atome de chlore, un radical méthyle ou un radical trifluorométhoxy;
- $R_2$ représente un atome d'hydrogène ou est en position -6- de l'indol-2-one et représente un atome de chlore, un radical méthyle, un radical méthoxy ou un radical trifluorométhyle ;
- $R_3$ représente un atome de chlore, un atome de fluor, un radical méthoxy ou un radical éthoxy ;
- $R_4$ représente un atome d'hydrogène ou est en position -3- ou -4- du phényle et représente un atome de fluor ou un radical méthoxy ;
- ou bien $R_4$ est en position -3- du phényle et ensemble avec $R_3$ représentent un radical méthylènedioxy ;
- $R_5$ représente un radical diméthylamino ou un radical méthoxy ;
- $R_6$ représente un atome d'hydrogène ; un radical méthyle ; un radical éthyle ; un radical *tert*-butyloxycarbonylméthyle ; un radical carboxyméthyle ; un radical [[2-hydroxy-1-{hydroxyméthyl)-1-méthyléthyl]amino]carbonylméthyle ; un radical (1-pipérazinyl)carbonylméthyle ; un radical (4-morpholinyl) carbonylméthyle ; un radical 3-(4-morpholinyl)propanoyle ;
- $R_7$ est en position -2- du phényle et représente un radical méthoxy ;
- $R_8$ représente un radical méthoxy ;

et ses sels avec des acides minéraux ou organiques, ses solvats et/ou hydrates.

**2.** Un composé selon la revendication 1 choisi parmi :

- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-méthoxy-N,N-diméthyl-2-pyrrolidine carboxamide, isomère lévogyre ;
- (2S, 4R)-1-(5-Chloro-3-(2-chlorophényl)-1-[(2,4-diméthoxyphényl)sulfonyl]-2-oxo-2,3-dihydro-1*H* indol-3-yl]-4-hydroxy-N,N-diméthyl-2-pyrrolidine carboxamide, isomère lévogyre ;
- (2S, 4R)-1-[5-Chloro-3-(2-chlorophényl)-1-[(2,4-diméthoxyphényl)sulfonyl]-6-méthoxy-2-oxo-2,3-dihydro-1*H*-indol-3-yl]-4-méthoxy-N,N-diméthyl-2-pyrrolidinecarboxamide, isomère lévogyre ;
- (2S, 4R)-1-(5-Méthyl-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5-Trifluorométhoxy-1-((2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2-méthoxyphényl)-6-méthyl-2-oxo-2,3-dihydro-1*H*- indol-3-yl]-4-hydroxy-N,N-diméthyl-2-pyrrolidine carboxamide, isomère lévogyre ;
- (2S, 4R)-1-[3-(2-chlorophényl)-1-[(2,4-diméthoxyphényl)sulfonyl]-5,6-diméthyl
- 2-oxo-2,3-dihydro-1*H*-indol-3-yl]-4-hydroxy-N,N-diméthyl-2-pyrrolidine carboxamide, isomère lévogyre ;
- (2S, 4R)-1-[5-Chloro-1-[(2,4-diméthoxyphényl)sulfonyl]-3-(2,3-diméthoxyphényl)-2-oxo-2,3-dihydro-1*H*-indol-3-yl]-4-méthoxy-N,N-diméthyl-2-pyrrolidinecarboxamide, isomère lévogyre ;

- (2S, 4R)-1-[5-Chloro-1-[(2,4-diméthoxyphényl)sulfonyl]-3-(2-méthoxyphényl)-6-trifluorométhyl-2-oxo-2,3-dihydro-1*H*-indol-3-yl]-4-méthoxy-N,N-diméthyl-2-pyrrolidinecarboxamide, isomère lévogyre ;
- (2S, 4R)-1-[6-Chloro-1-[(2,4-diméthoxyphényl)sulfonyl]-3-(2-méthoxyphényl)-5-méthyl-2-oxo-2,3-dihydro-1*H*- indol-3-yl]-4-méthoxy-N,N-diméthyl-2-pyrrolidinecarboxamide, isomère lévogyre ;
- (2S, 4R)-1-[5-Chloro-1-[(2,4-diméthoxyphényl)sulfonyl]-3-(2-méthoxyphényl)-2-oxo-2,3-dihydro-1*H* indol-3-yl]-4-éthoxy-N,N-diméthyl-2-pyrrolidine carboxamide, isomère lévogyre ;
- (2S, 4R)-1-[5-Chloro-1-[(2,4-diméthoxyphényl)sulfonyl]-3-(2,3-diméthoxy phényl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5,6-Dichloro-3-(2-chlorophényl)-1-[(2,4-diméthoxyphényl)sulfonyl]
- 2-oxo-2,3-dihydro-1*H* indol-3-yl]-4-hydroxy-N,N-diméthyl-2-pyrrolidine carboxamide, isomère lévogyre;
- Ester méthylique de l'acide (2S, 4R)-1-[5-chloro-1-[(2,4-diméthoxyphényl) sulfonyl)-3-(2-méthoxyphényl)-2-oxo-2,3-dihydro-1*H*-indol-3-yl)-4-méthoxy-2-pyrrolidinecarboxylique, isomère lévogyre ;
- Ester méthylique de l'acide (2S,4R)-1-[5-chloro-1-[(2,4-diméthoxyphényl) sulfonyl]-3-(2-méthoxyphényl)-6-méthyl-2-oxo-2,3-dihydro-1*H*-indol-3-yl]-4-méthoxy-2-pyrrolidinecarboxylique, isomère lévogyre ;
- (2S, 4R)-1-[5-Chloro-1-[(2,4-diméthoxyphényl)sulfonyl]-3-(2-éthoxyphényl)-2-oxo-2,3-dihydro-1*H*-indol-3-yl]-4-hydroxy-N,N-diméthyl-2-pyrrolidine carboxamide, isomère lévogyre;
- (2S, 4R)-1-[5-Chloro-1-[(2,4-diméthoxyphényl)sulfonyl]-3-(2,3-difluorophényl)-2-oxo-2,3-dihydro-1*H*-indol-3-yl]-4-hydroxy-N,N-diméthyl-2-pyrrolidine carboxamide, isomère lévogyre ;
- (2S, 4R)-1-[5-Chloro-1-[(2,4-diméthoxyphényl)sulfonyl]-3-(2,4-diméthoxy phényl)-2-oxo-2,3-dihydro-1*H*-indol-3-yl]-4-hydroxy-N,N-diméthyl-2-pyrrolidine carboxamide, isomère lévogyre;
- (2S, 4R)-1-[5-Chloro-1-[(2,4-diméthoxyphényl)sulfonyl]-3-(1,3-benzodioxol-4-yl)-2-oxo-2,3-dihydro-1*H*- indol-3-yl)-4-hydroxy-N,N-diméthyl-2-pyrrolidine carboxamide, isomère lévogyre ;
- (2S, 4R)-1-[5,6-Dichloro-1-[(2,4-diméthoxyphényl)sulfonyl]-3-(2-méthoxy phényl)-2-oxo-2,3-dihydro-1*H*-indol-3-yl]-4-hydroxy-N,N-diméthyl-2-pyrrolidine carboxamide, isomère lévogyre;
- Ester *tert*-butylique de l'acide 2-[[(3R, 5S)-1-[5-chloro-1-[(2,4-diméthoxy phényl)sulfonyl]-3-(2-méthoxyphényl)-2-oxo-2,3-dihydro-1*H*-indol-3-yl)-5-[(diméthyl amino)carbonyl]-3-pyrrolidinyl]oxy]acétique, isomère lévogyre;
- Acide 2-[[(3R, 5S)-1-[5-chloro-1-[(2,4-diméthoxy phényl)sulfonyl]-3-(2-méthoxyphényl)-2-oxo-2,3-dihydro-1*H*-indol-3-yl)-S-[(diméthyl amino)carbonyl]-3-pyrrolidinyl]oxy]acétique, isomère lévogyre ;
- (2S, 4R)-1-[5-chloro-1-[(2,4-diméthoxyphényl)sulfonyl]-3-(2-méthoxyphényl)-2-oxo-2,3-dihydro-1*H* -indol-3-yl]-4-[2-[[2-hydroxy-1-(hydroxyméthyl)-1-méthyléthyl]amino]-2-oxoéthoxy]-N,N-diméthyl-2-pyrrolidinecarboxamide, isomère lévogyre ;
- (2S, 4R)-1-[5-chloro-1 -[(2,4-diméthoxyphényl)sulfonyl]-3-(2-méthoxyphényl)-2-oxo-2,3-dihydro-1*H*-indol-3-yl]-N,N-diméthyl-4-[2-oxo-2-(1-pipérazinyl) éthoxy]-2-pyrrolidinecarboxamide, isomère lévogyre ;
- (2S, 4R)-1-[(2,4-diméthoxyphényl)sulfonyl]-3-(2-méthoxyphényl)-2-oxo-2,3-dihydro-1*H*-indol-3-yl]-N,N-diméthyl-4-[2-oxo-2-(4-morpholinyl)éthoxy]-2-pyrrolidinecarboxamide, isomère lévogyre;
- 3-(4-Morpholinyl)propanoate de (3R, 5S)-1-[5-chloro-1-[(2,4-diméthoxyphényl) sulfonyl]-3-(2-méthoxyphényl)-2-oxo-2,3-dihydro-1*H*-indol-3-yl]-5-[(diméthylamino) carbonyl]-3-pyrrolidinyle, isomère lévogyre;

ainsi que ses sels éventuels avec des acides minéraux ou organiques, ses solvats et/ou hydrates.

**3.** Composé selon la revendication 2, qui est :

- (2S, 4R)-1-[5-Chloro-1-[(2,4-diméthoxyphényl)sulfonyl]-3-(2-méthoxyphényl)-2-oxo-2,3-dihydro-1*H*-indol-3-yl]-4-hydroxy-N,N-diméthyl-2-pyrrolidinecarboxamide, isomère lévogyre ;

**4.** Procédé de préparation des composés de formule (Ia) isomère lévogyre selon la revendication 1, de leurs sels éventuels avec des acides minéraux ou organiques, de leurs solvats et/ou hydrates **caractérisé en ce que** : on fait réagir, en présence d'une base, un composé de formule :

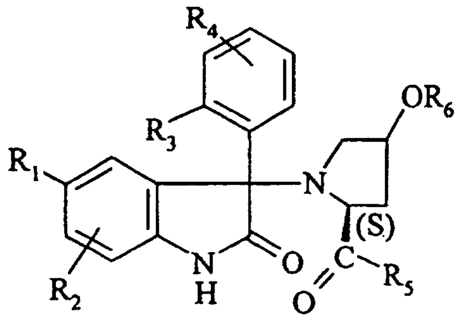

(II)

dans laquelle l'atome de carbone portant le substituant $OR_6$ a la configuration (R) et $R_1$, $R_2$, $R_3$, $R_4$, $R_5$ et $R_6$ sont tels que définis pour un composé de formule (Ia) isomère lévogyre dans la revendication 1, avec un halogénure de formule :

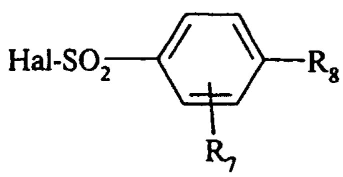

(III)

dans laquelle $R_7$ et $R_8$ sont tels que définis pour un composé de formule (Ia) isomère lévogyre dans la revendication 1 et Hal représente un atome d'halogène.

**5.** Composé de formule :

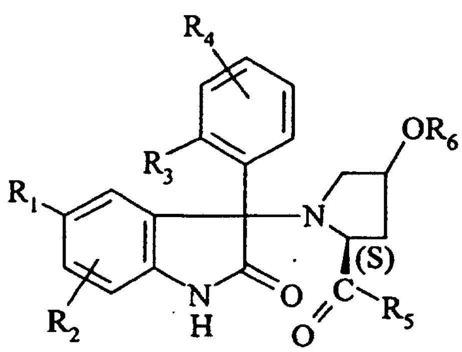

(II)

dans laquelle :

- l'atome de carbone portant le substituant $-OR_6$ a la configuration (R) ;
- $R_1$, $R_2$, $R_3$, $R_4$, $R_5$, $R_6$, sont tels que définis pour un composé de formule (Ia) isomère lévogyre dans la revendication 1 ;

et ses sels avec des acides minéraux ou organiques, sous forme d'isomère optiquement pur ou sous forme de mélange de diastéréoisomères.

**6.** Composition pharmaceutique comprenant en tant que principe actif un composé selon l'une quelconque des revendications 1 à 3, de ses sels avec des acides minéraux ou organiques, de ses solvats et/ou hydrates pharmaceutiquement acceptable.

**7.** Utilisation d'un composé selon l'une quelconque des revendications 1 à 3, de ses sels avec des acides minéraux ou organiques, de ses solvats et/ou hydrates pharmaceutiquement acceptables, pour la préparation de médicaments destinés à traiter toute pathologie où l'arginine-vasopressine et/ou ses récepteurs $V_{1b}$ ou à la fois ses

récepteurs $V_{1b}$ et ses récepteurs $V_{1a}$ sont impliqués.

**8.** Médicament **caractérisé en ce qu'**il est constitué d'un composé selon l'une quelconque des revendications 1 à 3.

## Claims

**1.** Compound of formula (Ia) in the form of a laevorotatory isomer:

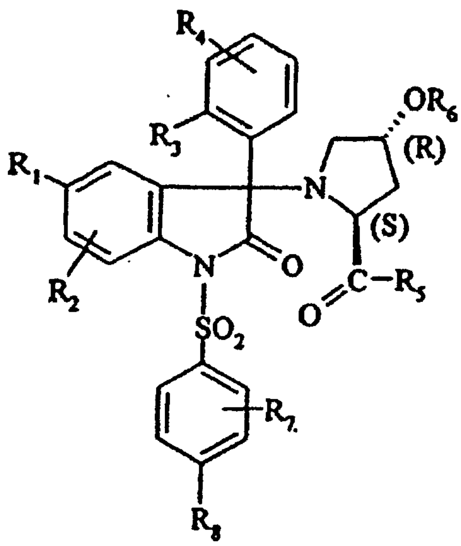

(Ia)

in which:

- $R_1$ represents a chlorine atom, a methyl radical or a trifluoromethoxy radical;
- $R_2$ represents a hydrogen atom or is at the 6-position of the indol-2-one and represents a chlorine atom, a methyl radical, a methoxy radical or a trifluoromethyl radical;
- $R_3$ represents a chlorine atom, a fluorine atom, a methoxy radical or an ethoxy radical;
- $R_4$ represents a hydrogen atom or is at the 3- or 4-position of the phenyl and represents a fluorine atom or a methoxy radical;
- or alternatively $R_4$ is at the 3-position of the phenyl and together with $R_3$ represents a methylenedioxy radical;
- $R_5$ represents a dimethylamino radical or a methoxy radical;
- $R_6$ represents a hydrogen atom; a methyl radical; an ethyl radical; a *tert*-butyloxycarbonylmethyl radical; a carboxymethyl radical; a [[2-hydroxy-1-(hydroxymethyl)-1-methylethyl]amino]carbonylmethyl radical; a (1-piperazinyl)carbonyl methyl radical; a (4-morpholinyl)carbonylmethyl radical; a 3-(4-morpholinyl)propanoyl radical;
- $R_7$ is at the 2-position of the phenyl and represents a methoxy radical;
- $R_8$ represents a methoxy radical;

and its salts with inorganic or organic acids, its solvates and/or hydrates.

**2.** Compound according to Claim 1, chosen from:

- (2S,4R)-1-[5-Chloro-1-[(2,4-dimethoxyphenyl)-sulphonyl]-3-(2-methoxyphenyl)-2-oxo-2,3-dihydro-1*H*-indol-3-yl]-4-hydroxy-N,N-dimethyl-2-pyrrolidinecarboxamide, laevorotatory isomer;
- (2S, 4R)-1-[5-Chloro-1-[(2,4-dimethoxyphenyl)-sulphonyl]-3-(2-methoxyphenyl)-2-oxo-2,3-dihydro-1*H*-indol-3-yl]-4-methoxy-N,N-dimethyl-2-pyrrolidinecarboxamide, laevorotatory isomer;
- (2S,4R)-1-[5-Chloro-3-(2-chlorophenyl)-1-[(2,4-dimethoxyphenyl)sulphonyl]-2-oxo-2,3-dihydro-1*H*-indol-3-yl]-4-hydroxy-N,N-dimethyl-2-pyrrolidinecarboxamide, laevorotatory isomer;
- (2S,4R)-1-[5-Chloro-3-(2-chlorophenyl)-1-[(2,4-dimethoxyphenyl)sulphonyl]-6-methoxy-2-oxo-2,3-dihydro-1*H*-indol-3-yl]-4-methoxy-N,N-dimethyl-2-pyrrolidinecarboxamide, laevorotatory isomer;
- (2S,4R)-1-[5-Methyl-1-[(2,4-dimethoxyphenyl)-sulphonyl]-3-(2-methoxyphenyl)-2-oxo-2,3-dihydro-1*H*-indol-3-yl]-4-hydroxy-N,N-dimethyl-2-pyrrolidinecarboxamide, laevorotatory isomer;

- (2S,4R)-1-[5-Trifluoromethoxy-1-[(2,4-dimethoxyphenyl)sulphonyl]-3-(2-methoxyphenyl)-2-oxo-2,3-dihydro-1*H*-indol-3-yl]-4-hydroxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2-methoxyphenyl)-6-methyl-2-oxo-2,3-dihydro-1*H*-indol-3-yl]-4-hydroxy-N,N-dimethyl-2-pyrrolidinecarboxamide, laevorotatory isomer;
- (2S,4R)-1-[3-(2-Chlorophenyl)-1-[(2,4-dimethoxyphenyl)sulphonyl]-5,6-dimethyl-2-oxo-2,3-dihydro-1*H*-indol-3-yl]-4-hydroxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2,3-dimethoxyphenyl)-2-oxo-2,3-dihydro-1*H*-indol-3-yl]-4-methoxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2-methoxyphenyl)-6-trifluoromethyl-2-oxo-2,3-dihydro-1*H*-indol-3-yl]-4-methoxy-N,N-dimethyl-2-pyrrolidinecarboxamide, laevorotatory isomer;
- (2S,4R)-1-[6-Chloro-1-[(2,4-dimethoxyphenyl)-sulphonyl]-3-(2-methoxyphenyl)-5-methyl-2-oxo-2,3-dihydro-1*H*-indol-3-yl]-4-methoxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2-methoxyphenyl)-2-oxo-2,3-dihydro-1*H*-indol-3-yl]-4-ethoxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2,3-dimethoxyphenyl)-2-oxo-2,3-dihydro-1*H*-indol-3-yl]-4-hydroxy-N,N-dimethyl-2-pyrrolidinecarboxamide, laevorotatory isomer;
- (2S,4R)-1-[5,6-Dichloro-3-(2-chlorophenyl)-1-[(2,4-dimethoxyphenyl)sulphonyl]-2-oxo-2,3-dihydro-1*H*-indol-3-yl]-4-hydroxy-N,N-dimethyl-2-pyrrolidinecarboxamide, laevorotatory isomer;
- Methyl ester of (2S,4R)-1-[5-chloro-1-[(2,4-dimethoxyphenyl)sulphonyl]-3-(2-methoxyphenyl)-2-oxo-2,3-dihydro-1*H*-indol-3-yl)-4-methoxy-2-pyrrolidinecarboxylic acid, laevorotatory isomer;
- Methyl ester of (2S,4R)-1-[5-chloro-1-[(2,4-dimethoxyphenyl)sulphonyl)-3-(2-methoxyphenyl)-6-methyl-2-oxo-2,3-dihydro-1H-indol-3-yl]-4-methoxy-2-pyrrolidinecarboxylic acid, laevorotatory isomer;
- (2S,4R)-1-[5-Chloro-1-[(2,4-dimethoxyphenyl)-sulphonyl]-3-(2-ethoxyphenyl)-2-oxo-2,3-dihydro-1*H*-indol-3-yl]-4-hydroxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2,3-difluorophenyl)-2-oxo-2,3-dihydro-1*H*-indol-3-yl)-4-hydroxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(2,4-dimethoxyphenyl)-2-oxo-2,3-dihydro-1*H*-indol-3-yl)-4-hydroxy-N,N-dimethyl-2-pyrrolidinecarboxamide, laevorotatory isomer;
- (2S,4R)-1-[5-Chloro-1-[(2,4-dimethoxyphenyl)-sulphonyl]-3-(1,3-benzodioxol-4-yl)-2-oxo-2,3-dihydro-1*H*-indol-3-yl]-4-hydroxy-N,N-dimethyl-2-pyrrolidinecarboxamide, laevorotatory isomer;
- (2S,4R)-1-[5,6-Dichloro-1-[(2,4-dimethoxyphenyl)sulphonyl]-3-(2-methoxyphenyl)-2-oxo-2,3-dihydro-1*H*-indol-3-yl]-4-hydroxy-N,N-dimethyl-2-pyrrolidinecarboxamide, laevorotatory isomer;
- tert-Butyl ester of 2-[[(3R,5S)-1-[5-chloro-1-[(2,4-dimethoxyphenyl)sulphonyl]-3-(2-methoxyphenyl)-2-oxo-2,3-dihydro-1*H*-indol-3-yl]-5-[(dimethylamino)carbonyl]-3-pyrrolidinyl]oxy]-acetic acid, laevorotatory isomer;
- 2-[[(3R,5S)-1-[5-Chloro-1-[(2,4-dimethoxyphenyl)sulphonyl]-3-(2-methoxyphenyl)-2-oxo-2,3-dihydro-1*H*-indol-3-yl]-5-[(dimethylamino)-carbonyl]-3-pyrrolidinyl]oxy]acetic acid, laevorotatory isomer;
- (2S,4R)-1-[5-Chloro-1-[(2,4-dimethoxyphenyl)-sulphonyl]-3-(2-methoxyphenyl)-2-oxo-2,3-dihydro-1*H*-indol-3-yl]-4-[2-[[2-hydroxy-1-(hydroxymethyl)-1-methylethyl]amino]-2-oxoethoxy]-N,N-dimethyl-2-pyrrolidinecarboxamide, laevorotatory isomer;
- (2S,4R)-1-[5-Chloro-1-[(2,4-dimethoxyphenyl)-sulphonyl]-3-(2-methoxyphenyl)-2-oxo-2,3-dihydro-1*H*-indol-3-yl]-N,N-dimethyl-4-[2-oxo-2-(1-piperazinyl)ethoxy]-2-pyrrolidinecarboxamide, laevorotatory isomer;
- (2S,4R)-1-[(2,4-dimethoxyphenyl)sulphonyl]-3-(2-methoxyphenyl)-2-oxo-2,3-dihydro-1*H*-indol-3-yl]-N,N-dimethyl-4-[2-oxo-2-(4-morpholinyl)ethoxy]-2-pyrrolidinecarboxamide, laevorotatory isomer;
- (3R,5S)-1-[5-Chloro-1-[(2,4-dimethoxyphenyl)-sulphonyl]-3-(2-methoxyphenyl)-2-oxo-2,3-dihydro-1*H*-indol-3-yl]-5-[(dimethylamino)carbonyl]-3-pyrrolidinyl 3-(4-morpholinyl)propanoate, laevorotatory isomer;

and its possible salts with inorganic or organic acids, its solvates and/or hydrates.

**3.** Compound according to Claim 2, which is:

- (2S,4R)-1-[5-Chloro-1-[(2,4-dimethoxyphenyl)-sulphonyl]-3-(2-methoxyphenyl)-2-oxo-2,3-dihydro-1*H*-indol-3-yl]-4-hydroxy-N,N-dimethyl-2-pyrrolidinecarboxamide, laevorotatory isomer.

**4.** Method for preparing the compounds of formula (Ia), laevorotatory isomer, according to Claim 1, their possible salts with inorganic or organic acids, their solvates and/or hydrates, **characterized in that**: a compound of formula:

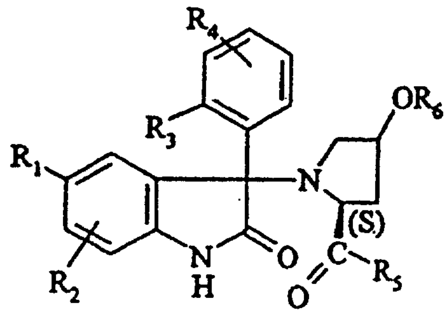

(II)

in which the carbon atom carrying the substituent $OR_6$ has the (R) configuration and $R_1$, $R_2$, $R_3$, $R_4$, $R_5$ and $R_6$ are as defined for a compound of formula (Ia), laevorotatory isomer, in Claim 1, is reacted, in the presence of a base, with a halide of formula:

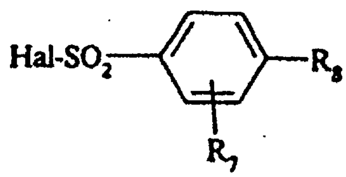

(III)

in which $R_7$ and $R_8$ are as defined for a compound of formula (Ia), laevorotatory isomer, in Claim 1 and Hal represents a halogen atom.

**5.** Compound of formula:

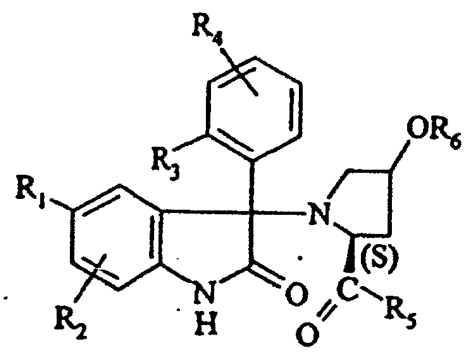

(II)

in which:

- the carbon atom carrying the substituent $-OR_6$ has the (R) configuration;
- $R_1$, $R_2$, $R_3$, $R_4$, $R_5$ and $R_6$ are as defined for a compound of formula (Ia), laevorotatory isomer, in Claim 1;

and its salts with inorganic or organic acids, in the form of an optically pure isomer or in the form of the mixture of diastereoisomers.

**6.** Pharmaceutical composition comprising, as active ingredient, a compound according to any one of Claims 1 to 3, its salts with inorganic or organic acids, its solvates and/or hydrates which are pharmaceutically acceptable.

**7.** Use of a compound according to any one of Claims 1 to 3, of its salts with inorganic or organic acids, of its solvates and/or hydrates which are pharmaceutically acceptable, for the preparation of medicaments intended for treating any pathology where arginine-vasopressin and/or its $V_{1b}$ receptors or both its $V_{1b}$ receptors and its $V_{1a}$ receptors

are involved.

**8.** Medicament, **characterized in that** it consists of a compound according to any one of Claims 1 to 3.

**Patentansprüche**

**1.** Verbindung der Formel (Ia) in Form des linksdrehenden Isomeren:

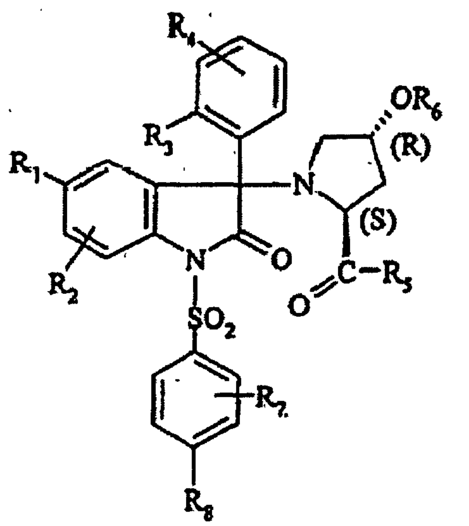

(Ia)

in der:

- $R_1$ ein Chloratom, eine Methylgruppe oder eine Trifluormethoxygruppe bedeutet;
- $R_2$ ein Wasserstoffatom bedeutet oder in der Stellung -6- des Indol-2-ons steht und ein Chloratom, eine Methylgruppe, eine Methoxygruppe oder eine Trifluormethylgruppe bedeutet;
- $R_3$ ein Chloratom, ein Fluoratom, eine Methoxygruppe oder eine Ethoxygruppe darstellt;
- $R_4$ ein Wasserstoffatom bedeutet oder in der Stellung -3- oder -4- des Phenyls steht und ein Fluoratom oder eine Methoxygruppe bedeutet;

oder $R_4$ in der Stellung -3- des Phenyls steht und zusammen mit $R_3$ eine Methylendioxygruppe bedeutet;

- $R_5$ eine Dimethylaminogruppe oder eine Methoxygruppe bedeutet;
- $R_6$ ein Wasserstoffatom; eine Methylgruppe; eine Ethylgruppe; eine *tert.*-Butyloxycarbonylmethylgruppe; eine Carboxymethylgruppe; eine [[2-Hydroxy-1-(hydroxymethyl)-1-methylethyl]-amino]-carbonylmethylgruppe; eine (1-Piperazinyl)-carbonylmethylgruppe; eine (4-Morpholinyl)-carbonylmethylgruppe; oder eine 3-(4-Morpholinyl)-propanoylgruppe darstellt;
- $R_7$ in der Stellung -2- des Phenyls steht und eine Methoxygruppe bedeutet;
- $R_8$ eine Methoxygruppe bedeutet;

und deren Salze mit anorganischen oder organischen Säuren, deren Solvate und/ oder Hydrate.

**2.** Verbindung nach Anspruch 1, ausgewählt aus:

- (2S, 4R)-1-[5-Chlor-1-[(2,4-dimethoxyphenyl)-sulfonyl]-3-(2-methoxyphenyl)-2-oxo-2, 3-dihydro-1*H*-indol-3-yl]-4-hydroxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2-methoxyphenyl)-2-oxo-2,3-dihydro-1*H*-indol-3-yl]-4-methoxy-N,N-dimethyl-2-pyrrolidin-carboxamid, linksdrehendes Isomeres;
- (2S, 4R)-1-[5-Chlor-1-(2-chlorphenyl)-1-[(2,4-dimethoxyphenyl)-sulfonyl]-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-[5-Chlor-3-(2-chlorphenyl)-1-[(2,4-dimethoxyphenyl)-sulfonyl]-6-methoxy-2-oxo-2,3-dihydro-1*H*-

indol-3-yl]-4-methoxy-N,N-dimethyl-2-pyrrolidincarboxamid, linksdrehendes Isomeres;

- (2S, 4R)-1-[5-Methyl-1-[(2,4-dimethoxyphenyl)-sulfonyl]-3-(2-methoxyphenyl)-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-[5-Trifluormethoxy-1-[(2,4-dimethoxyphenyl)-sulfonyl)-3-(2-methoxyphenyl)-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2-methoxyphenyl)-6-methyl-2-oxo-2,3-dihydro-1*H*-indol-3-yl]-4-hydroxy-N,N-dimethyl-2-pyrrolidincarboxamid, linksdrehendes Isomeres;
- (2S, 4R)-1-[3-(2-Chlorphenyl)-1-[(2,4-dimethoxyphenyl)-sulfonyl]-5,6-dimethyl-2-oxo-2, 3-dihydro-1*H*-indol-3-yl]-4-hydroxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2,3-dimethoxyphenyl)-2-oxo-2,3-dihydro-1*H*-indol-3-yl]-4-methoxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2-methoxyphenyl)-6-trifluormethyl-2-oxo-2,3-dihydro-1*H*-indol-3-yl]-4-methoxy-N, N-dimethyl-2-pyrrolidin-carboxamid, linksdrehendes Isomeres;
- (2S, 4R)-1-[6-Chlor-1-[(2,4-dimethoxyphenyl)-sulfonyl]-3-(2-methoxyphenyl)-5-methyl-2-oxo-2,3-dihydro-1*H*-indol-3-yl]-4-methoxy-N,N-dimethyl-2-pyrrolidincarboxamid, linksdrehendes Isomeres;
- (2S, 4R)-1-[5-Chlor-1-[(2,4-dimethoxyphenyl)-sulfonyl]-3-(2-methoxyphenyl)-2-oxo-2,3-dihydro-1*H*-indol-3-yl]-4-ethoxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2-methoxyphenyl)-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-[5,6-Dichlor-3-(2-chlorphenyl)-1-[(2,4-dimethoxyphenyl)-sulfonyl]-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-(5-Chlor-1-[(2,4-dimethoxyphenyl)-sulfonyl]-3-(2-methoxyphenyl)-2-oxo-2, 3-dihydro-1*H*-indol-3-yl] -4-methoxy-2-2-pyrrolidin-carbonsäuremethylester, linksdrehendes Isomeres;
- (2S, 4R)-1-[5-Chlor-1-[(2,4-dimethoxyphenyl)-sulfonyl]-3-(2-methoxyphenyl)-6-methyl-2-oxo-2,3-dihydro-1*H*-indol-3-yl]-4-methoxy-2-pyrrolidin-carbonsäuremethylester, linksdrehendes Isomeres;
- (2S, 4R)-1-[5-Chlor-1-[(2,4-dimethoxyphenyl)-sulfonyl]-3-(2-ethoxyphenyl)-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2,3-difluorphenyl)-2-oxo-2,3-dihydro- 1H-indol-3-yl]0-4-hydroxy-N,N-dimethyl-2-pyrrolidin-carboxamid, linksdrehendes Isomeres;
- (2S, 4R)-1-[5-Chlor-1-[(2,4-dimethoxyphenyl)-sulfonyl]-3-(2,4-dimethoxyphenyl)-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-(5-Chlor-1-[(2,4-dimethoxyphenyl)-sulfonyl]-3-(1,3-benzodioxol-4-yl)-2-oxo-2,3-dihydro-1*H*-indol-3-yl]-4-hydroxy-N,N-dimethyl-2-pyrrolidin-carboxamid, linksdrehendes Isomeres;
- (2S, 4R)-1-[5,6-Dichlor-1-[(2,4-dimethoxyphenyl)-sulfonyl]-3-(2-methoxyphenyl)-2-oxo-2,3-dihydro-1*H*-indol-3-yl]-4-hydroxy-N,N-dimethyl-2-pyrrolidin-carboxamid, linksdrehendes Isomeres;
- 2-[[(3R, 5S)-1-[5-Chlor-1-[(2,4-dimethoxyphenyl)-sulfonyl]-3-(2-methoxyphenyl)-2-oxo-2,3-dihydro-1*H*-indol-3-yl]-5-[(dimethylamino)-carbonyl]-3-pyrrolidinyl]-oxy]-essigsäure-*tert*.-butylester, linksdrehendes Isomeres;
- 2-[[(3R, 5S)-1-[5-Chlor-1-[(2,4-dimethoxyphenyl)-sulfonyl]-3-(2-methoxyphenyl)-2-oxo-2,3-dihydro-1*H*-indol-3-yl]-5-[(dimethylamino)-carbonyl]-3-pyrrolidinyl]-oxy]-essigsäure, linksdrehendes Isomeres;
- (2S, 4R)-1-[5-Chlor-1-[(2,4-dimethoxyphenyl)-sulfonyl]-3-(2-methoxyphenyl)-2-oxo-2,3-dihydro-1*H*-indol-3-yl]-4-[2-[[2-hydroxy-1-(hydroxymethyl)-1-methylethyl]-amino]-2-oxoethoxy]-N,N-dimethyl-2-pyrrolidincarboxamid, linksdrehendes Isomeres;
- (2S, 4R)-1-[5-Chlor-1-[(2,4-dimethoxyphenyl)-sulfonyl]-3-(2-methoxyphenyl)-2-oxo-2,3-dihydro-1*H*-indol-3-yl]-N,N-dimethyl-4-[2-oxo-2-(1-piperazinyl)-ethoxy]-2-pyrrolidin-carboxamid, linksdrehendes Isomeres;
- (2S. 4R)-1-[(2,4-Dimethoxyphenyl)-sulfonyl]-3-(2-methoxyphenyl)-2-oxo-2,3-dihydro-1*H*-indol-3-yl]-N,N-dimethyl-4-[2-oxo-2-(4-morpholinyl)-ethoxy]-2-pyrrolidin-carboxamid, linksdrehendes Isomeres;
- (3R, 5S)-1-[5-Chlor-1-[(2,4-dimethoxyphenyl)-sulfonyl]-3-(2-methoxyphenyl)-2-oxo-2,3-dihydro-1*H*-indol-3-yl] 5-[(dimethylamino)-carbonyl]-3-pyrrolidinyl-3-(4-morpholinyl)-propanoat, linksdrehendes Isomeres;

sowie deren eventuellen Salze mit anorganischen oder organischen Säuren, deren Solvate und/oder Hydrate.

**3.** Verbindung nach Anspruch 2, nämlich:

- (2S, 4R)-1-[5-Chlor-1-[(2,4-dimethoxyphenyl)-sulfonyl]-3-(2-methoxyphenyl)-2-oxo-2,3-dihydro-1*H*-indol-3-yl]-4-hydroxy-N,N-dimethyl-2-pyrrolidin-carboxamid, linksdrehendes Isomeres.

**4.** Verfahren zur Herstellung der Verbindungen der Formel (Ia) in Form des linksdrehenden Isomeren nach Anspruch 1, von deren eventuellen Salzen mit anorganischen oder organischen Säuren, von deren Solvaten und/oder deren

Hydraten, **dadurch gekennzeichnet, daß** man: eine Verbindung der Formel:

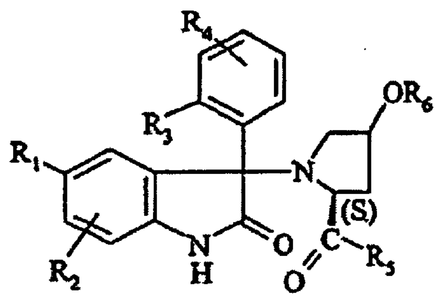

(II)

in der das den Substituenten $OR_6$ tragende Kohlenstoffatom die Konfiguration (R) aufweist und $R_1$, $R_2$, $R_3$, $R_4$, $R_5$ und $R_6$ die bezüglich der Verbindung der Formel (Ia) in Form des linksdrehenden Isomeren in Anspruch 1 angegebenen Bedeutungen besitzen, in Gegenwart einer Base mit einem Halogenid der Formel umsetzt:

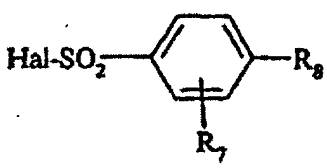

(III)

in der $R_7$ und $R_8$ die bezüglich der Verbindung der Formel (Ia) in Form des linksdrehenden Isomeren in Anspruch 1 angegebenen Bedeutungen besitzen und Hal ein Halogenatom darstellt.

**5.** Verbindung der Formel:

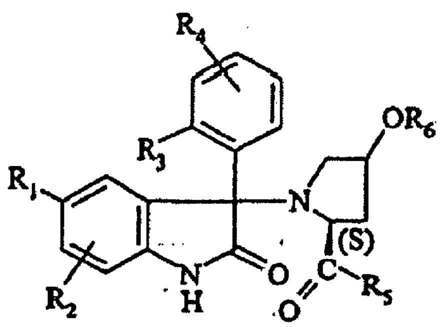

(II)

in der:

- das den Substituenten $-OR_6$ tragende Kohlenstoffatom die Konfiguration (R) aufweist; und
- $R_1$, $R_2$, $R_3$, $R_4$, $R_5$ und $R_6$ die bezüglich der Verbindung der Formel (Ia) in Form des linksdrehenden Isomeren in Anspruch 1 angegebenen Bedeutungen besitzen;

sowie deren Salze mit anorganischen oder organischen Säuren, in Form des optisch reinen Isomeren oder in Form einer Mischung der Diastereoisomeren.

**6.** Pharmazeutische Zubereitung enthaltend als Wirkstoff eine Verbindung nach einem der Ansprüche 1 bis 3 oder eines seiner pharmazeutisch annehmbaren Salze mit anorganischen oder organischen Säuren, Solvate und/oder Hydrate.

**7.** Verwendung einer Verbindung nach einem der Ansprüche 1 bis 3, ihrer pharmazeutisch annehmbaren Salze mit anorganischen oder organischen Säuren, ihrer Solvate und/oder Hydrate für die Herstellung von Arzneimitteln zur Behandlung sämtlicher Krankheiten, bei denen Arginin-Vasopressin und/oder seine Rezeptoren $V_{1b}$ oder gleichzeitig seine Rezeptoren $V_{1b}$ und seine Rezeptoren $V_{1a}$ eine Rolle spielen.

8. Arzneimittel, **dadurch gekennzeichnet, daß** es aus einer Verbindung nach einem der Ansprüche 1 bis 3 gebildet ist.